# EUROPEAN PATENT SPECIFICATION

(11) **EP 4 185 570 B1**
(45) Date of publication and mention of the grant of the patent: **09.10.2024**
(21) Application number: 21749129.9
(22) Date of filing: 21.07.2021
(51) Int. Cl.: C07D 249/08, C07D 401/08, C07D 403/08, A61P 25/00, A61K 31/496, A61K 31/497, A61K 31/439

(54) **CYCLOHEXYL SUBSTITUTED TRIAZOLES AS VASOPRESSIN RECEPTOR V1A ANTAGONISTS**
CYCLOHEXYL-SUBSTITUIERTE TRIAZOLE ALS VASOPRESSIN-REZEPTOR-V1A-ANTAGONISTEN
TRIAZOLES SUBSTITUÉS PAR CYCLOHEXYLE UTILISÉS EN TANT QU'ANTAGONISTES DU RÉCEPTEUR V1 DE LA VASOPRESSINE

(30) Priority: 23.07.2020 EP 20187302
(43) Date of publication of application: 31.05.2023
(73) Proprietor: F. Hoffmann-La Roche AG, 4070 Basel (CH)
(72) Inventor: DOLENTE, Cosimo, 4070 Basel (CH); GALLEY, Guido, 4070 Basel (CH); SCHNIDER, Patrick, 4070 Basel (CH)
(74) Representative: Jochnowitz, Evan
(86) International application number: PCT/EP2021/070347
(87) International publication number: WO 2022/018121

(56) References cited:
- WO-A1-2005/079808
- WO-A1-2005/105779
- WO-A1-2006/114706
- WO-A1-2006/123242
- WO-A1-2011/131596
- WO-A1-2013/050334
- WO-A1-2015/024819

## Description

The present invention provides cyclohexyl substituted triazoles, which act as V1a receptor modulators, and in particular as V1a receptor antagonists, their manufacture, pharmaceutical compositions containing them and their use as medicaments. The present compounds are useful as therapeutics acting peripherally and centrally in the conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

### Technical Field

The present invention provides a compound of formula I, 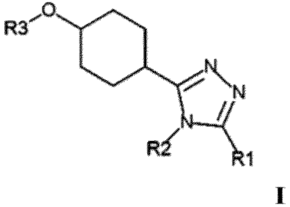 wherein the substituents and variables are as described below and in the claims, or a pharmaceutically acceptable salt thereof.

Also disclosed, is a method is provided for antagonizing the V1a receptor, the method comprising contacting the receptor with an effective amount of a compound of formula I as described herein, or a pharmaceutically acceptable salt thereof, or a pharmaceutical composition comprising the same.

Also disclosed, is a method is provided for treatment of a malcondition in a subject for which antagonism of the V1a receptor is medically indicated. Such method comprises administering to the subject an effective amount of a compound of formula I as described herein, or a pharmaceutically acceptable salt thereof, or a pharmaceutical composition comprising the same, at a frequency and for duration sufficient to provide a beneficial effect to the subject.

In an embodiment, a pharmaceutical composition is provided comprising a compound of formula I as described herein, or a pharmaceutically acceptable salt thereof, in combination with at least one pharmaceutically acceptable carrier, diluent, or excipient.

In an embodiment, a method of synthesis is provided for a compound of formula I as described herein, or a pharmaceutically acceptable salt thereof.

WO2005/105779 discloses 3-Heterocyclyl-4-Phenyl-Triazole Derivatives as Inhibitors of the Vasopressin V1A Receptor.

The present compounds are V1a receptor antagonists, useful for the treatment of autistic spectrum disorders, anxiety, social anxiety disorder, post-traumatic stress disorder (PTSD), brain edema in stroke or traumatic brain injury, and phase shift sleep disorders, in particular jetlag.

### Background Art

Three vasopressin receptors, all belonging to the class I G-protein coupled receptors, are known. The V1a receptor is expressed in the brain, liver, vascular smooth muscle, lung, uterus and testis, the V1b or V3 receptor is expressed in the brain and pituitary gland, the V2 receptor is expressed in the kidney where it regulates water reabsorption and mediates the antidiuretic effects of vasopressin (Robben, *et al*.)¹. Compounds with activity at the V2 receptor may therefore cause side-effects on blood homeostasis.

The oxytocin receptor is related to the Vasopressin receptor family and mediates the effects of the neurohormone oxytocin in the brain and the periphery. Oxytocin is believed to have central anxiolytic effects (Neumann)². Central oxytocin receptor antagonism might therefore lead to anxiogenic effects, which are regarded as undesired side-effects.

In the brain vasopressin acts as a neuromodulator and is elevated in the amygdala during stress (Ebner, *et al*.)³. It is known that stressful life events can trigger major depression and anxiety (Kendler, *et al*.)⁴ and that both have very high comorbidity, with anxiety often preceding major depression (Regier, *et al*.)⁵. The V1a receptor is extensively expressed in the brain and particularly in limbic areas like the amygdala, lateral septum and hippocampus which are playing an important role in the regulation of anxiety. Indeed V1a knock-out mice show a reduction in anxious behavior in the plus-maze, open field and light-dark box (Bielsky, *et al*.)⁶. The down-regulation of the V1a receptor using antisense oligonucleotide injection in the septum also causes a reduction in anxious behavior (Landgraf, *et al*.)⁷. Vasopressin or the V1a receptor are also implicated in other neuropsychological disorders: genetic studies linked sequence polymorphism in the promoter of the human V1a receptor to autistic spectrum disorders (Yirmiya, *et al*.)⁸, intranasal administration of vasopressin was shown to influence aggression in human males (Thompson, *et al*.)⁹ and vasopressin levels were found to be elevated in schizophrenic patients (Raskind, *et al*.)¹⁰ and patients with obsessive-compulsive disorder (Altemus, *et al*.)¹¹.

Autistic Spectrum Disorders (ASD) are a clinically heterogeneous condition characterized by defects in socialization and language. ASD include a wide range of abnormalities including a genuine incapacity to organize affective relations, behavioral anomalies in reciprocal social interactions, verbal and non-verbal communication, limited interest in the surrounding environment associated with stereotyped movements and repetitive plays (Bourreau *et al,* 2009)¹². Research to date indicates that a genetic predisposition may be involved, but also environmental factors have to be taken into consideration (Bourgeron, 2009)¹³. There is at present no efficient biological/ pharmaceutical treatment to ASD.

The suprachiasmatic nucleus (SCN) is the endogenous clock of the body regulating circadian rhythmicity and is known to be rich in vasopressin neurons (Kalsbeek *et al.* 2010)¹⁴, producing and releasing vasopressin with a 24h circadian rhythm (Schwartz *et al.* 1983)¹⁵. A major regulatory effect of vasopressin on circadian rhythm could not be demonstrated by the prior art. The Brattleboro rat, a rat strain naturally lacking vasopressin due to a point mutation, has no obvious defect in its circadian rhythm (Groblewski *et al.* 1981)¹⁶. Injection of vasopressin directly in the hamster SCN had no effect on circadian phase shift (Albers *et al.* 1984)¹⁷. In contrast, the vasopressin receptors were shown to modulate the circadian clock in a more subtle way. Yamaguchi *et al* (2013)¹⁸ demonstrated that V1a knock-out and V1a/V1b double knock-out mice show faster reentrainment to the new light/dark cycle after a circadian phase advance or a phase delay, an experiment mimicking jet-lag in humans. The same result was obtained after chronic administration of a mixture of V1a and V1b small molecule antagonists through a minipump directly on the SCN.

### Detailed description of the invention

The subject-matter for which protection is sought is as defined by the claims. Any reference to a "disclosure" or an "embodiment" not falling within the scope of the claims is present for explanatory purposes only and does not form part of the invention.

Object of the present invention is a compound of formula I as described herein and their pharmaceutically acceptable salts thereof, the preparation of the above mentioned compounds, medicaments containing them and their manufacture as well as the use of the above mentioned compounds in the therapeutic and/or prophylactic treatment of diseases and disorders which are associated with modulation of the V1a receptor, and in particular with V1a receptor antagonism. A further object of the invention is to provide selective inhibitors of the V1a receptor, since selectivity for the V1a receptor is expected to afford a low potential to cause unwanted off-target related side effects such as discussed above.

The following definitions of the general terms used in the present description apply irrespectively of whether the terms in question appear alone or in combination with other groups.

The term "alkyl", alone or in combination with other groups, stands for a hydrocarbon radical which may be linear or branched, with single or multiple branching, wherein the alkyl group in general comprises 1 to 6 carbon atoms, for example, methyl (Me), ethyl (Et), propyl, isopropyl (i-propyl), n-butyl, i-butyl (isobutyl), 2-butyl (sec-butyl), t-butyl (tert-butyl), isopentyl, 2-ethylpropyl, 1,2-dimethyl-propyl and the like. Particular "alkyl" groups have 1 to 4 carbon atoms ("alkyl"). A specific group is methyl.

The term "halogen", alone or in combination with other groups, denotes chloro (Cl), iodo (I), fluoro (F) and bromo (Br). Particular "halogen" groups are chloro or fluoro.

The term "halogen-alkyl" of "haloalkyl", alone or in combination with other groups, refers to alkyl as defined herein, which is substituted by one or multiple halogen, in particular 1-5 halogen, more particular 1-3 halogen. Particular "haloalkyl" groups are difluoromethyl and trifluoromethyl.

The term "hydroxy", alone or in combination with other groups, refers to -OH.

The term "hydroxy-alkyl", alone or in combination with other groups, denotes an alkyl group as defined above, with 1 to n carbon atoms, wherein at least one of the hydrogen atoms of the alkyl group is replaced by a hydroxy, i.e. by an OH group. An example for hydroxyalkyl is hydroxymethyl.

The term "alkoxy", alone or in combination with other groups, stands for an -O-alkyl radical which may be linear or branched, with single or multiple branching, wherein the alkyl group in general comprises 1 to 6 carbon atoms, for example, methoxy (OMe, MeO), ethoxy (OEt), propoxy, isopropoxy (i-propoxy), n-butoxy, i-butoxy (iso-butoxy), 2-butoxy (sec-butoxy), t-butoxy (tert-butoxy), isopentyloxy (i-pentyloxy) and the like. Particular "alkoxy" groups have 1 to 4 carbon atoms. Specific alkoxy groups include methoxy and ethoxy.

The term "alkoxyalkyl" denotes a C₁₋₆-alkyl group wherein at least one of the hydrogen atoms of the C₁₋₆-alkyl group is replaced by a C₁₋₆-alkoxy group. Particular examples are methoxymethyl, methoxyethyl, ethoxymethyl, ethoxyethyl, iso-propoxymethyl and isopropoxyethyl. Preferred example is methoxymethyl.

The term "amino" or "amine", alone or in combination with other groups, should be understood as being broadly applied to both a molecule, or a moiety or functional group, as generally understood in the art, and can be primary, secondary, or tertiary. The term "amine" or "amino" includes compounds where a nitrogen atom is covalently bonded to at least one carbon, hydrogen or heteroatom. The terms include, for example, but are not limited to, "amino-alkyl" and can include such moieties as NH-Alkyl, N-Alkyl, and NH₂. Examples for amine or amino groups include dimethylamino.

The term "amino-alkyl" denotes a C₁₋₆-alkyl group wherein at least one of the hydrogen atoms of the C₁₋₆-alkyl group is replaced by an amine group. Examples for amino-alkyl groups include methylaminomethyl, dimethylaminomethyl, and dimethylaminoethyl.

The term "amino-alkoxy" comprises groups and compounds wherein the nitrogen is bound to at least one additional alkoxy group. An example for an "amino-alkoxy" group includes dimethylaminoethoxy.

The term "cyano" denotes the group -CN.

The term "cycloalkyl" denotes monocyclic or polycyclic saturated or partially unsaturated, non-aromatic hydrocarbon. In some embodiments, unless otherwise described, cycloalkyl comprises 3 to 8 carbon atoms (C₃₋₈-cycloalkyl), 3 to 6 carbon atoms (C₃₋₆-cycloalkyl), or 3 to 5 carbon atoms (C₃₋₅-cycloalkyl). In some embodiments, cycloalkyl is a saturated monocyclic or polycyclic hydrocarbon. In other embodiments, cycloalkyl comprises one or more double bonds (e.g., cycloalkyl fused to an aryl or heteroaryl ring, or a non-aromatic monocyclic hydrocarbon comprising one or two double bonds). Polycyclic cycloalkyl groups may include spiro, fused, or bridged polycyclic moieties wherein each ring is a saturated or partially unsaturated, non-aromatic hydrocarbon. Examples of cycloalkyl groups include cyclopropyl, cyclobutyl, cyclopentyl, cyclohexyl, octahydropentalenyl, spiro[3.3]heptanyl, and the like. Preferred "cycloalkyl" group is cyclopropyl.

The term "aryl", alone or in combination with other groups, denotes a monovalent cyclic aromatic hydrocarbon moiety consisting of a mono- or bicyclic aromatic ring. Preferred aryl is phenyl. Aryl may be unsubstituted or substituted as described herein.

In case of "substituted aryl", examples include chlorophenyl, methoxyphenyl, chlorofluorophenyl, chloromethylphenyl, chloromethylsulfanylphenyl, chloro-[(dimethylamino)methyl]phenyl, dimethylphenyl, fluoromethylphenyl, difluorophenyl, trimethylphenyl, p-tolyl, fluorophenyl, (difluoromethyl)phenyl, cyanophenyl and bromophenyl.

The terms "heteroaryl", alone or in combination with other groups, refers to a monovalent aromatic containing from one to four ring heteroatoms selected from N, O, or S, the remaining ring atoms being C. Preferably, the monocyclic heteroaryl bears one or two heteroatoms. 5- or 6-membered heteroaryl are preferred. Examples for heteroaryl moieties include but are not limited to pyridyl, pyrazinyl, and thienyl. Heteroaryl may be unsubstituted or substituted as described herein. Preferred heteroaryl groups are pyridyl and pyrazinyl.

In case of "substituted heteroaryl", examples include chloropyridyl, chlorofluoropyridyl, chlorothienyl, methylpyridyl, (difluoromethyl)pyridyl, bromopyridyl, methoxypyridyl, hydroxypyridyl and methylpyrazinyl

The term "sulfonyl", alone or in combination with other groups, stands for an -S(O)₂-alkyl radical which may be linear or branched, with single or multiple branching, wherein the alkyl group in general comprises 1 to 6 carbon atoms, for example, methylsulfonyl or ethylsulfonyl. Particular "sulfonyl" group is methylsulfonyl.

The term "sulfanyl", alone or in combination with other groups, stands for an -S-alkyl radical which may be linear or branched with single or multiple branching, wherein the alkyl group in general comprises 1 to 6 carbon atoms, for example, methylsulfanyl or ethylsulfanyl. Particular "sulfanyl" group is methylsulfanyl.

The term "sulfinyl", alone or in combination with other groups, stands for an -S(O)-alkyl radical which may be linear or branched with single or multiple branching, wherein the alkyl group in general comprises 1 to 6 carbon atoms, for example, methylsulfinyl or ethylsulfinyl. Particular "sulfinyl" group is methylsulfinyl.

The term "sulfonylalkyl" denotes a C₁₋₆-alkyl group wherein at least one of the hydrogen atoms of the C₁₋₆-alkyl group is replaced by a sulfonyl group. Examples for "sulfonylalkyl" groups include methylsulfonylmethyl.

The term "sulfanylalkyl" denotes a C₁₋₆-alkyl group wherein at least one of the hydrogen atoms of the C₁₋₆-alkyl group is replaced by a sulfanyl group. Examples for "sulfanylalkyl" groups include methylsulfanylmethyl and methylsulfanylethyl.

The term "sulfinylalkyl" denotes a C₁₋₆-alkyl group wherein at least one of the hydrogen atoms of the C₁₋₆-alkyl group is replaced by a sulfinyl group. Examples for "sulfinylalkyl" groups include methylsulfinylmethyl.

The term [tert-butoxycarbonyl-(methyl)amino]alkyl denotes a C₁₋₆-alkyl group wherein at least one of the hydrogen atoms of the C₁₋₆-alkyl group is replaced by tert-butoxycarbonyl-(methyl)amino. A specific example of "[tert-butoxycarbonyl-(methyl)amino]alkyl" is [tert-butoxycarbonyl(methyl)amino]methyl.

The term "acetyl(methyl)aminoalkyl" denotes a C₁₋₆-alkyl group wherein at least one of the hydrogen atoms of the C₁₋₆-alkyl group is replaced by acetyl(methyl)amino. A specific example of "acetyl(methyl)aminoalkyl" is [acetyl(methyl)amino]methyl.

The term "benzloxyalkyl" denotes a C₁₋₆-alkyl group wherein at least one of the hydrogen atoms of the C₁₋₆-alkyl group is replaced by benzloxy. A specific example of "benzloxyalkyl" is 2-benzyloxyethyl.

When indicating the number of substituents, the term "one or more" means from one substituent to the highest possible number of substitution, i.e. replacement of one hydrogen up to replacement of all hydrogens by substituents. Thereby, one, two or three substituents are preferred. Even more preferred are one or two substituents or one substituent.

The term "pharmaceutically acceptable salts" refers to salts that are suitable for use in contact with the tissues of humans and animals. Examples of suitable salts with inorganic and organic acids are, but are not limited to acetic acid, citric acid, formic acid, fumaric acid, hydrochloric acid, lactic acid, maleic acid, malic acid, methane-sulfonic acid, nitric acid, phosphoric acid, p-toluenesulphonic acid, succinic acid, sulfuric acid, sulphuric acid, tartaric acid, trifluoroacetic acid and the like. Particular acids are formic acid, trifluoroacetic acid and hydrochloric acid. Particular are hydrochloric acid, trifluoroacetic acid and fumaric acid.

The terms "pharmaceutically acceptable carrier" and "pharmaceutically acceptable auxiliary substance" refer to carriers and auxiliary substances such as diluents or excipients that are compatible with the other ingredients of the formulation.

The term "pharmaceutical composition" encompasses a product comprising specified ingredients in pre-determined amounts or proportions, as well as any product that results, directly or indirectly, from combining specified ingredients in specified amounts. In particular, it encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients.

The term "half maximal inhibitory concentration" (IC₅₀) denotes the concentration of a particular compound required for obtaining 50% inhibition of a biological process in vitro. IC₅₀ values can be converted logarithmically to pIC₅₀ values (-log IC₅₀), in which higher values indicate exponentially greater potency. The IC₅₀ value is not an absolute value but depends on experimental conditions e.g. concentrations employed. The IC₅₀ value can be converted to an absolute inhibition constant (Ki) using the Cheng-Prusoff equation (Biochem. Pharmacol. (1973) 22:3099). The term "inhibition constant" (Ki) denotes the absolute binding affinity of a particular inhibitor to a receptor. It is measured using competition binding assays and is equal to the concentration where the particular inhibitor would occupy 50% of the receptors if no competing ligand (e.g. a radioligand) was present. Ki values can be converted logarithmically to pKi values (-log Kᵢ), in which higher values indicate exponentially greater potency.

The term "hydrate" refers to a compound that exists in combination with water molecules. The combination can include water in stoichiometric quantities, such as a monohydrate or a dehydrate, or can include water in random amounts. As the term is used herein a "hydrate" refers to a solid form; that is, a compound in a water solution, while it may be hydrated, is not a hydrate as the term is used herein.

The term "solvate" refers to a hydrate except that a solvent other that water is present. For example, methanol or ethanol can form an "alcoholate", which can again be stoichiometric or non-stoichiometric. As the term is used herein a "solvate" refers to a solid form; that is, a compound in a solvent solution, while it may be solvated, is not a solvate as the term is used herein.

The term "antagonist" denotes a compound that diminishes or prevents the action of another compound as defined e.g. in Goodman and Gilman's "The Pharmacological Basis of Therapeutics, 7th ed." in page 35, Macmillan Publ. Company, Canada, 1985. In particular, antagonists refer to a compound that attenuates the effect of an agonist. A "competitive antagonist" binds to the same site of a receptor as the agonist but does not activate the receptor, thus blocks the agonist's action. A "non-competitive antagonist" binds to an allosteric (non-agonist) site on the receptor to prevent activation of the receptor. A "reversible antagonist" binds non-covalently to the receptor, therefore can be "washed out". An "irreversible antagonist" binds covalently to the receptor and cannot be displaced by either competing ligands or washing.

"Isotope" refers to atoms with the same number of protons but a different number of neutrons, and an isotope of a compound of formula I as described herein includes any such compound wherein one or more atoms are replaced by an isotope of that atom. For example, carbon 12, the most common form of carbon, has six protons and six neutrons, whereas carbon 13 has six protons and seven neutrons, and carbon 14 has six protons and eight neutrons. Hydrogen has two stable isotopes, deuterium (one proton and one neutron) and tritium (one proton and two neutrons). While fluorine has a number of isotopes, fluorine 19 is longest-lived. Thus, an isotope of a compound of formula I as described herein includes, but not limited to, compounds of formula I as described herein wherein one or more carbon 12 atoms are replaced by carbon-13 and/or carbon-14 atoms, wherein one or more hydrogen atoms are replaced with deuterium and/or tritium, and/or wherein one or more fluorine atoms are replaced by fluorine-19.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The term "as defined herein" and "as described herein" when referring to a variable incorporates by reference the broad definition of the variable as well as preferred, more preferred and most preferred definitions, if any.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

The term "aromatic" denotes the conventional idea of aromaticity as defined in the literature, in particular in IUPAC¹⁹.

The term "pharmaceutically acceptable excipient" denotes any ingredient having no therapeutic activity and being non-toxic such as disintegrators, binders, fillers, solvents, buffers, tonicity agents, stabilizers, antioxidants, surfactants or lubricants used in formulating pharmaceutical products.

The terms "Autistic Spectrum" and "Autistic Spectrum Disorders" summarize conditions classified as pervasive developmental disorders, which include but are not limited to autism, Asperger syndrome, pervasive developmental disorder not otherwise specified (PDD-NOS), childhood disintegrative disorder, Rett syndrome and Fragile X, in particular autism. These disorders are typically characterized by social deficits, communication difficulties, stereotyped or repetitive behaviors and interests, and cognitive delays.

The term "phase shift sleep disorders" summarizes conditions classified as disturbances in the circadian rhythm, i.e. the approximately 24-hour cycles that are generated by an organism, e.g. a human being. Phase shift sleep disorders include, but are not limited to transient disorders like jetlag or a changed sleep schedule due to work, social responsibilities, or illness, as well as chronic disorders like delayed sleep-phase syndrome (DSPS), delayed sleep-phase type (DSPT), advanced sleep-phase syndrome (ASPS), and irregular sleep-wake cycle.

In detail, aspect 1, the present invention provides compounds of the general formula I 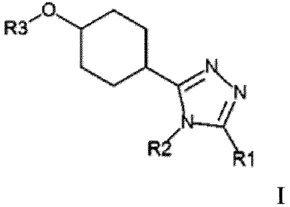 Wherein
R¹ is H, alkyl, cycloalkyl, haloalkyl, hydroxyalkyl, alkoxyalkyl, sulfanylalkyl, sulfinylalkyl, sulfonylalkyl, [tert-butoxycarbonyl-(methyl)amino]alkyl, aminoalkyl, acetyl(methyl)aminoalkyl, benzoxyalkyl, sulfonyl, alkoxy, aminoalkoxy, or amino;
R² is an unsubstituted aryl ring, a substituted aryl ring or a substituted heteroaryl ring wherein substituted aryl ring or substituted heteroaryl ring is substituted with one or more substituents independently selected from halogen, alkyl, alkoxy, sulfanyl, aminoalkyl, haloalkyl or cyano;
R³ is an unsubstituted aryl ring, an unsubstituted heteroaryl ring, or a substituted heteroaryl ring, wherein substituted heteroaryl ring is substituted with one or more substituents independently selected from alkyl, halogen, haloalkyl, alkoxy or hydroxy;
or a pharmaceutically acceptable salts thereof.

A certain embodiment of this invention, aspect 2, refers to a compound of formula I according to aspect 1, wherein R¹ is alkyl or alkoxyalkyl, or a pharmaceutically acceptable salt thereof.

A certain embodiment of this invention, aspect 3, refers to a compound of formula I according to aspects 1 to 2, wherein R¹ is selected from the group consisting of methyl or methoxymethyl; or a pharmaceutically acceptable salt thereof.

A certain embodiment of this invention, aspect 4, refers to a compound of formula I according to aspects 1 to 3, wherein R² is unsubstituted phenyl, substituted phenyl, a substituted 6-member nitrogen containing heteroaryl ring, or a substituted 5-member sulfur containing heteroaryl ring, wherein substituted phenyl, substituted 6-member nitrogen containing heteroaryl ring or substituted 5-member sulfur containing heteroaryl ring is substituted with one or more substituents independently selected from halogen, alkyl, alkoxy, sulfanyl, aminoalkyl, haloalkyl or cyano; or a pharmaceutically acceptable salt thereof.

A certain embodiment of this invention, aspect 5, refers to a compound of formula I according to aspects 1 to 4, wherein R² is unsubstituted phenyl, substituted phenyl, substituted pyridyl or substituted thienyl, wherein substituted phenyl, substituted pyridyl or substituted thienyl are substituted with one to three substituents independently selected from halogen, alkyl, alkoxy, sulfanyl, aminoalkyl, haloalkyl or cyano; or a pharmaceutically acceptable salt thereof.

A certain embodiment of this invention, aspect 6, refers to a compound of formula I according to aspects 1 to 5, wherein R² is a substituted phenyl ring substituted with alkyl or halogen; or a pharmaceutically acceptable salt thereof.

A certain embodiment of this invention, aspect 7, refers to a compound of formula I according to aspects 1 to 6, wherein R² is a substituted phenyl ring substituted with methyl or chloro; or a pharmaceutically acceptable salt thereof.

A certain embodiment of this invention, aspect 8, refers to a compound of formula I according to aspects 1 to 7, wherein R³ is an unsubstituted phenyl, an unsubstituted heteroaryl ring containing nitrogen, or a substituted heteroaryl ring containing nitrogen, wherein substituted heteroaryl ring containing nitrogen is substituted with alkyl, halogen, haloalkyl, alkoxy or hydroxy; or a pharmaceutically acceptable salt thereof.

A certain embodiment of this invention, aspect 9, refers to a compound of formula I according to aspects 1 to 8, wherein R³ is an unsubstituted phenyl, an unsubstituted pyridyl, an unsubstituted pyrazinyl ring, a substituted pyridyl or a substituted pyrazinyl ring, wherein substituted pyridyl ring or substituted pyrazinyl ring is substituted with alkyl, halogen, haloalkyl, alkoxy or hydroxy; or a pharmaceutically acceptable salt thereof.

A certain embodiment of this invention, aspect 10, refers to a compound of formula I according to aspects 1 to 9, wherein R³ is an unsubstituted pyridyl, unsubstituted pyrazinyl ring, a substituted pyridyl ring or a substituted pyrazinyl ring, wherein substituted pyridyl ring or substituted pyrazinyl ring is substituted with alkyl; or a pharmaceutically acceptable salt thereof.

A certain embodiment of this invention, aspect 11, refers to a compound of formula I according to aspects 1 to 9, wherein R³ is an unsubstituted pyridyl, an unsubstituted pyrazinyl ring, a substituted pyridyl ring or a substituted pyrazinyl ring, wherein substituted pyridyl ring or substituted pyrazinyl ring is substituted with methyl; or a pharmaceutically acceptable salt thereof.

A certain embodiment of this invention, aspect 12, refers to a compound of formula I wherein R¹ is alkyl or alkoxyalkyl; R² is phenyl substituted with one to three substituents independently selected from halogen or alkyl; R³ is a 6-membered heteroaryl ring, unsubstituted or substituted with alkyl; or a pharmaceutically acceptable salt thereof.

A certain embodiment of this invention, aspect 13, refers to a compound of formula I wherein R¹ is methyl or methoxymethyl; R² is a substituted phenyl ring substituted with methyl or chloro; and R³ is an unsubstituted pyridyl ring, an unsubstituted pyrazinyl ring, a substituted pyridyl ring or a substituted pyrazinyl ring, wherein substituted pyridyl ring or substituted pyrazinyl ring is substituted with methyl; or a pharmaceutically acceptable salts thereof;

Examples for the compound according to the invention are shown in the experimental part and the table below.

| **Ex** | **Structure** | **Ex** | **Structure** | **Ex** | **Structure** |
|---|---|---|---|---|---|
| 1 | 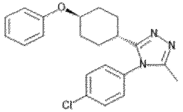 | 2 | 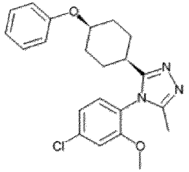 | 3 | 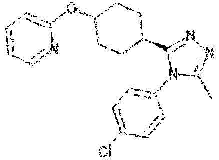 |
| 4 | 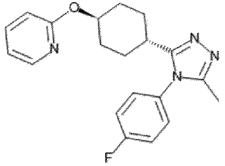 | 5 | 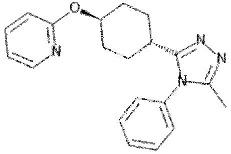 | 6 | 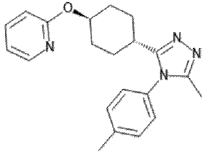 |
| 7 | 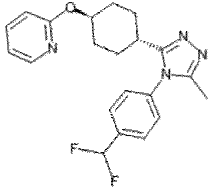 | 8 | 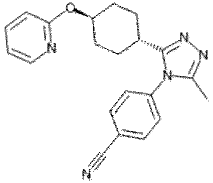 | 9 | 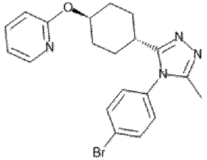 |
| 10 | 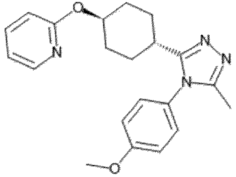 | 11 | 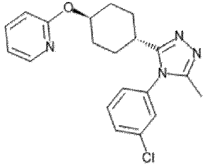 | 12 | 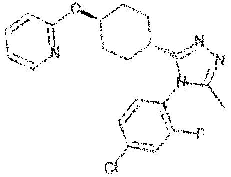 |
| 13 | 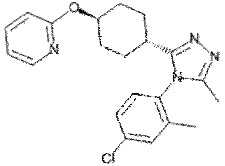 | 14 | 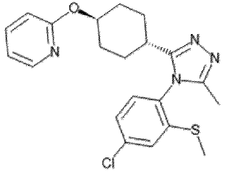 | 15 | 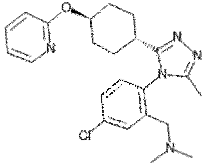 |
| 16 | 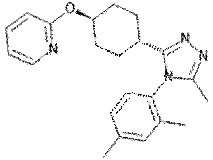 | 17 | 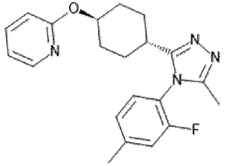 | 18 | 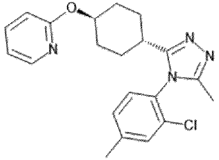 |
| 19 | 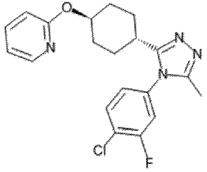 | 20 | 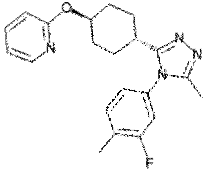 | 21 | 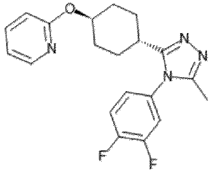 |
| 22 | 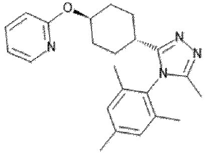 | 23 | 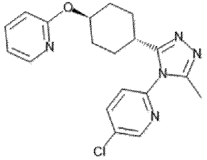 | 24 | 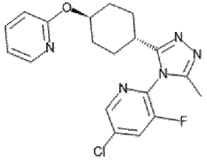 |
| 25 | 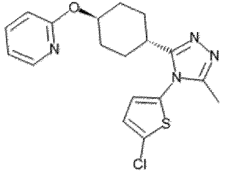 | 26 | 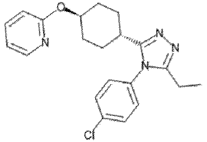 | 27 | 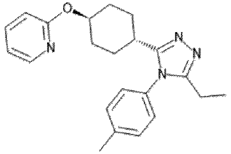 |
| 28 | 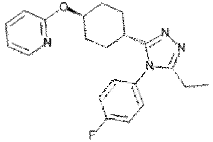 | 29 | 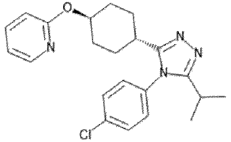 | 30 | 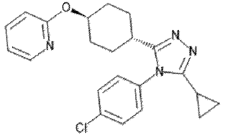 |
| 31 | 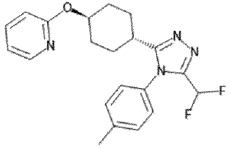 | 32 | 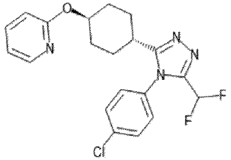 | 33 | 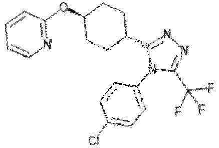 |
| 34 | 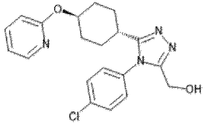 | 35 | 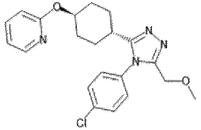 | 36 | 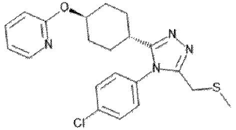 |
| 37 | 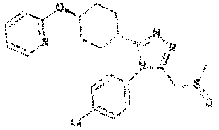 | 38 | 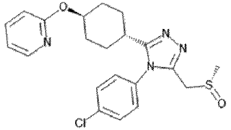 | 39 | 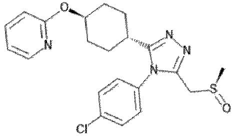 |
| 40 | 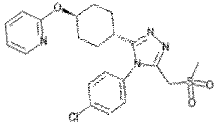 | 41 | 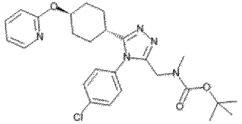 | 42 | 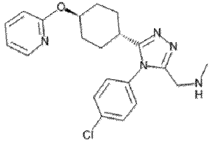 |
| 43 | 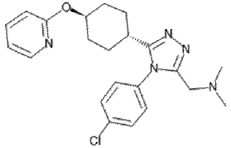 | 44 | 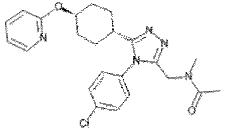 | 45 | 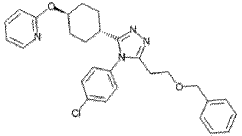 |
| 46 | 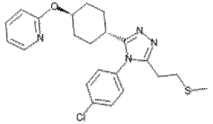 | 47 | 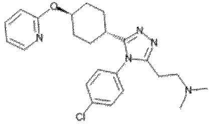 | 48 | 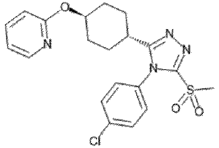 |
| 49 | 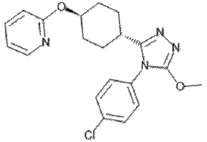 | 50 | 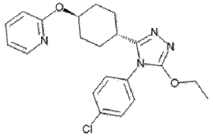 | 51 | 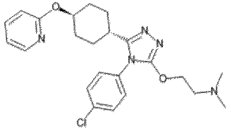 |
| 52 | 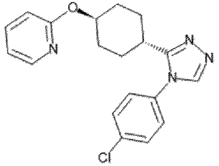 | 53 | 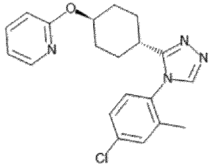 | 54 | 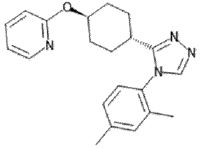 |
| 55 | 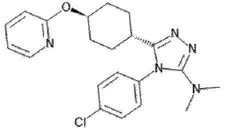 | 56 | 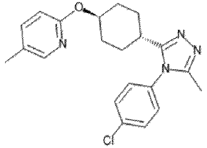 | 57 | 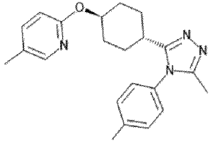 |
| 58 | 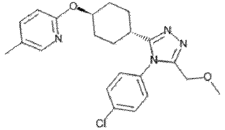 | 59 | 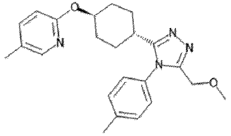 | 60 | 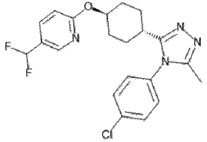 |
| 61 | 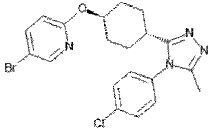 | 62 | 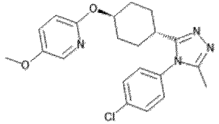 | 63 | 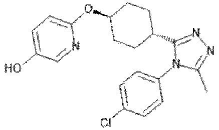 |
| 64 | 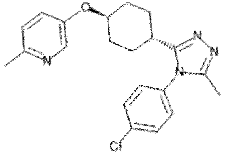 | 65 | 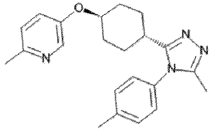 | 66 | 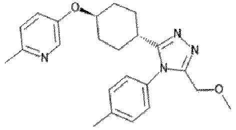 |
| 67 | 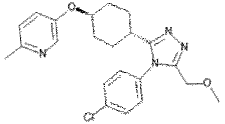 | 68 | 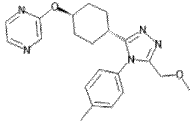 | 69 | 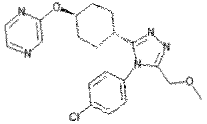 |
| 70 | 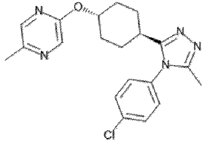 | 71 | 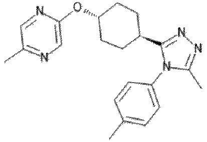 | 72 | 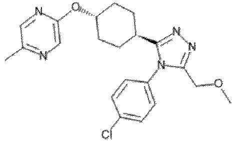 |

Preferred compounds of the invention are shown in the examples.

Particularly preferred compounds of the invention are:
trans-4-(4-Chloro-phenyl)-3-methyl-5-(4-phenoxy-cyclohexyl)-4H-[1,2,4]triazole;
cis-4-(4-Chloro-2-methoxy-phenyl)-3-methyl-5-(4-phenoxy-cyclohexyl)-4H-[1,2,4]triazole;
trans-2-(4-(4-(4-chlorophenyl)-5-methyl-4H-1,2,4-triazol-3-yl)cyclohexyloxy)pyridine;
trans-2-((4-(4-(4-fluorophenyl)-5-methyl-4H-1,2,4-triazol-3-yl)cyclohexyl)oxy)pyridine;
trans-2-((4-(5-methyl-4-phenyl-4H-1,2,4-triazol-3-yl)cyclohexyl)oxy)pyridine;
trans-2-((4-(5-methyl-4-(p-tolyl)-4H-1,2,4-triazol-3-yl)cyclohexyl)oxy)pyridine;
trans-2-((4-(4-(4-(difluoromethyl)phenyl)-5-methyl-4H-1,2,4-triazol-3-yl)cyclohexyl)oxy)pyridine;
trans-4-(3-methyl-5-(4-(pyridin-2-yloxy)cyclohexyl)-4H-1,2,4-triazol-4-yl)benzonitrile;
trans-2-((4-(4-(4-bromophenyl)-5-methyl-4H-1,2,4-triazol-3-yl)cyclohexyl)oxy)pyridine;
trans-2-((4-(4-(4-methoxyphenyl)-5-methyl-4H-1,2,4-triazol-3-yl)cyclohexyl)oxy)pyridine;
trans-2-((4-(4-(3-chlorophenyl)-5-methyl-4H-1,2,4-triazol-3-yl)cyclohexyl)oxy)pyridine;
trans-2-((4-(4-(4-chloro-2-fluorophenyl)-5-methyl-4H-1,2,4-triazol-3-yl)cyclohexyl)oxy)pyridine;
trans-2-((4-(4-(4-chloro-2-methylphenyl)-5-methyl-4H-1,2,4-triazol-3-yl)cyclohexyl)oxy)pyridine;
trans-2-[4-[4-(4-chloro-2-methylsulfanylphenyl)-5-methyl-1,2,4-triazol-3-yl]cyclohexyl]oxypyridine;
trans-1-[5-chloro-2-[3-methyl-5-(4-pyridin-2-yloxycyclohexyl)-1,2,4-triazol-4-yl]phenyl]-N,N-dimethylmethanamine;
trans-2-((4-(4-(2,4-dimethylphenyl)-5-methyl-4H-1,2,4-triazol-3-yl)cyclohexyl)oxy)pyridine;
trans-2-((4-(4-(2-fluoro-4-methylphenyl)-5-methyl-4H-1,2,4-triazol-3-yl)cyclohexyl)oxy)pyridine;
trans-2-((4-(4-(2-chloro-4-methylphenyl)-5-methyl-4H-1,2,4-triazol-3-yl)cyclohexyl)oxy)pyridine;
trans-2-((4-(4-(4-chloro-3-fluorophenyl)-5-methyl-4H-1,2,4-triazol-3-yl)cyclohexyl)oxy)pyridine;
trans-2-((4-(4-(3-fluoro-4-methylphenyl)-5-methyl-4H-1,2,4-triazol-3-yl)cyclohexyl)oxy)pyridine;
trans-2-((4-(4-(3,4-difluorophenyl)-5-methyl-4H-1,2,4-triazol-3-yl)cyclohexyl)oxy)pyridine;
trans-2-[4-[5-methyl-4-(2,4,6-trimethylphenyl)-1,2,4-triazol-3-yl]cyclohexyl]oxypyridine;
trans-5-chloro-2-(3-methyl-5-(4-(pyridin-2-yloxy)cyclohexyl)-4H-1,2,4-triazol-4-yl)pyridine;
trans-5-chloro-3-fluoro-2-(3-methyl-5-(4-(pyridin-2-yloxy)cyclohexyl)-4H-1,2,4-triazol-4-yl)pyridine;
trans-2-((4-(4-(5-chlorothiophen-2-yl)-5-methyl-4H-1,2,4-triazol-3-yl)cyclohexyl)oxy)pyridine;
trans-2-((4-(4-(4-chlorophenyl)-5-ethyl-4H-1,2,4-triazol-3-yl)cyclohexyl)oxy)pyridine;
trans-2-((4-(5-ethyl-4-(p-tolyl)-4H-1,2,4-triazol-3-yl)cyclohexyl)oxy)pyridine;
trans-2-((4-(5-ethyl-4-(4-fluorophenyl)-4H-1,2,4-triazol-3-yl)cyclohexyl)oxy)pyridine;
trans-2-((4-(4-(4-chlorophenyl)-5-isopropyl-4H-1,2,4-triazol-3-yl)cyclohexyl)oxy)pyridine;
trans-2-[4-[4-(4-chlorophenyl)-5-cyclopropyl-1,2,4-triazol-3-yl]cyclohexyl]oxypyridine;
trans-2-((4-(5-(difluoromethyl)-4-(p-tolyl)-4H-1,2,4-triazol-3-yl)cyclohexyl)oxy)pyridine;
trans-2-((4-(4-(4-chlorophenyl)-5-(difluoromethyl)-4H-1,2,4-triazol-3-yl)cyclohexyl)oxy)pyridine;
trans-2-((4-(4-(4-chlorophenyl)-5-(trifluoromethyl)-4H-1,2,4-triazol-3-yl)cyclohexyl)oxy)pyridine;
trans-(4-(4-chlorophenyl)-5-(4-(pyridin-2-yloxy)cyclohexyl)-4H-1,2,4-triazol-3-yl)methanol;
trans-2-(4-(4-(4-chlorophenyl)-5-(methoxymethyl)-4H- 1,2,4-triazol-3-yl)cyclohexyloxy)pyridine;
trans-2-[4-[4-(4-chlorophenyl)-5-(methylsulfanylmethyl)-1,2,4-triazol-3-yl]cyclohexyl]oxypyridine;
trans-(rac)-2-((4-(4-(4-chlorophenyl)-5-((methylsulfinyl)methyl)-4H-1,2,4-triazol-3-yl)cyclohexyl)oxy)pyridine;
trans-(-)-2-((4-(4-(4-chlorophenyl)-5-((methylsulfinyl)methyl)-4H-1,2,4-triazol-3-yl)cyclohexyl)oxy)pyridine;
trans-(+)-2-((4-(4-(4-chlorophenyl)-5-((methylsulfinyl)methyl)-4H-1,2,4-triazol-3-yl)cyclohexyl)oxy)pyridine;
trans-2-((4-(4-(4-chlorophenyl)-5-((methylsulfonyl)methyl)-4H-1,2,4-triazol-3-yl)cyclohexyl)oxy)pyridine;
trans-tert-butyl ((4-(4-chlorophenyl)-5-(4-(pyridin-2-yloxy)cyclohexyl)-4H-1,2,4-triazol-3-yl)methyl)(methyl)carbamate;
trans-1-(4-(4-chlorophenyl)-5-(4-(pyridin-2-yloxy)cyclohexyl)-4H-1,2,4-triazol-3-yl)-N-methylmethanamine;
trans-1-(4-(4-chlorophenyl)-5-(4-(pyridin-2-yloxy)cyclohexyl)-4H-1,2,4-triazol-3-yl)-N,N-dimethylmethanamine;
trans-N-((4-(4-chlorophenyl)-5-(4-(pyridin-2-yloxy)cyclohexyl)-4H-1,2,4-triazol-3-yl)methyl)-N-methylacetamide;
trans-2-[4-[4-(4-chlorophenyl)-5-(2-phenylmethoxyethyl)-1,2,4-triazol-3-yl]cyclohexyl]oxypyridine;
trans-2-((4-(4-(4-chlorophenyl)-5-(2-(methylthio)ethyl)-4H-1,2,4-triazol-3-yl)cyclohexyl)oxy)pyridine;
trans-2-[4-(4-chlorophenyl)-5-(4-pyridin-2-yloxycyclohexyl)-1,2,4-triazol-3-yl]-N,N-dimethylethanamine;
trans-2-((4-(4-(4-chlorophenyl)-5-(methylsulfonyl)-4H-1,2,4-triazol-3-yl)cyclohexyl)oxy)pyridine;
trans-2-((4-(4-(4-chlorophenyl)-5-methoxy-4H-1,2,4-triazol-3-yl)cyclohexyl)oxy)pyridine;
trans-2-((4-(4-(4-chlorophenyl)-5-ethoxy-4H-1,2,4-triazol-3-yl)cyclohexyl)oxy)pyridine;
trans-2-[[4-(4-chlorophenyl)-5-(4-pyridin-2-yloxycyclohexyl)-1,2,4-triazol-3-yl]oxy]-N,N-dimethylethanamine;
trans-2-((4-(4-(4-chlorophenyl)-4H-1,2,4-triazol-3-yl)cyclohexyl)oxy)pyridine;
trans-2-((4-(4-(4-chloro-2-methylphenyl)-4H-1,2,4-triazol-3-yl)cyclohexyl)oxy)pyridine;
trans-2-((4-(4-(2,4-dimethylphenyl)-4H-1,2,4-triazol-3-yl)cyclohexyl)oxy)pyridine;
trans-4-(4-chlorophenyl)-N,N-dimethyl-5-(4-(pyridin-2-yloxy)cyclohexyl)-4H-1,2,4-triazol-3 -amine;
trans-2-((4-(4-(4-chlorophenyl)-5-methyl-4H-1,2,4-triazol-3-yl)cyclohexyl)oxy)-5-methylpyridine;
trans-5-methyl-2-[4-[5-methyl-4-(4-methylphenyl)-1,2,4-triazol-3-yl]cyclohexyl]oxypyridine;
trans-2-((4-(4-(4-chlorophenyl)-5-(methoxymethyl)-4H-1,2,4-triazol-3-yl)cyclohexyl)oxy)-5-methylpyridine;
trans-2-[4-[5-(methoxymethyl)-4-(4-methylphenyl)-1,2,4-triazol-3-yl]cyclohexyl]oxy-5-methylpyridine;
trans-2-((4-(4-(4-chlorophenyl)-5-methyl-4H-1,2,4-triazol-3-yl)cyclohexyl)oxy)-5-(difluoromethyl)pyridine;
trans-5-bromo-2-(4-(4-(4-chlorophenyl)-5-methyl-4H-1,2,4-triazol-3-yl)cyclohexyloxy)pyridine;
trans-2-(4-(4-(4-chlorophenyl)-5-methyl-4H-1,2,4-triazol-3-yl)cyclohexyloxy)-5-methoxypyridine;
trans-6-(4-(4-(4-chlorophenyl)-5-methyl-4H-1,2,4-triazol-3-yl)cyclohexyloxy)pyridin-3-ol;
trans-5-[4-[4-(4-chlorophenyl)-5-methyl-1,2,4-triazol-3-yl]cyclohexyl]oxy-2-methylpyridine;
trans-2-methyl-5-[4-[5-methyl-4-(4-methylphenyl)-1,2,4-triazol-3-yl]cyclohexyl]oxypyridine;
trans-5-[4-[5-(methoxymethyl)-4-(4-methylphenyl)-1,2,4-triazol-3-yl]cyclohexyl]oxy-2-methylpyridine;
trans-5-[4-[4-(4-chlorophenyl)-5-(methoxymethyl)-1,2,4-triazol-3-yl]cyclohexyl]oxy-2-methylpyridine;
trans-2-[4-[5-(methoxymethyl)-4-(4-methylphenyl)-1,2,4-triazol-3-yl]cyclohexyl]oxypyrazine;
trans-2-[4-[4-(4-chlorophenyl)-5-(methoxymethyl)-1,2,4-triazol-3-yl]cyclohexyl]oxypyrazine;
trans-2-[4-[4-(4-chlorophenyl)-5-methyl-1,2,4-triazol-3-yl]cyclohexyl]oxy-5-methylpyrazine;
trans-2-methyl-5-[4-[5-methyl-4-(4-methylphenyl)-1,2,4-triazol-3-yl]cyclohexyl]oxypyrazine;
trans-2-[4-[4-(4-chlorophenyl)-5-(methoxymethyl)-1,2,4-triazol-3-yl]cyclohexyl]oxy-5-methylpyrazine;
or pharmaceutically acceptable salt thereof.

More preferred compounds are:
trans-2-(4-(4-(4-chlorophenyl)-5-methyl-4H-1,2,4-triazol-3-yl)cyclohexyloxy)pyridine;
trans-2-(4-(4-(4-chlorophenyl)-5-(methoxymethyl)-4H-1,2,4-triazol-3-yl)cyclohexyloxy)pyridine;
trans-2-((4-(4-(4-chlorophenyl)-5-methyl-4H-1,2,4-triazol-3-yl)cyclohexyl)oxy)-5-methylpyridine;
trans-5-methyl-2-[4-[5-methyl-4-(4-methylphenyl)-1,2,4-triazol-3-yl]cyclohexyl] oxypyridine;
trans-2-((4-(4-(4-chlorophenyl)-5-(methoxymethyl)-4H-1,2,4-triazol-3-yl)cyclohexyl)oxy)-5-methylpyridine;
trans-2-[4-[5-(methoxymethyl)-4-(4-methylphenyl)-1,2,4-triazol-3-yl]cyclohexyl]oxy-5-methylpyridine;
trans-5-[4-[4-(4-chlorophenyl)-5-methyl-1,2,4-triazol-3-yl]cyclohexyl]oxy-2-methylpyridine;
trans-2-methyl-5-[4-[5-methyl-4-(4-methylphenyl)-1,2,4-triazol-3-yl]cyclohexyl]oxypyridine;
trans-5-[4-[5-(methoxymethyl)-4-(4-methylphenyl)-1,2,4-triazol-3-yl]cyclohexyl]oxy-2-methylpyridine;
trans-5-[4-[4-(4-chlorophenyl)-5-(methoxymethyl)-1,2,4-triazol-3-yl]cyclohexyl]oxy-2-methylpyridine;
trans-2-[4-[4-(4-chlorophenyl)-5-(methoxymethyl)-1,2,4-triazol-3-yl]cyclohexyl]oxy-5-methylpyrazine;
or pharmaceutically acceptable salt thereof.

Particularly preferred are:
trans-2-(4-(4-(4-chlorophenyl)-5-(methoxymethyl)-4H-1,2,4-triazol-3-yl)cyclohexyloxy)pyridine;
trans-2-((4-(4-(4-chlorophenyl)-5-methyl-4H-1,2,4-triazol-3-yl)cyclohexyl)oxy)-5-methylpyridine;
trans-2-((4-(4-(4-chlorophenyl)-5-(methoxymethyl)-4H-1,2,4-triazol-3-yl)cyclohexyl)oxy)-5-methylpyridine;
trans-2-[4-[5-(methoxymethyl)-4-(4-methylphenyl)-1,2,4-triazol-3-yl]cyclohexyl]oxy-5-methylpyridine;
trans-5-[4-[4-(4-chlorophenyl)-5-(methoxymethyl)-1,2,4-triazol-3-yl]cyclohexyl]oxy-2-methylpyridine;
or pharmaceutically acceptable salt thereof.

In a certain embodiment, the invention relates to a process comprising reacting a compound of formula **VII** to a compound of formula **I:** 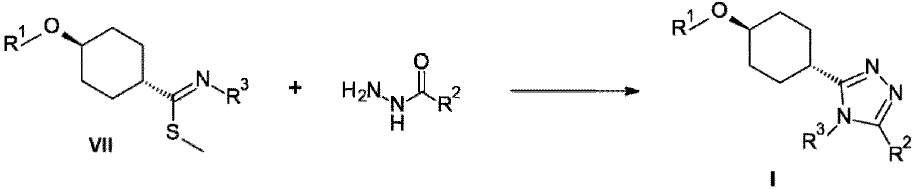 wherein R¹, R² and R³ are as defined hereinabove for formula I.

In a certain embodiment, the invention relates to a process comprising reacting a compound of formula V to a compound of formula I: 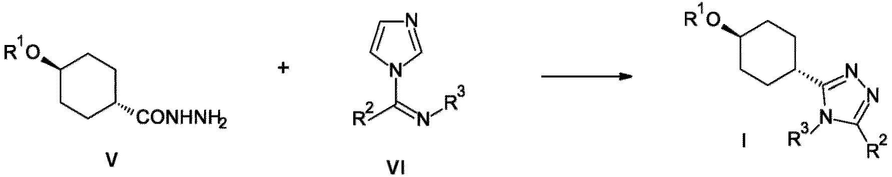 wherein R¹, R² and R³ are as defined hereinabove for formula I.

A certain embodiment of the invention relates to the intermediate VII: 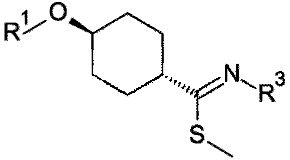 wherein R¹, and R³ are as defined hereinabove for formula I.

A certain embodiment of the invention relates to the intermediate V: 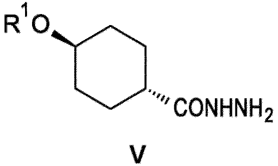 wherein R¹ is as defined hereinabove for formula **I.**

A certain embodiment of the invention relates to a process to synthesize an intermediate of formula V comprising the following steps: 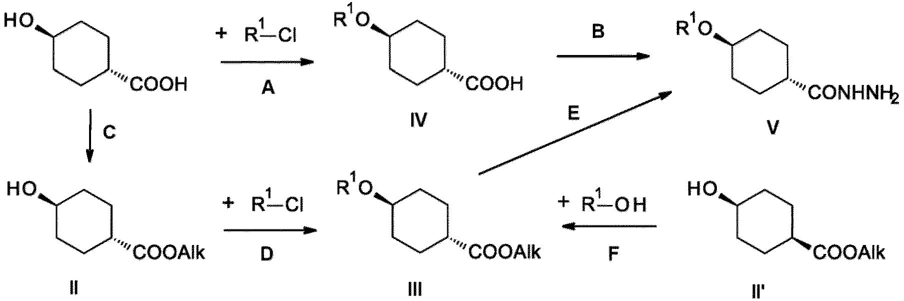 wherein R¹ is as defined hereinabove for formula **I.**

Also disclosed, is a compound of formula I as described herein, whenever prepared by a process as defined herein.

A certain embodiment of this invention refers to a compound of formula I as described herein for use as therapeutically active substance.

A certain embodiment of this invention refers to a pharmaceutical composition comprising a compound of formula I as described herein and a pharmaceutically acceptable carrier and/or a pharmaceutically acceptable auxiliary substance or excipient.

A certain embodiment of this invention refers to a compound of formula I as described herein for the use as therapeutically active substance for the therapeutic and/or prophylactic treatment of diseases and disorders which are associated with V1a receptor antagonism.

Also disclosed, is a method for the treatment of a vasopressin-dependent condition, whether organic, stress-induced or iatrogenic.

The terms "vasopressin-dependent condition" refer to conditions related to inappropriate secretion of vasopressin, particularly in the response to chronic stress and in circuits that are dysregulated in affective disorders, such as disorders of stress, mood, and behavioral disorders, including stress-related affective disorders. Vasopressin-dependent conditions, include conditions such as cardiovascular conditions, for example hypertension, pulmonary hypertension, cardiac insufficiency, myocardial infarction or coronary vasospasm, in particular in smokers, Raynaud's syndrome, unstable angina and PTCA (percutaneous transluminal coronary angioplasty), cardiac ischemia, hemostasis disturbances or thrombosis; conditions of the central nervous system, such as migraine, cerebral vasospasm, cerebral hemorrhage, trauma and cerebral edema, depression, anxiety, stress, emotional disorders, obsessive-compulsive disorder, panic attacks, psychotic states, aggression, memory or sleep disorders, or cognitive disorders, for example disorders associated with impaired social cognition (e.g., schizophrenia, autism spectrum disorder); conditions of the renal system, such as renal vasospasm, necrosis of the renal cortex, nephrogenic diabetes insipidus or diabetic nephropathy; or conditions of the gastric system, such as gastric vasospasm, cirrhosis of the liver, ulcers or the pathology of vomiting, for example nausea, including nausea due to chemotherapy, or travel sickness; circadian rhythm-related disorders such as phase shift sleep disorders, jet-lag, sleep disorders and other chronobiological disorders. Additional examples of vasopressin-dependent conditions include but are not limited to neuropsychiatric disorders, neuropsychiatric symptoms in neurodegenerative diseases, PTSD, inappropriate aggression, anxiety, depressive disorders, major depression, obsessive compulsive disorder, autistic spectrum disorders, schizophrenia, and aggressive behavior, and other affective disorders.

Also disclosed, is a method for the treatment of an autism spectrum disorder, comprising administering to a subject in need thereof an effective amount of a compound of formula I, or a pharmaceutically acceptable salt thereof, or a pharmaceutical composition comprising the same, at a frequency and for a duration sufficient to provide a beneficial effect to the subject.

Autism spectrum disorder (ASD), also referred to herein as autistic spectrum disorders, is a blanket term describing a complex developmental disorder that affects the brain's normal development of social and communication skills. Core symptoms of ASD include impaired social interactions such as social interaction difficulties, communication challenges including impaired verbal and nonverbal communication, problems processing information from the senses, and a tendency to engage in restricted and repetitive patterns of behavior. In one embodiment, the core symptoms of the autism spectrum disorder are impaired social interactions and communication challenges. In one embodiment, the core symptom of the autism spectrum disorder is impaired social interactions. In one embodiment, the core symptom of the autism spectrum disorder is impaired communication challenges. In one embodiment, the core symptom of the autism spectrum disorder is the tendency to engage in restricted and repetitive patterns of behavior.

Also disclosed is a method for the treatment of an anxiety disorder, comprising administering to a subject in need thereof an effective amount of a compound of formula I as described herein, or a pharmaceutically acceptable salt thereof, or a pharmaceutical composition comprising the same, at a frequency and for a duration sufficient to provide a beneficial effect to the subject.

Anxiety disorder is a blanket term covering several different forms of abnormal and pathological fear and anxiety. Current psychiatric diagnostic criteria recognize a wide variety of anxiety disorders, including generalized anxiety disorder, panic disorder, stress-related disorders, obsessive compulsive disorder, phobia, social anxiety disorder, separation anxiety disorder and post-traumatic stress disorder (PTSD). In one embodiment, the anxiety disorder is a social anxiety disorder. In one embodiment, the anxiety disorder is phobia. In one embodiment, the anxiety disorder is a stress-related disorder. In one embodiment, the anxiety related disorder is PTSD.

Generalized anxiety disorder is a common chronic disorder characterized by long-lasting anxiety that is not focused on any one object or situation. A person suffering from generalized anxiety experience non-specific persistent fear and worry and become overly concerned with everyday matters. Generalized anxiety disorder is the most common anxiety disorder to affect older adults.

In panic disorder, a person suffers from brief attacks of intense terror and apprehension, often marked by trembling, shaking, confusion, dizziness, nausea, difficulty breathing. These panic attacks, defined by the APA as fear or discomfort that abruptly arises and peaks in less than ten minutes, can last for several hours and can be triggered by stress, fear, or even exercise; although the specific cause is not always apparent. In addition to recurrent unexpected panic attacks, a diagnosis of panic disorder also requires that said attacks have chronic consequences: either worry over the attack's potential implications, persistent fear of future attacks, or significant changes in behavior related to the attacks. Accordingly, those suffering from panic disorder experience symptoms even outside of specific panic episodes. Often, normal changes in heartbeat are noticed by a panic sufferer, leading them to think something is wrong with their heart or they are about to have another panic attack. In some cases, a heightened awareness (hypervigilance) of body functioning occurs during panic attacks, wherein any perceived physiological change is interpreted as a possible life threatening illness (i.e. extreme hypochondriasis).

Obsessive compulsive disorder is a type of anxiety disorder primarily characterized by repetitive obsessions (distressing, persistent, and intrusive thoughts or images) and compulsions (urges to perform specific acts or rituals). The OCD thought pattern may be likened to superstitions insofar as it involves a belief in a causative relationship where, in reality, one does not exist. Often the process is entirely illogical; for example, the compulsion of walking in a certain pattern may be employed to alleviate the obsession of impending harm. And in many cases, the compulsion is entirely inexplicable, simply an urge to complete a ritual triggered by nervousness. In a minority of cases, sufferers of OCD may only experience obsessions, with no overt compulsions; a much smaller number of sufferers experience only compulsions.

The single largest category of anxiety disorders is that of Phobia, which includes all cases in which fear and anxiety is triggered by a specific stimulus or situation. Sufferers typically anticipate terrifying consequences from encountering the object of their fear, which can be anything from social phobia, specific phobia, agoraphobia, phobia of an animal to a location to a bodily fluid.

Post-traumatic stress disorder or PTSD is an anxiety disorder which results from a traumatic experience. Post-traumatic stress can result from an extreme situation, such as combat, rape, hostage situations, or even serious accident. It can also result from long term (chronic) exposure to a severe stressor, for example soldiers who endure individual battles but cannot cope with continuous combat. Common symptoms include flashbacks, avoidant behaviors, and depression.

Also disclosed, is a method for the treatment of a depressive disorder, depression, or depressive illness, comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject. Examples of such disorders include major depression, MDD, drug-resistant depression, dysthymia and bipolar disorder.

Also disclosed, is a method for the treatment of a mood disorder, or an affective disorder comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject.

Examples of a mood disorder or a affective disorder include major depressive disorder (MDD); bipolar disorder; anhedonia; dysthymia; major depression, Psychotic major depression (PMD), or psychotic depression; postpartum depression; seasonal affective disorder (SAD); and catatonic depression is a rare and severe form of major depression involving disturbances of motor behavior and other symptoms.

The terms "anhedonia" and "anhedonic symptom" are used interchangeably and is defined as the inability to experience pleasure from activities usually found enjoyable, e.g. exercise, hobbies, music, sexual activities or social interactions. The terms"anhedonia" and"anhedonic symptom" are closely related to criterion of "depressive disorder with melancholic features" which is defined in DSM-5 as melancholic depression characterized by a loss of pleasure in most or all activities, a failure of reactivity to pleasurable stimuli, a quality of depressed mood more pronounced than that of grief or loss, a worsening of symptoms in the morning hours, early morning waking, psychomotor retardation, excessive weight loss, or excessive guilt. The term"treatment of depressive disorder with melancholic features" comprises treatment of both the depressive disorder and melancholic features associated herewith. In one embodiment, the mood disorder is anhedonia. In one embodiment, the mood disorder is major depression. In one embodiment, the mood disorder is seasonal affective disorder (SAD).

Also disclosed, is a method for the treatment of an affective disorder, comprising administering to a subject in need thereof an effective amount of a compound of formula I as described herein, or a pharmaceutically acceptable salt thereof, or a pharmaceutical composition comprising the same, at a frequency and for a duration sufficient to provide a beneficial effect to the subject. Affective disorders such as disorders of stress, mood, and behavioral disorders, including stress-related affective disorders, obsessive compulsive disorder, autistic spectrum disorders, Personality disorders, ADHD, panic attacks and the like. As used herein,"autistic spectrum disorders" and "Autism spectrum disorders" are used interchangeably and refer to autism, monogenetic causes of autism such as synaptophathies, e.g., Rett syndrome, Fragile X syndrome, Angelman syndrome and the like.

Also disclosed, is a method for the treatment of Anger, Aggression or Aggressive Disorder, or Impulse Control Disorders comprising administering to a subject in need thereof an effective amount of a compound of formula I as described herein, or a pharmaceutically acceptable salt thereof, or a pharmaceutical composition comprising the same, at a frequency and for a duration sufficient to provide a beneficial effect to the subject. Examples of Anger, Aggression or Aggressive Disorder, or Impulse Control Disorders include, but are not limited to, inappropriate aggression, aggressive behavior, aggression related to social isolation, for treatment of interpersonal violence co-occurring with such illness as ADHD, autism, bipolar disorder, emotional disorders, disorders of memory and/or cognition and cognitive disorders (such as Alzheimer's disease, Parkinson's disease, Huntington's disease and the like), and addictive disorder/substance abuse.

Also disclosed, is a method for the treatment of Intermittent Explosive Disorder (sometimes abbreviated as IED) comprising administering to a subject in need thereof an effective amount of a compound of formula I as described herein, salt thereof, or a pharmaceutical composition comprising the same, at a frequency and for a duration sufficient to provide a beneficial effect to the subject. Intermittent explosive disorder is a behavioral disorder characterized by explosive outbursts of anger and violence, often to the point of rage, that are disproportionate to the situation at hand (e.g., impulsive screaming triggered by relatively inconsequential events). Impulsive aggression is unpremeditated, and is defined by a disproportionate reaction to any provocation, real or perceived. Some individuals have reported affective changes prior to an outburst (e.g., tension, mood changes, energy changes, etc.). The disorder is currently categorized in the Diagnostic and Statistical Manual of Mental Disorders (DSM-5) under the "Disruptive, Impulse-Control, and Conduct Disorders" category. The disorder itself is not easily characterized and often exhibits comorbidity with other mood disorders, particularly bipolar disorder. Individuals diagnosed with IED report their outbursts as being brief (lasting less than an hour), with a variety of bodily symptoms (sweating, stuttering, chest tightness, twitching, palpitations) reported by a third of one sample. Aggressive acts are frequently reported accompanied by a sensation of relief and in some cases pleasure, but often followed by later remorse.

Also disclosed, is a method for the treatment of a Schizophrenia spectrum disorders, comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject. Examples of Schizophrenia spectrum disorders include schizophrenia, schizoaffective disorder, psychotic states and memory disorders.

Also disclosed, is a method or the treatment of a circadian rhythm related disorders, comprising administering to a subject in need thereof an effective amount of a compound of formula I as described herein, or a pharmaceutically acceptable salt thereof, or a pharmaceutical composition comprising the same, at a frequency and for duration sufficient to provide a beneficial effect to the subject. Circadian rhythm sleep disorders are caused by desynchronization or misalignment between internal sleep-wake rhythms (body clock) and the external light-darkness cycle. Circadian rhythm disorders (sometimes also referred to as phase shift disorders) include sleep disorders associated with jet lag, shift work, or altered sleep phase types, resulting in sleep drifting later each day, abnormal nigh sleep patterns, and/or difficulty staying awake during the day. The cause may be internal (e.g., delayed or advanced sleep phase syndrome, or Non-24-h sleep-wake syndrome) or external (e.g., jet lag, shift work). If the cause is external, other circadian body rhythms, including temperature and hormone secretion, can become out of sync with the light-darkness cycle (external desynchronization) and with one another (internal desynchronization); in addition to insomnia and excessive sleepiness, these alterations may cause nausea, malaise, irritability, and depression. Risk of cardiovascular and metabolic disorders may also be increased. Compounds of the invention are useful for treating circadian rhythm-related disorders, such as depression, jet-lag, work-shift syndrome, sleep disorders, glaucoma, reproduction, cancer, premenstrual syndrome, immune disorders, inflammatory articular diseases and neuroendocrine disorders, Non-24 Hour Disorder.

The compounds according to the invention may also be used in the treatment or prevention of Neuropsychiatric Disorders such as anorexia nervosa, bulimia, mood disorders, depression, anxiety, sleeping disorders, addictive disorders, panic attacks, phobias, obsession, pain-perception disorders (fibromyalgia), dependency on a substance, hemorrhagic stress, muscular spasms and hypoglycemia. Addictive disorder, including disorders related to substance abuse or addiction, and compulsive behavior.

The compounds according to the invention may also be used in the treatment or prevention of chronic stress states such as immunodepression, fertility disorders and dysfunctions of the hypothalamopituitaryadrenal axis.

The compounds according to the invention can also be used in the treatment of disorders such as primary or secondary dysmenorrhea, premature labor or endometriosis, male or female sexual dysfunction, hypertension, chronic heart failure, inappropriate secretion of vasopressin, liver cirrhosis and nephrotic syndrome.

The compounds according to the invention can also be used in the treatment or prevention of any pathology resulting from stress, such as fatigue and its syndromes, ACTH-dependent disorders, cardiac disorders, pain, modifications in gastric emptying, in fecal excretion (colitis, irritable bowel syndrome or Crohn's disease) or in acid secretion, hyperglycemia, immunosuppression, inflammatory processes (rheumatoid arthritis and osteoarthritis), multiple infections, septic shock, cancers, asthma, psoriasis and allergies.

The compounds according to the invention may also be used as psychostimulants, bringing about an increase in consciousness/alertness and/or in emotional reactivity towards the environment and making adaptation easier.

The compounds according to the present invention can be used in healing, in analgesia, in anxiolysis, in the prevention of pain, in the prevention of anxiety, depression, schizophrenia, autism or obsessive-compulsive syndrome, in maternal behavior (facilitation of recognition and acceptance of the mother by the child) and social behavior, memory; regulation of food and drink intake, dependence on drugs, withdrawal and sexual motivation; hypertension, hyponatremia, cardiac insufficiency, atherosclerosis, angiogenesis, the proliferation of tumors, Kaposi's sarcoma, to regulate the storage of fat by the adipocyte, to control hyperlipidemia, triglyceridemia and metabolic syndrome.

The compounds according to the invention can also be used in the treatment of cancers, such as small cell lung cancers or breast cancers; hyponatremic encephalopathy; pulmonary syndrome; Meniere's disease; ocular hypertension; glaucoma; cataracts; obesity; type-I and type-II diabetes; atherosclerosis; metabolic syndrome; hyperlipidemia; insulin resistance; or hypertriglyceridemia; in post-operative treatments, in particular after abdominal surgery; autism; hypercortisolemia; hyperaldosteronemia; pheochromocytoma; Cushing's syndrome; preeclampsia; disorders of micturition; or premature ejaculation.

Also disclosed, is the use of a compound of formula I as described herein for the therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

Also disclosed, is the use of a compound of formula I as described herein for the manufacture of a medicament for acting peripherally and centrally in the conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

A certain embodiment of this invention refers to a compound of formula I as described herein for the therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

A certain embodiment of this invention refers to a compound of formula I as described herein for the use as therapeutically active substance acting peripherally and centrally in the conditions of inappropriate secretion of vasopressin anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

Also disclosed, is a method for the use of a compound as described herein, which is acting peripherally and centrally in the conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag, which method comprises administering said compound of formula I to a human being or animal.

Also disclosed, is a method for the therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag, which method comprises administering a therapeutically effective amount of a compound of formula I.

### Synthesis of Intermediates

The synthesis of the intermediates **II** - **VII** can be accomplished according to the following Schemes **1-3.**

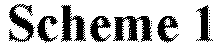

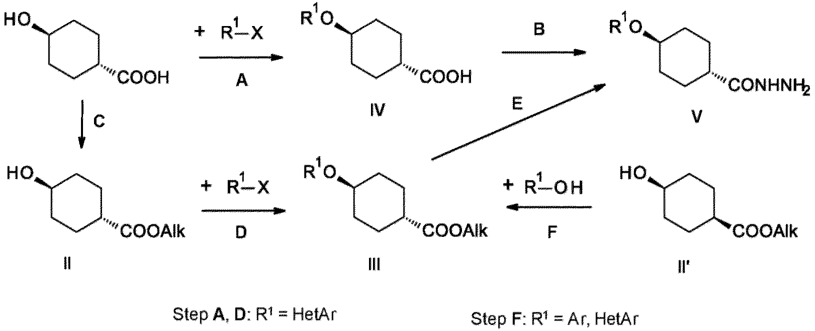

**Step A:** Reaction of trans-4-hydroxy-cyclohexanecarboxylic acid with a heteroaryl chloride or heteroaryl fluoride R¹X (X=Cl, F) to form the 4-(heteroaryloxy)-cyclohexanecarboxylic acid **IV** can be accomplished with an additional base such as sodium tert-pentoxide, sodium tert-butoxide, potassium tert-butoxide, sodium hydride or the like in a suitable solvent such as N-methyl-2-pyrrolidinone (NMP), dimethylacetamide (DMA), dimethylformamide (DMF), tetrahydrofuran (THF) or dimethoxyethane at 20 °C to 140 °C for 1 h to 4 days.

Convenient conditions are heating of trans-4-hydroxy-cyclohexanecarboxylic acid with a heteroaryl chloride or heteroaryl fluoride R¹X (X=Cl, F) and sodium tert-pentoxide in N-methyl-2-pyrrolidinone at 90 °C for 15 h to 2 days.

**Step B:** Formation of 4-(heteroaryloxy)-cyclohexanecarboxylic acid hydrazide **V** can be accomplished by reaction of 4-(heteroaryloxy)-cyclohexanecarboxylic acid **IV** with isobutyl chloroformate or ethyl chloroformate and a base such as triethylamine, diisopropylethylamine, N-methylmorpholine or the like in a suitable solvent like tetrahydrofuran or dichloromethane at 0-5 °C for 1-18 h, followed by removal of the salts and addition of hydrazine hydrate in methanol and further stirring at 0-25 °C for 1-18 h.

Convenient conditions are the reaction with isobutyl chloroformate and triethylamine in tetrahydrofuran at 0-5 °C for 1 h, followed by filtration and addition of of hydrazine hydrate in methanol until completion of the reaction.

**Step C:** The esterification to form trans-4-hydroxy-cyclohexanecarboxylic acid ester II can be accomplished by reaction of trans-4-hydroxy-cyclohexanecarboxylic acid with the alcohol Alk-OH in presence of an acid such as sulfuric acid or hydrochlorid acid using the alcohol as solvent at reflux for 1-24 h.

Convenient conditions are the reaction with sulfuric acid in methanol at 65 °C for 18 h.

A cis-4-hydroxy-cyclohexanecarboxylic acid ester **II'** can be prepared in analogy by starting from cis-4-hydroxy-cyclohexanecarboxylic acid instead of trans-4-hydroxy-cyclohexanecarboxylic acid.

**Step D:** The reaction of a 4-hydroxy-cyclohexanecarboxylic acid ester **II** with a heteroaryl chloride or heteroaryl fluoride R¹X (X=Cl, F) to form a 4-(heteroaryloxy)-cyclohexanecarboxylic acid ester **III** can be accomplished by treatment with a base such as potassium carbonate, cesium carbonate, sodium methanesulfinate, potassium tert-butoxide, sodium hydride or the like in a suitable solvent such as dimethylformamide (DMF), N-methyl-2-pyrrolidinone (NMP), dimethylacetamide (DMA), tetrahydrofuran (THF) or dimethoxyethane at 50 °C to 140 °C for 1 h to 120 h.

Convenient conditions are heating of a 4-hydroxy-cyclohexanecarboxylic acid ester with a heteroaryl chloride and a mixture of sodium methanesulfinate and potassium carbonate in dimethylformamide (DMF), at 120 °C for 3-18 h.

**Step E:** Formation of a trans-4-(aryloxy)-cyclohexanecarboxylic acid hydrazide **V** can be accomplished by reaction of a trans-4-(aryloxy)-cyclohexanecarboxylic acid ester **III** with hydrazine hydrate in a suitable solvent such as methanol, ethanol, propanol, butanol or water at 50-120 °C for 1-24 h.

Convenient conditions are heating a mixture of a 4-(aryloxy)-cyclohexanecarboxylic acid ester and hydrazine hydrate in butanol at 120 °C for 5 h.

**Step F:** Furthermore, formation of a trans-4-(aryloxy)-cyclohexanecarboxylic acid ester **III** can be achieved by reaction of a cis-4-hydroxy-cyclohexanecarboxylic acid ester **II'** with a phenol R¹-OH or a hydroxy-heteroaryl compound R¹-OH in the presence of an azodicarboxylate such as diethyl azodicarboxylate, diisopropyl azodicarboxylate or di-tert-butyl azodicarboxylate in the presence of a phosphine such as triphenylphosphine, tricyclohexylphosphine or the like in a suitable solvent such as tetrahydrofuran, dichloromethane, chloroform, dimethylformamid, dimethylsulfoxide or acetonitrile at 0 °C-50 °C for 1 h -48 h.

Convenient conditions are the use of triphenylphosphine and diethyl azodicarboxylate in tetrahydrofuran at 0 °C-20 °C for 3-18 h.

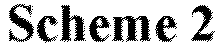

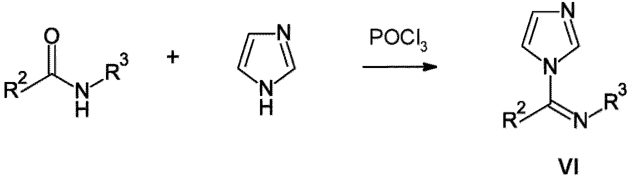

The reaction of a secondary amide with imidazole to form an imidazole intermediate **VI** can be accomplished in a suitable solvent such as acetonitrile or tetrahydrofuran in the presence of phosphoroxychloride at 0-75 °C for 1-24 h.

Convenient conditions are acetonitrile as solvent and a temperature of 0-5 °C for the addition of phosphoroxychloride followed by heating the mixture at 68 °C for 16 h.

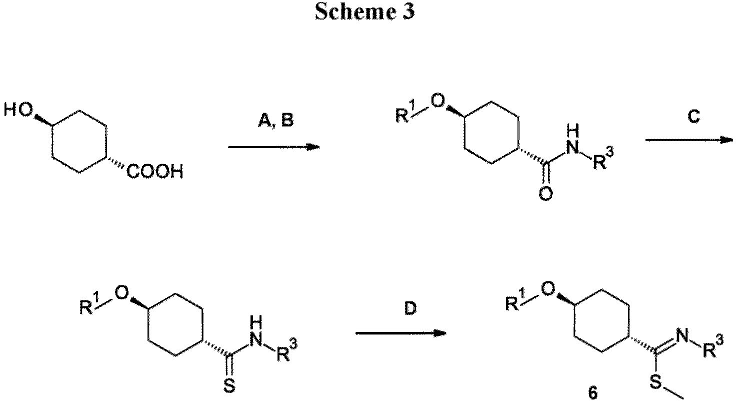

**Step A:** Reaction of trans-4-hydroxycyclohexanecarboxylic acid with a heteroaryl chloride R¹Cl can be accomplished with a base such as sodium tert-pentoxide, sodium tert-butoxide, potassium tert-butoxide, sodium hydride or the like in a suitable solvent such as N-methyl-2-pyrrolidinone (NMP), dimethylacetamide, dimethylformamide, tetrahydrofuran or dimethoxyethane at 20 °C to 140 °C for 1 h to 120 h.

Convenient conditions are heating of trans-4-hydroxycyclohexanecarboxylic acid with a heteroaryl chloride and sodium tert-pentoxide in N-methyl-2-pyrrolidinone at 90 °C for 96 h.

**Step B:** The amide coupling with an aromatic amine R³-NH₂ can be accomplished by transforming the acid into an acid chloride by using oxalyl chloride, thionyl chloride or phosphorus oxychloride followed by addition of the aromatic amine R³-NH₂ and a base such as triethylamine, diisopropylethylamine or N-methylmorpholine in various solvents such as dichloromethane, 1,2-dichloroethane, diethyl ether, dioxane, tetrahydrofuran or tert-butyl methyl ether at -20 °C to 70 °C for 1 to 24 h. Alternatively, acylation can be accomplished by a coupling reaction between the aromatic amine R³-NH₂ and the carboxylic acid in the presence of a coupling reagent such as DCC, EDC, TBTU or HATU in the presence of an organic base such as triethylamine, diisopropylethylamine or N-methylmorpholine in various solvents such as dichloromethane, 1,2-dichloroethane, diethyl ether, dioxane, tetrahydrofuran or tert-butyl methyl ether at 0 °C to 60 °C.

Convenient conditions are the reaction of the acid with oxalyl chloride in dichloromethane at 0 °C to 25 °C for 2.5 h followed by reaction of the acid chloride intermediate with the aromatic amine R³-NH₂ at 0 °C to 25 °C for 1.5 h.

The steps A and B can also be reversed.

**Step C:** Formation of the thioamide can be accomplished by reaction of the amide with a thionation reagent such as Lawesson's reagent or phosphorous pentasulfide in a suitable solvent such as tetrahydrofuran, toluene, xylene, dioxane or 1,2-dimethoxyetane at 20 °C to 140 °C for 1 h to 24 h.

Convenient conditions are the use of Lawesson's reagent in tetrahydrofuran at 65 °C for 3h.

**Step D:** Formation of the S-methylthioimidate **VI** can be accomplished by reaction of the thioamide with a methylation reagent such as methyl iodide, dimethyl sulfate or methyl 4-methylbenzenesulfonate and an inorganic base such as potassium tert-butoxide, sodium tert-butoxide, sodium hydride or the like in a suitable solvent such as tetrahydrofuran, dioxane, dimethylformamide or dimethylacetamide at 20 °C to 70 °C for 1 h to 24 h.

Convenient conditions are the use of methyl 4-methylbenzenesulfonate and potassium tert-butoxide in tetrahydrofuran at room temperature for 1h.

### Synthesis of Compounds of Formula I

The synthesis of the compounds of formula I can be, for example, accomplished according to the following Schemes **4 - 12.**

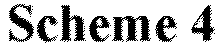

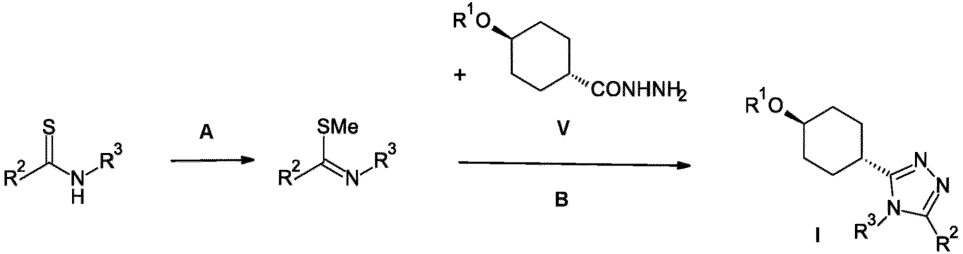

**Step A:** The reaction of the thioamide with a methylating reagent, such as iodomethane or dimethyl sulfate and an inorganic base such as potassium carbonate, cesium carbonate or the like in a suitable solvent such as dimethylformamide, dimethylacetamide, tetrahydrofuran or dioxane at 0 °C to 70 °C for 1 h to 24 h leads to formation of the S-methylthioimidate.

Convenient conditions are the use of iodomethane and potassium carbonate in dimethylformamide at 25 °C for 18 h.

**Step B:** Compounds of formula **I** can be synthesized by reaction of the S-methylthioimidate with trans-4-(aryloxy)-cyclohexanecarboxylic acid hydrazide **V** in a suitable solvent such as n-butanol, n-propanol, isopropanol or ethanol at elevated temperatures for 1 h to 24 h. Convenient conditions are the use of n-butanol and 120 °C for 18 h.

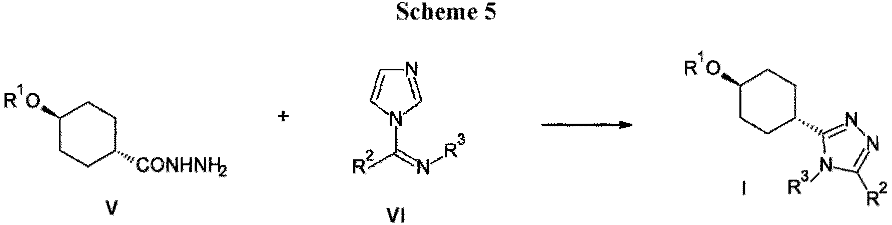

Furthermore, compounds of formula **I** can be synthesized by reaction of a trans-4-(aryloxy)-cyclohexanecarboxylic acid hydrazide **V** with an imidazole intermediate **VI** in a suitable solvent such as tetrahydrofuran, dioxane or dichloromethane in presence of an acid such as trifluoroacetic acid or acetic acid at 0 °C-25 °C for 1 h to 24 h.

Convenient conditions are stirring the reactants in presence of trifluoroacetic acid in tetrahydrofuran at 25 °C for 16 h.

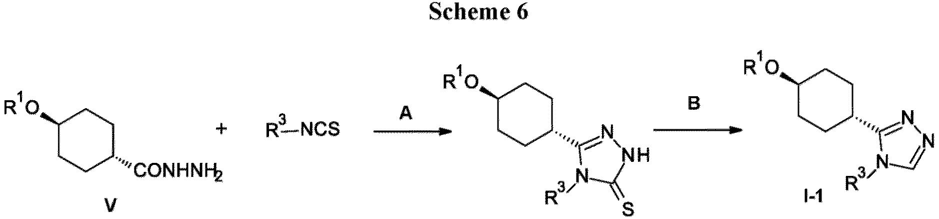

**Step A:** The reaction of a trans-4-(aryloxy)-cyclohexanecarboxylic acid hydrazide V with an isothiocyanate can be accomplished in a suitable solvent such as methanol, ethanol, n-propanol, n-butanol, dioxane or tetrahydrofuran at 25 °C to 100 °C for 15 min to 5 h, followed by heating the solid formed in an aqueous solution of sodium hydroxide, lithium hydroxide or potassium hydroxide at 60 °C to 100 °C for 15 min to 5 h.

Convenient conditions are heating the components in methanol to reflux for 1 h, followed by heating the solid formed in 1N sodium hydroxide solution to 100 °C for 1h.

**Step B:** Formation of compound **I-1** can be accomplished by reaction of the triazole-3-thione with hydrogenperoxide in an acid, such as acetic acid at 0 °C to 40 °C for 15 min to 5 h. Convenient conditions are reacting the triazole-3-thione in acetic acid with hydrogenperoxide at 25 °C for 1 h.

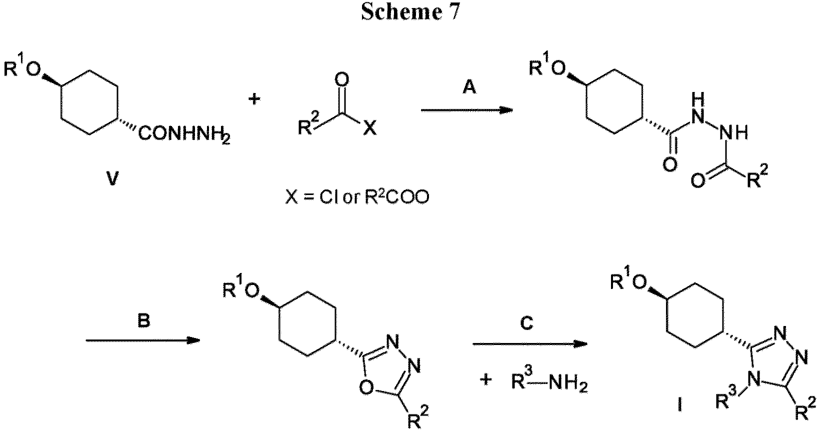

**Step A:** Reaction of a trans-4-(aryloxy)-cyclohexanecarboxylic acid hydrazide V with an acid chloride R²COCl or an acid anhydride (R²CO)₂O can be accomplished in a suitable solvent such as dichloromethane, 1,2-dichloroethane, diethyl ether, dioxane, tetrahydrofuran or tert-butyl methyl ether in presence of a base such as triethylamine, diisopropylethylamine or N-methylmorpholine in various solvents such as at -20 °C to 70 °C for 1 to 24 h.

Convenient conditions are stirring the components in dichloromethane at room temperature for 18 h.

**Step B:** Cyclisation to form the oxadiazole can be accomplished by subjecting the N'-acyl-hydrazide to dehydrating conditions, such as reaction with trifluoromethanesulfonic anhydride and pyridine in a suitable solvent such as dichloromethane or thetrahydrofuran at 0 °C-40 °C for 1 h to 24 h.

Convenient conditions are the use of trifluoromethanesulfonic anhydride and pyridine in dichloromethane at room temperature for 18 h.

**Step C:** Reaction of the oxadiazole with an aniline R³NH₂ to form compound **I** can be accomplished in a suitable solvent such as toluene, benzene or xylene in presence of a strong acid such as trifluoroacetic acid at elevated temperatures for 1 h to 24 h.

Convenient conditions are heating the components in toluene to 130 °C for 5 h to 24 h.

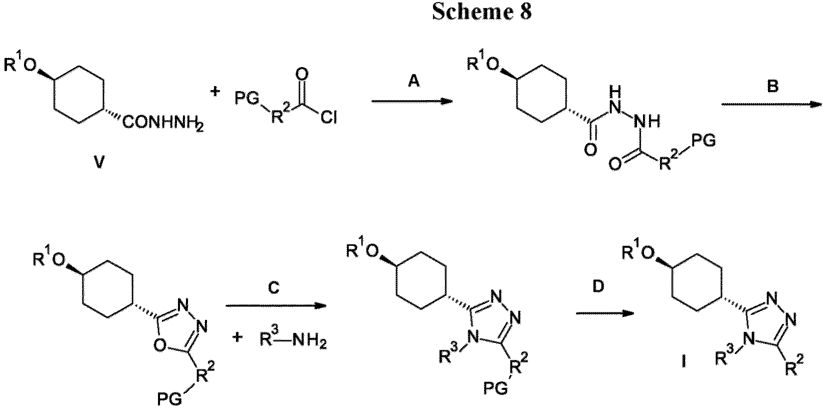

**Step A:** Reaction of trans-4-(aryloxy)-cyclohexanecarboxylic acid hydrazide **V** with an acid chloride carriyng a protecting group PG such as acetyl, benzoyl, benzyl or the like can be accomplished in a suitable solvent such as dichloromethane, 1,2-dichloroethane, diethyl ether, dioxane, tetrahydrofuran or tert-butyl methyl ether in presence of a base such as triethylamine, diisopropylethylamine or N-methylmorpholine in various solvents such as at -20 °C to 70 °C for 1 to 24 h. Convenient conditions are stirring the components in dichloromethane at room temperature for 18 h.

**Step B:** Cyclcondensation to form the oxadiazole can be accomplished by subjecting the N'-acyl-hydrazide to dehydrating conditions, such as reaction with trifluoromethanesulfonic anhydride and pyridine in a suitable solvent such as dichloromethane or thetrahydrofuran at 0 °C-40 °C for 1 h to 24 h.

Convenient conditions are the use of trifluoromethanesulfonic anhydride and pyridine in dichloromethane at room temperature for 18 h.

**Step C:** Reaction of the oxadiazole with an aniline R³NH₂ can be accomplished in a suitable solvent such as toluene, benzene or xylene in presence of a strong acid such as trifluoroacetic acid at elevated temperatures for 1 h to 24 h.

Convenient conditions are heating the components in toluene to 130 °C for 5 h to 24 h.

**Step D:** The deprotection step to form final compound **I** can be accomplished by various methods known to people skilled in the art depending on the group PG used. If PG = acetyl, treatment with an inorganic base such as potassium carbonate in methanol or ethanol at 20 °C to 80 °C for 30 min to 5 h can be used. If PG = benzyl, hydrogenation using hydrogen at atmospheric or elevated pressure and a heterogenous catalyst such as palladium on charcoal can be used.

A suitable protecting group is acetyl and convenient conditions are stirring with potassium carbonate in methanol at 65 °C for 45 min.

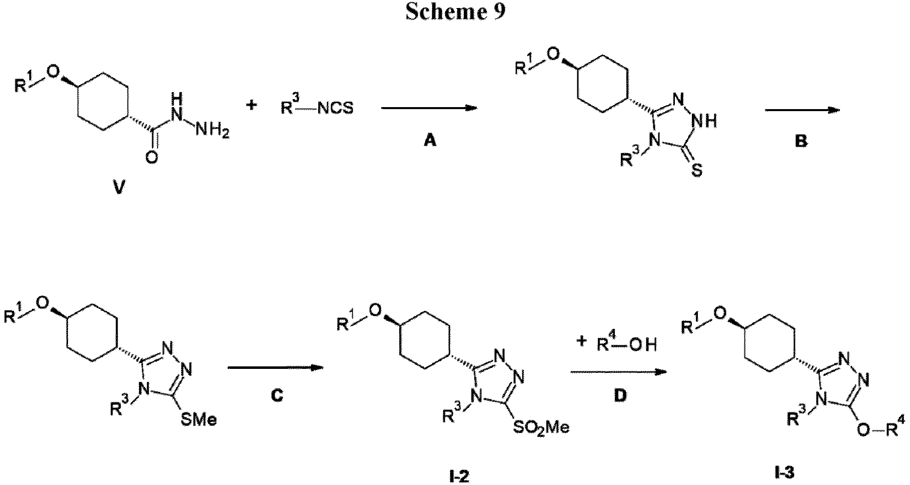

**Step A:** The reaction of a trans-4-(aryloxy)-cyclohexanecarboxylic acid hydrazide **V** with an isothiocyanate can be accomplished in a suitable solvent such as methanol, ethanol, n-propanol, n-butanol, dioxane or tetrahydrofuran at 25 °C to 100 °C for 15 min to 5 h, followed by heating the solid formed in an aqueous solution of sodium hydroxide, lithium hydroxide or potassium hydroxide at 60 °C to 100 °C for 15 min to 5 h.

Convenient conditions are heating the components in methanol to reflux for 1 h, followed by heating the solid formed in 1N sodium hydroxide solution to 100 °C for 1h.

**Step B:** S-Methylation can be accomplished by reaction of the triazole-3-thione with a methylating reagent, such as iodomethane or dimethyl sulfate and an inorganic base such as potassium carbonate, cesium carbonate or the like in a suitable solvent such as dimethylformamide, dimethylacetamide, tetrahydrofuran or dioxane at 0 °C to 70 °C for 1 h. Convenient conditions are the use of iodomethane and potassium carbonate in dimethylformamide at 25 °C for 18 h.

**Step C:** Oxidation to the methylsulfone **I-2** can be accomplished by reaction with a suitable oxygenation reagent such as 3-chloroperoxybenzoic acid in amounts >2 equivalents in a solvent such as dichloromethane at 0 °C to 40 °C for 1 h to 24 h.

Convenient conditions are the use of 4 equivalents of 3-chloroperoxybenzoic acid in dichloromethane at 25 °C for 18 h.

**Step D:** Synthesis of compound **I-3** can be accomplished by reacting compound **I-2** with an alcohol R⁴-OH and a base such as sodium hydride or potassium tert-butoxide in a suitable solvent such as tetrahydrofuran or acetonitrile at 0 °C to 40 °C for 1 h to 24 h.

Convenient conditions are the use of sodium hydride in tetrahydrofuran at 25 °C for 3 h.

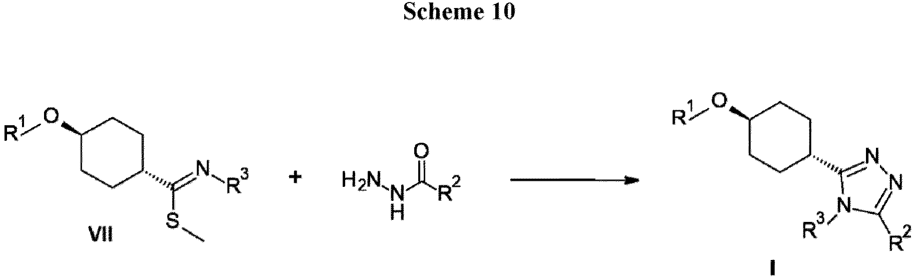

Formation of the triazoles **I** can be accomplished by reaction of an S-methylthioimidate **VII** with a hydrazide and an acid such as trifluoroacetic acid in a suitable solvent such as n-butanol, n-propanol or 1,4-dioxane at 50 °C to 130 °C for 1 h to 24 h.

Convenient conditions are heating the reactants with trifluoroacetic acid in n-butanol at 120 °C for 2 h.

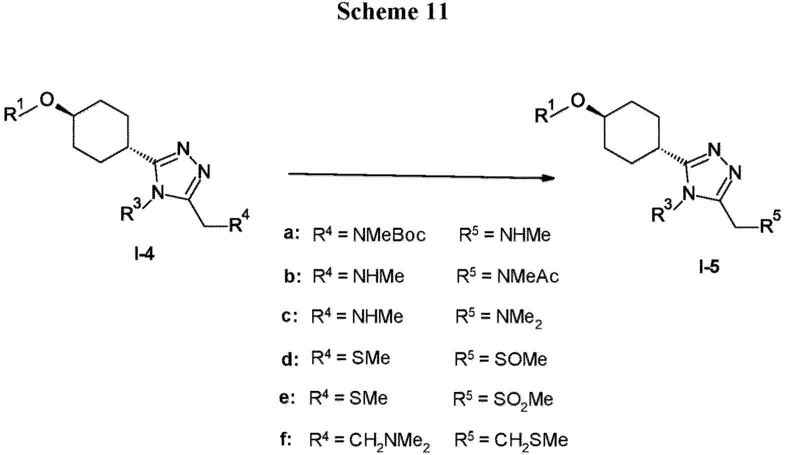

Various compounds of formula **I-5** can be synthesized by variations in the side chain starting from compounds **I-4:**

**Compound I-5a** can be synthesized by reaction of compound **I-4a** with an acid such as hydrochloric acid or trifluoroacetic acid in a suitable solvent, such as dioxane or dichloromethane.

Convenient conditions are the use of a hydrogen chloride solution in dioxane at 25 °C for 2.5 h.

**Compound I-5b** can be synthesized by reaction of compound **I-4b** with acetylchloride or acetanhydride and a base such as triethylamine or diisopropylethylamine in a suitable solvent such as tetrahydrofuran or dichloromethane at 0 °C to 40 °C for 1 h to 24 h.

Convenient conditions are the use of acetylchloride and triethylamine in dichloromethane at 25 °C for 2 h.

**Compound I-5c** can be synthesized by reaction of compound **I-4c** with formaldehyde in the presence of a reducing agent such as sodium triacetoxyborohydride with or without the addition of acetic acid and sodium acetate, or sodium cyanoborohydride in a suitable solvent such as dichloromethane, 1,2-dichloroethane or methanol at 0 °C to 40 °C for 5 h to 24 h.

Convenient conditions are the use of formaldehyde, acetic acid, anhydrous sodium acetate and sodium triacetoxyborohydride in dichloromethane at 25 °C for 24 h.

**Compound I-5d** can be synthesized by reaction of compound **I-4d** with 3-chloroperoxybenzoic acid (<1 equivalent) in a suitable solvent such as dichloromethane at 0 °C to 25 °C for 1 h to 24 h. The enantiomers formed can be separated by chiral HPLC.

Convenient conditions are the use of 0.95 eq. of 3-chloroperoxybenzoic acid in dichloromethane at 25 °C for 16 h. The enantiomers can be separated using chiral HPLC.

**Compound I-5e** can be synthesized by reaction of compound **I-4e** with 3-chloroperoxybenzoic acid (around 2 equivalents) in a suitable solvent such as dichloromethane at 0 °C to 25 °C for 1 h to 24 h.

Convenient conditions are the use of 1.95 eq. of 3-chloroperoxybenzoic acid in dichloromethane at 25 °C for 1 h.

**Compound I-5f can** be synthesized by reaction of compound **I-4f** with methyl sulfide and is formed during the synthesis of compound **I-4f** from the S-methylthioimidate because methyl sulfide occurs as by-product.

Conveniently, it is isolated by column chromatography as by-product from the synthesis of compound I-4f from the S-methylthioimidate as described in Scheme 10.

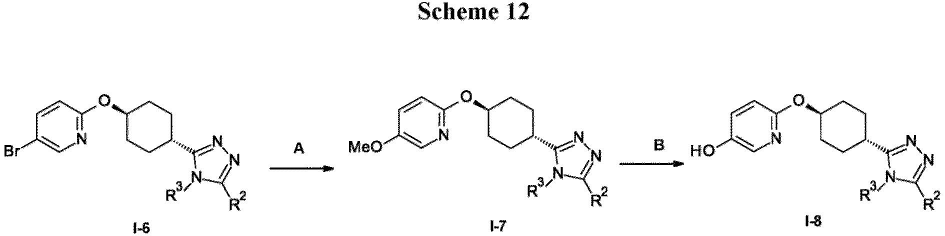

Step A: Formation of compound 1-7 can be accomplished by reaction with methanol, a base such as cesium carbonate or potassium carbonate, a copper salt such as copper(I)-iodide or copper(I)-bromide and a ligand such as 1,10-phenanthroline or 3,4,7,8-tetramethyl-1,10-phenanthroline in a suitable solvent such as toluene at elevated temperatures for 1 h to 50 h.

Convenient conditions are the use of cesium carbonate, copper(I)-iodide and 3,4,7,8-tetramethyl-1,10-phenanthroline in toluene as solvent at 85 °C for 45 h.

**Step B:** De-methylation to form compound **I-8** can be accomplished by reacting compound **I-7** with boron tribromide in a suitable solvent such as dichloromethane or chloroform at 0 °C to 25 °C for 30 min to 5 h.

Convenient conditions are the use of boron tribromide in dichloromethane at 25 °C for 90 min.

Starting from trans-configurated intermediates as shown in Schemes **4 - 12,** the trans-configurated compounds **I** are produced exclusively (or as major isomer, due to partial razemization taking place to a minor extent). If a cis-configurated compound **I** is desired, then a cis-configurated intermediate, available from cis-4-hydroxy-cyclohexanecarboxylic acid in a similar way as described can be used as starting material. The minor isomer which may be formed through the synthesis can be removed by standard purification steps as described in the experimental procedures, namely column chromatography or crystallization.

The corresponding pharmaceutically acceptable salts with acids can be obtained by standard methods known to the person skilled in the art, e.g. by dissolving the compound of formula I as described herein in a suitable solvent such as e.g. dioxane or tetrahydrofuran (THF) and adding an appropriate amount of the corresponding acid. The products can usually be isolated by filtration or by chromatography. The conversion of a compound of formula I into a pharmaceutically acceptable salt with a base can be carried out by treatment of such a compound with such a base. One possible method to form such a salt is e.g. by addition of 1/n equivalents of a basic salt such as e.g. M(OH)ₙ, wherein M = metal or ammonium cation and n = number of hydroxide anions, to a solution of the compound in a suitable solvent (e.g. ethanol, ethanol-water mixture, tetrahydrofuran-water mixture) and to remove the solvent by evaporation or lyophilization.

Insofar as their preparation is not described in the examples, the compounds of formula I as well as all intermediate products can be prepared according to analogous methods or according to the methods set forth herein. Starting materials are commercially available, known in the art or can be prepared by methods known in the art or in analogy thereto.

It will be appreciated that the compounds of general formula I in this invention may be derivatised at functional groups to provide derivatives which are capable of conversion back to the parent compound *in vivo.*

### Pharmacological Tests

The compounds of the present invention exhibit V1a activity. They are selective inhibitors of the V1a receptor and are therefore likely to have a low potential to cause unwanted off-target related side-effects. The V1a activity may be detected as described below.

The human V1a receptor was cloned by RT-PCR from total human liver RNA. The coding sequence was subcloned in an expression vector after sequencing to confirm the identity of the amplified sequence. To demonstrate the affinity of the compounds from the present invention to the human V1a receptor binding studies were performed. Cell membranes were prepared from HEK293 cells transiently transfected with the expression vector and grown in 20 liter fermenters with the following protocol.

50 g of cells are re-suspended in 30ml freshly prepared ice cold Lysis buffer (50 mM HEPES, 1mM EDTA, 10 mM MgCh adjusted to pH= 7.4 + complete cocktail of protease inhibitor (Roche Diagnostics)). Homogenized with Polytron for 1min and sonicated on ice for 2x 2 minutes at 80% intensity (Vibracell sonicator). The preparation is centrifuged 20 min at 500 g at 4 °C, the pellet is discarded and the supernatant centrifuged 1hour at 43'000g at 4 °C (19'000rpm). The pellet is re-suspended in 12.5 ml Lysis buffer+12.5ml Sucrose 20% and homogenized using a Polytron for 1-2 min. The protein concentration is determined by the Bradford method and aliquots are stored at -80 °C until use. For binding studies 60mg Yttrium silicate SPA beads (Amersham) are mixed with an aliquot of membrane in binding buffer (50 mM Tris, 120 mM NaCl, 5 mM KCl, 2 mM CaCl₂, 10 mM MgCh) for 15 minutes with mixing. 50µl of bead/membrane mixture is then added to each well of a 96 well plate, followed by 50 µl of 4 nM 3H-Vasopressin (American Radiolabeled Chemicals). For total binding measurement 100 µl of binding buffer are added to the respective wells, for non-specific binding 100 µl of 8.4 mM cold vasopressin and for compound testing 100 µl of a serial dilution of each compound in 2% DMSO. The plate is incubated 1h at room temperature, centrifuged 1 min at 1000 g and counted on a Packard Top-Count. Non-specific binding counts are subtracted from each well and data is normalized to the maximum specific binding set at 100%. To calculate an IC50 the curve is fitted using a non-linear regression model (XLfit) and the Kᵢ is calculated using the Cheng-Prussoff equation.

The following representative data show the antagonistic activity against human V₁ₐ receptor of compounds according to present invention.

**Table 1: pKi values of selected examples**

| **Ex #** | **pKi (hV1a)** | **Ex #** | **pKi (hV1a)** | **Ex #** | **pKi (hV1a)** | **Ex #** | **pKi (hV1a)** |
|---|---|---|---|---|---|---|---|
| 2 | 8.12 | 19 | 9.43 | 36 | 9.54 | 53 | 9.05 |
| 3 | 9.04 | 20 | 8.74 | 37 | 7.19 | 54 | 8.66 |
| 4 | 8.69 | 21 | 8.15 | 38 | 7.04 | 55 | 7.77 |
| 5 | 8.28 | 22 | 8.56 | 39 | 7.50 | 56 | 9.19 |
| 6 | 9.06 | 23 | 7.77 | 40 | 7.39 | 57 | 9.35 |
| 7 | 7.73 | 24 | 7.21 | 41 | 9.07 | 58 | 9.48 |
| 8 | 7.41 | 25 | 8.52 | 42 | 7.99 | 59 | 9.19 |
| 9 | 9.71 | 26 | 9.23 | 43 | 8.67 | 60 | 8.64 |
| 10 | 8.37 | 27 | 9.07 | 44 | 8.15 | 64 | 9.49 |
| 11 | 7.51 | 28 | 8.42 | 45 | 8.41 | 65 | 8.47 |
| 12 | 9.12 | 29 | 8.60 | 46 | 9.24 | 66 | 8.46 |
| 13 | 9.57 | 30 | 8.75 | 47 | 7.48 | 67 | 9.18 |
| 14 | 8.80 | 31 | 8.47 | 48 | 7.70 | 68 | 8.09 |
| 15 | 8.88 | 32 | 9.06 | 49 | 9.19 | 69 | 8.61 |
| 16 | 8.86 | 33 | 8.67 | 50 | 9.42 | 70 | 8.30 |
| 17 | 8.23 | 34 | 8.22 | 51 | 7.49 | 71 | 7.75 |
| 18 | 8.18 | 35 | 9.20 | 52 | 8.65 | 72 | 8.53 |

### Pharmaceutical Compositions

The compounds of formula I and the pharmaceutically acceptable salts can be used as therapeutically active substances, e.g. in the form of pharmaceutical preparations. The pharmaceutical preparations can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compounds of formula I and the pharmaceutically acceptable salts thereof can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also provided by the present invention, as is a process for their production, which comprises bringing one or more compounds of formula I and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration, the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, in particular 1-100 mg, of a compound of formula I. Examples of compositions according to the invention are:

### Example A

Tablets of the following composition are manufactured in the usual manner:

**Table 2: possible tablet composition**

| ingredient | mg/tablet | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50 °C.
3. Pass the granules through suitable milling equipment.
4. Add ingredient 5 and mix for three minutes; compress on a suitable press.

### Example B-1

Capsules of the following composition are manufactured:

**Table 3: possible capsule ingredient composition**

| ingredient | mg/capsule | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula I, lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 4: possible soft gelatin capsule ingredient composition**

| ingredient | mg/capsule |
|---|---|
| Compound of formula I | 5 |
| Yellow wax | 8 |
| Hydrogenated Soya bean oil | 8 |
| Partially hydrogenated plant oils | 34 |
| Soya bean oil | 110 |
| Total | 165 |

**Table 5: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula I is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 6: possible suppository composition**

| ingredient | mg/supp. |
|---|---|
| Compound of formula I | 15 |
| Suppository mass | 1285 |
| Total | 1300 |

### Manufacturing Procedure

The suppository mass is melted in a glass or steel vessel, mixed thoroughly and cooled to 45 °C. Thereupon, the finely powdered compound of formula I is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 7: possible injection solution composition**

| ingredient | mg/injection solution. |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure

The compound of formula I is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 8: possible sachet composition**

| ingredient | mg/sachet |
|---|---|
| Compound of formula I | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula I is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

The following table lists abbreviations used within the present document.

| | |
|---|---|
| brine | saturated sodium chloride solution in water |
| DMAP | 4-(N,N-dimethylamino)-pyridine |
| DMSO | dimethylsulfoxide |
| RT | room temperature |
| THF | tetrahydrofuran |

### Experimental Part

The following examples are provided for illustration of the invention. They should not be considered as limiting the scope of the invention, but merely as being representative thereof.

### 4-Hydroxy-cyclohexanecarboxylic acid ester intermediates of formula II

### General Procedure 1: Esterification

A solution of the 4-hydroxy-cyclohexanecarboxylic acid (10.0 g, 69.4 mmol) and 30 drops of concentrated sulfuric acid in methanol (700 ml) was heated at reflux over night. After cooling to room temperature solid sodium carbonate (0.15 g, 1.4 mmol) was added. The mixture was stirred for 30 min, filtered and concentrated in vacuo. The residue was triturated in ethyl acetate (150 ml). The solids were removed by filtration. The filtrate was concentrated in vacuo to give the crude product II which was used in the following steps without further purification.

### 4-Hydroxy-cyclohexanecarboxylic acid ester II-1 cis-4-Hydroxy-cyclohexanecarboxylic acid methyl ester

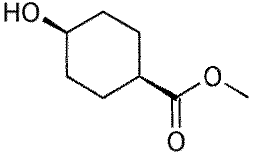

The title compound was obtained as colorless oil in 94% yield according to General Procedure 1.
Hydroxy-acid: cis-4-Hydroxy-cyclohexanecarboxylic acid

### 4-Hydroxy-cyclohexanecarboxylic acid ester II-2 trans-4-Hydroxy-cyclohexanecarboxylic acid methyl ester

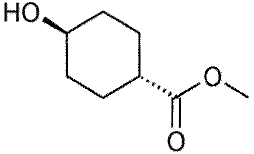

The title compound was obtained as colorless oil in 63% yield according to General Procedure 1.
Hydroxy-acid: trans-4-Hydroxy-cyclohexanecarboxylic acid
MS (EI) m/e: 159 (M⁺, 1%), 140 (M⁺-H₂0, 45%)

### 4-Aryloxy-cyclohexanecarboxylic acid ester intermediates of formula III

### General Procedure 2-I: Etherification under Mitsunobu conditions

To a solution of triphenylphosphine (1.2 eq) in dry tetrahydrofuran (0.1 M) is added diethyl azodicarboxylate (1.2 eq) at 0 °C. After 20 min a phenol derivative (1.2 eq) and a solution of a 4-hydroxy-cyclohexanecarboxylic acid ester of formula **II** in tetrahydrofuran (1-3 M) are added consecutively at 5 °C. After completed addition the cooling bath is removed and the reaction mixture is allowed to warm to room temperature and stirred for 3-18 h. The solvent is evaporated and the residue is dissolved in ethyl acetate. The ethyl acetate solution is washed with one to two portions of 1 M aqueous sodium hydroxide solution. The aqueous layer is extracted with one to two portions of ethyl acetate. The combined organic layers are dried over anhydrous sodium sulfate and concentrated in vacuo. Purification by flash-chromatography gives a 4-aryloxy-cyclohexanecarboxylic acid ester of formula **III.**

### General Procedure 2-II: Sodium methanesulfinate mediated arylation

To a solution of a 4-hydroxy-cyclohexanecarboxylic acid ester of formula **II** (1 eq) and a heteroaryl chloride derivative (1 eq) in dry N,N-dimethylformamide (1 M) are added consecutively sodium methanesulfinate (85%, 0.25 - 1 eq) and potassium carbonate (1.5 eq). After completed addition the reaction mixture is stirred at 120 °C for 3-18 h. After cooling to room temperature the reaction mixture is partitioned between tert-butyl methyl ether and water. The layers are separated and the aqueous layer is extracted with one to two portions of tert-butyl methyl ether. The combined organic layers are washed with one to two portions of water, dried over anhydrous sodium sulfate and concentrated in vacuo. Purification by flash-chromatography gives a 4-heteroaryloxy-cyclohexanecarboxylic acid ester of formula **III.**

### 4-Aryloxy-cyclohexanecarboxylic acid ester III-1 trans-4-Phenoxy-cyclohexanecarboxylic acid methyl ester

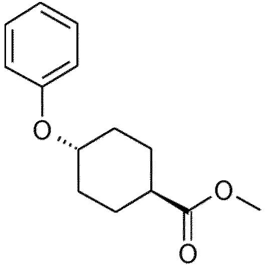

The title compound was obtained as colorless oil in 23% yield according to General Procedure 2-I.
Phenol: Phenol
4-Hydroxy-cyclohexanecarboxylic acid ester: cis-4-Hydroxy-cyclohexanecarboxylic acid methyl ester
MS m/e: 234 (M⁺)

### 4-Aryloxy-cyclohexanecarboxylic acid ester III-2 cis-4-Phenoxy-cyclohexanecarboxylic acid methyl ester

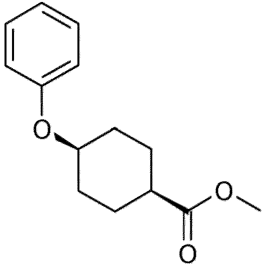

The title compound was obtained as colorless oil in 54% yield according to General Procedure 2-I.
Phenol: Phenol
4-Hydroxy-cyclohexanecarboxylic acid ester: trans-4-Hydroxy-cyclohexanecarboxylic acid methyl ester
MS m/e: 234 (M⁺)

### 4-Aryloxy-cyclohexanecarboxylic acid ester III-3 trans-4-(Pyridin-2-yloxy)-cyclohexanecarboxylic acid methyl ester

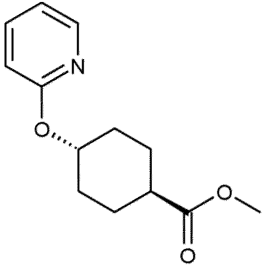

The title compound was obtained as light red solid in 36% yield according to General Procedure 2-1.
Phenol: 2-Hydroxypyridine
4-Hydroxy-cyclohexanecarboxylic acid ester: cis-4-Hydroxy-cyclohexanecarboxylic acid methyl ester
MS m/e: 236 (M+H⁺)

### 4-Aryloxy-cyclohexanecarboxylic acid ester III-4 cis/trans-4-(Pyrazin-2-yloxy)-cyclohexanecarboxylic acid ethyl ester (1:1)

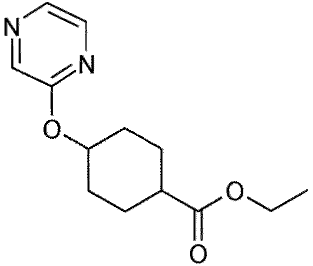

The title compound was obtained as white solid in 15% yield according to General Procedure 2-II.
Heteroaryl chloride : 2-Chloropyrazine
4-Hydroxy-cyclohexanecarboxylic acid ester: cis/trans-4-Hydroxy-cyclohexanecarboxylic acid ethyl ester ester (2 :1)
MS m/e: 251 (M+H⁺)

### 4-Aryloxy-cyclohexanecarboxylic acid ester III-5 trans-4-(6-Methylpyridin-3-yloxy)-cyclohexanecarboxylic acid methyl ester

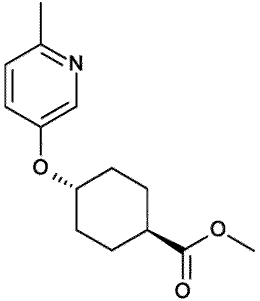

The title compound was obtained as red oil in 31% yield according to General Procedure 2-1.
Phenol: 6-Methylpyridin-3-ol
4-Hydroxy-cyclohexanecarboxylic acid ester: cis-4-Hydroxy-cyclohexanecarboxylic acid methyl ester
MS m/e: 250.2 (M+H⁺)

### 4-Aryloxy-cyclohexanecarboxylic acid ester III-6 trans-4-(5-Methylpyrazin-2-yl)oxycyclohexanecarboxylic acid methyl ester

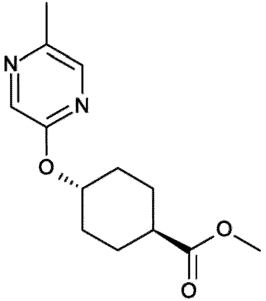

The title compound was obtained as white solid in 10% yield according to General Procedure 2-II.
Heteroaryl chloride: 2-Chloro-5-methylpyrazine
4-Hydroxy-cyclohexanecarboxylic acid ester: trans-4-Hydroxy-cyclohexanecarboxylic acid methyl ester
MS m/e: 251.1 (M+H⁺)

### 4-Aryloxy-cyclohexanecarboxylic acid ester III-7 trans-4-((5-Methylpyridin-2-yl)oxy)cyclohexanecarboxylic acid methyl ester

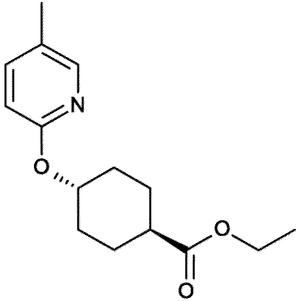

The title compound was obtained as colorless oil in 30% yield according to General Procedure 2-I.
Phenol: 5-Methylpyridin-2-ol
4-Hydroxy-cyclohexanecarboxylic acid ester: cis-4-Hydroxy-cyclohexanecarboxylic acid ethyl ester
MS m/e: 264.1 (M+H⁺)

### 4-Aryloxy-cyclohexanecarboxylic acid intermediates of formula IV

### General Procedure 3

A 100 ml three-necked round-bottomed flask which had been dried and cooled under Ar-stream, was charged with sodium tert-pentoxide (3.73 g, 33.8 mmol, eq: 2.44) and N-methyl-2-pyrrolidinone (34 ml). The reaction mixture was heated to 90 °C followed by addition of trans-4-hydroxycyclohexanecarboxylic acid (2 g, 13.9 mmol, eq: 1.00) as solution in N-methyl-2-pyrrolidinone (7 ml). The reaction mixture was stirred for 10 min. After addtion of the heteroaryl halide (eq: 1.2) the reaction mixture was stirred for 15 h to 4 days at 90°C. The reaction mixture was poured on ice/water (50 ml) and set to pH 1-2 by addition of 2 M aq HCl (13.9 ml, 27.7 mmol, eq: 2). The mixture was stirred for 1 h at 0-5 °C. The precipitate was collected by filtration, washed with ice/water (pH adjusted to 1 by addition of 2 M HCl) and dried in vacuo. The crude material was purified by flash chromatography (SiO2, n-heptane/ethyl acetate). After removal of the solvent the residue was triturated with hot n-heptane. The solid was collected by filtration, washed with n-heptane and dried in vacuo to give the 4-aryloxy-cyclohexanecarboxylic acid intermediate of formula **IV.**

### 4-Aryloxy-cyclohexanecarboxylic acid IV-1 trans-4-(5-Bromopyridin-2-yloxy)cyclohexanecarboxylic acid

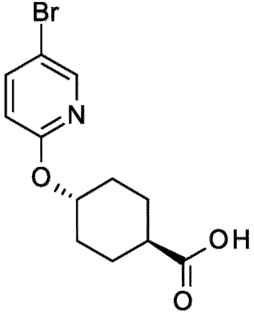

The title compound was obtained as pink solid in 38% yield according to the General Procedure 3.
Heteroaryl halide: 5-Bromo-2-fluoropyridine
MS m/e: 300; 302 (M⁺)

### 4-Aryloxy-cyclohexanecarboxylic acid IV-2 trans-4-((5-Difluoromethylpyridin-2-yl)oxy)-cyclohexanecarboxylic acid

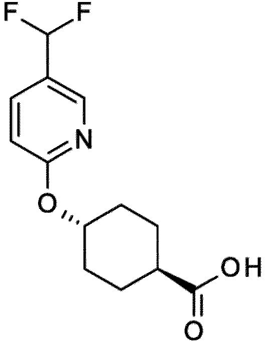

The title compound was obtained as white solid in 3% yield according to the General Procedure 3.
Heteroaryl halide: 2-chloro-5-(difluoromethyl)pyridine
MS m/e: 272 (M⁺)

### Hydrazide intermediates of formula V

### General Procedure 4-I: Conversion of an ester of formula III to a hydrazide of formula V

A mixture of a 4-(aryloxy)-cyclohexanecarboxylic acid ester of formula **III** (1 eq) and hydrazine hydrate (5 eq) is heated at 120 °C for 5 h. After cooling to room temperature the reaction mixture is partitioned between dichloromethane and water. The layers are separated and the organic layer is washed with water and brine. The organic layer is dried over anhydrous sodium sulfate and concentrated in vacuo to give a crude hydrazide of formula V, which is used in the next step without further purification.

### General Procedure 4-II: Conversion of an acid of formula IV to a hydrazide of formula V

To a solution of a 4-(aryloxy)-cyclohexanecarboxylic acid of formula **IV** (1 eq) and triethylamine (1.2 eq) in tetrahydrofuran is added isobutyl chloroformate or ethyl chloroformate (1.2 eq) at 0-5 °C. The reaction mixture is stirred for 1 h at 0-5 °C. The salts are removed by filtration. The filtrate is added to a cold solution of hydrazine hydrate (2.5 eq) in methanol at 0-5 °C. The reaction mixture is stirred at room temperature until completion of the reaction. The reaction mixture is diluted with water. The solvents are evaporated, and the residue is triturated with solvent and dried in vacuo to give a hydrazide of formula **V.**

### Hydrazide V-1 trans-4-Phenoxy-cyclohexanecarboxylic acid hydrazide

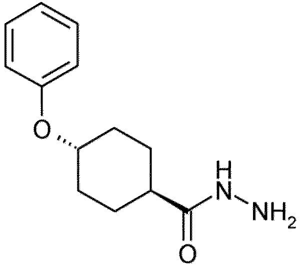

According to General Procedure 4-I a mixture of trans-4-phenoxy-cyclohexanecarboxylic acid methyl ester (1.07 g, 4.57 mmol) and hydrazine hydrate (0.22 g, 4.48 mmol) was heated at 120 °C for 4 h. After cooling to room temperature the reaction mixture was concentrated in vacuo to give the crude title compound as white solid in quantitative yield, which was used in the next step without further purification.
MS m/e: 235 (M+H⁺).

### Hydrazide V-2 cis-4-Phenoxy-cyclohexanecarboxylic acid hydrazide

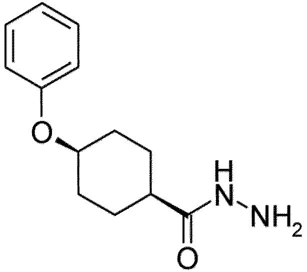

According to General Procedure 4-I a mixture of cis-4-phenoxy-cyclohexanecarboxylic acid methyl ester (0.55 g, 2.3 mmol) and hydrazine hydrate (0.11 g, 2.3 mmol) was heated at 120 °C for 24 h. After cooling to room temperature the reaction mixture was suspended in toluene (70 ml). After evaporation of the solvent, the residue was suspended in toluene (70 ml) again. The solvent was evaporated and the residue was dried in high vacuo (1-2 mbar) to give the crude title compound (0.50 g, 92%) as light yellow foam, which was used in the next step without further purification.
MS m/e: 235 (M+H⁺).

### Hydrazide V-3 trans-4-(Pyridin-2-yloxy)-cyclohexanecarboxylic acid hydrazide

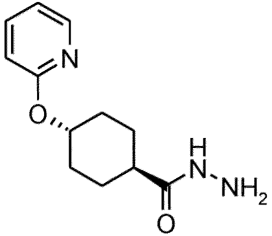

According to General Procedure 4-I a mixture of trans-4-(pyridin-2-yloxy)-cyclohexanecarboxylic acid methyl ester (1.33 g, 5.66 mmol) and hydrazine hydrate (0.55 ml, 11 mmol) in n-butanol (1 ml) was heated at 120 °C for 68 h. After cooling to room temperature the reaction mixture was evaporated and dried in high vacuo (ca. 1-2 mbar) at 100 °C for 2 h to give the crude title compound (1.28 g, 96%) as white solid, which was used in the next step without further purification.
MS m/e: 236 (M+H⁺)

### Hydrazide V-4 trans-4-(Pyrazin-2-yloxy)-cyclohexanecarboxylic acid hydrazide

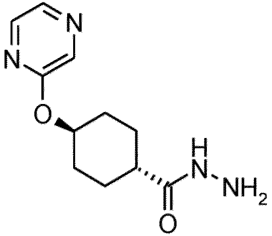

According to General Procedure 4-I a mixture of cis/trans-4-(pyrazin-2-yloxy)-cyclohexanecarboxylic acid ethyl ester (1.11 g, 4.41 mmol) and hydrazine hydrate (0442 g, 8.83 mmol) was heated at 120 °C for 72 h. After cooling to room temperature the reaction mixture was partitioned between ethyl acetate (50 ml) and water (30 ml). The organic layer was separated. The aqueous layer was extracted with two 50.ml portions of ethyl acetate. The combined organic layers were washed with brine (30 ml), dried over anhydrous sodium sulfate and concentrated in vacuo. The crude cis/trans-hydrazide was triturated in ethyl acetate (5 ml). The precipitate was collected by filration and dried in vacuo to give the crude title compound (0.236 g, 23%) as white solid, which was used in the next step without further purification.
MS m/e: 237 (M+H⁺)

### Hydrazide V-5 trans-4-((6-Methylpyridin-3-yl)oxy)-cyclohexanecarboxylic acid hydrazide

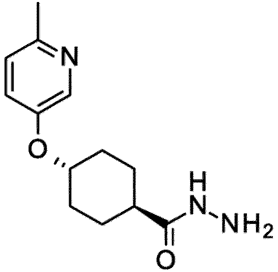

According to General Procedure 4-I a mixture of trans-4-(6-methylpyridin-3-yloxy)-cyclohexanecarboxylic acid methyl ester (15 g, 60.2 mmol) and hydrazine hydrate (103 ml, 1.81 mol) in methanol (30 ml) was heated at 75 °C for 10 h. After cooling to room temperature the reaction mixture was concentrated to give crude yellow solid. The crude product was triturated by methanol and dried to give the title compound (5.1 g, 16.6%) as white solid, which was used in the next step without further purification.
MS m/e: 250.2 (M+H⁺)

### Hydrazide V-6 trans-4-(5-Methylpyrazin-2-yl)oxycyclohexanecarboxylic acid hydrazide

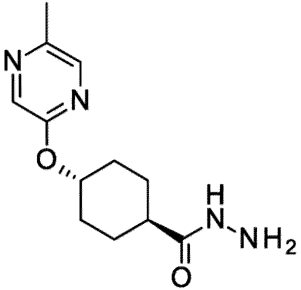

According to General Procedure 4-I a mixture of trans-4-(5-methylpyrazin-2-yl)oxycyclohexanecarboxylic acid methyl ester (2.0 g, 8.0 mmol) and hydrazine hydrate (1.55 ml, 32 mmol) in n-butanol (8 ml) was heated at 130 °C for 15 h. The reaction mixture was cooled to room temperature and stirred 30 min. The solids was collected by filtration, washed with ethyl acetate and dried in vacuo to yield the title compound (1.69 g, 80%) as white solid, which was used in the next step without further purification.
MS m/e: 251 (M+H⁺)

### Hydrazide V-7 trans-4-((5-Methylpyridin-2-yl)oxy)-cyclohexanecarboxylic acid hydrazide

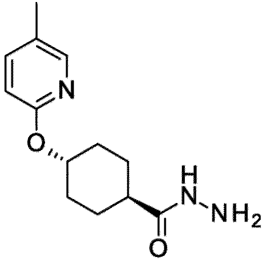

According to General Procedure 4-I a mixture of trans-4-((5-methylpyridin-2-yloxy)-cyclohexanecarboxylic acid methyl ester (1.37 g, 5.2 mmol) and hydrazine hydrate (1.03 ml, 20.8 mmol) in n-butanol (20 ml) was heated at 120 °C for 50 h. After cooling to room temperature the reaction mixture was evaporated and dried in high vacuo (ca. 1-2 mbar) at 100 °C for 2 h to give the crude product. This was then triturated with 10 ml of methyl tert-butyl ether to yield the title compound (0.79 g, 61%) as white solid, which was used in the next step without further purification.
MS m/e: 250 (M+H⁺)

### Hydrazide V-8 trans-4-(5-Bromopyridin-2-yloxy)cyclohexanecarboxylic acid hydrazide

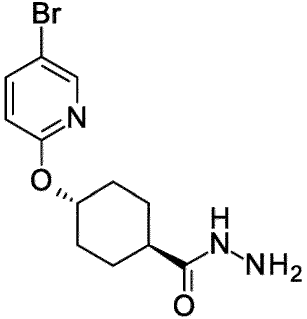

According to General Procedure 4-11: to a solution of trans-4-(5-bromopyridin-2-yloxy)cyclohexanecarboxylic acid (1.58 g, 5.26 mmol) and triethylamine (639 mg, 880 µl, 6.32 µmol) in tetrahydrofuran (26 ml) was added isobutyl chloroformate (863 mg, 830 µl, 6.32 µmol) at 0-5 °C. The reaction mixture was stirred for 1 h at 0-5 °C. The salts were removed by filtration. The filtrate was added to a cold solution of hydrazine hydrate (672 mg, 651 µl, 13.2 mmol) in methanol (26 ml) at 0-5 °C. The reaction mixture was stirred for 2 h at room temperature. The reaction mixture was was diluted with water (10 ml). The solvents were evaporated. The residue was triturated in hot water (60 ml). The precipitate was collected by filtration, washed with water and dried in vacuo to give the title compound (1.4 g, 85%) as white solid.
MS m/e: 314; 316 (M+H⁺)

### Hydrazide V-9 trans-4-((5-Difluoromethylpyridin-2-yl)oxy)-cyclohexanecarboxylic acid hydrazide

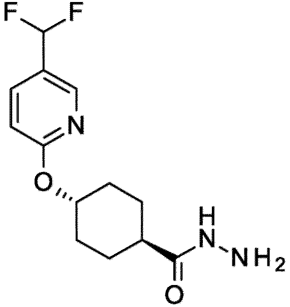

According to General Procedure 4-11: to a solution of trans-4-((5-(difluoromethyl)pyridin-2-yl)oxy)cyclohexanecarboxylic acid (31 mg, 114 µmol) and triethylamine (12.7 mg, 17.4 µl, 126 µmol) in tetrahydrofuran (1.5 ml) was added ethyl chloroformate (13.6 mg, 12 µl, 126 µmol) at 0-5 °C. The reaction mixture was stirred for 1 h at 0-5 °C. The salts were removed by filtration. The filtrate was added to a cold solution of hydrazine hydrate (11.7 mg, 11.3 µl, 229 µmol) in methanol (1 ml) at 0-5 °C. The reaction mixture was stirred for 4 days at room temperature. The reaction mixture was partitioned between ethyl acetate (10 ml) and water/brine (1:1) (5 ml). The layers were separated. The aqueous layer was extracted with two 10 ml portions of ethyl acetate. The combined organic layers were washed with one 5 ml portion of brine, dried over anhydrous sodium sulfate, filtered and concentrated in vacuo. The residue was triturated in TBME/n-heptane (2 ml, 1:1). The solids was collected by filtration, washed with n-heptane and dried in vacuo to yield the title compound (18 mg, 52%) as a white solid.
MS m/e: 286 (M+H⁺)

### Imidazole intermediates of formula VI

### General Procedure 5:

To a ca. 0.22 M solution of the corresponding amide (1 eq) in acetonitrile is added imidazole (12 eq) at room temperature. The reaction mixture is cooled to 0-5 °C prior to the dropwise addition of phosphorus oxychloride (2 eq). The reaction mixture is allowed to stir for 30 min at 0-5 °C. After removal of the ice bath the reaction mixture is heated to 68 °C for ca. 16 h. After cooling the reaction mixture to room temperature the solids are removed by filtration and washed with acetonitrile. The filtrate is concentrated in vacuo. The residue is partitioned between ethyl acetate and water, and the layers are separated. The aqueous layer is extracted with three portions of ethyl acetate. The combined organic layers are washed with one portion of water, one portion of saturated ammonium chloride solution, and one portion of brine. The combined organic layers are dried over anhydrous sodium sulfate, filtered and concentrated in vacuo. The crude product is triturated with a mixture of ethyl acetate and n-heptane. The solids are collected by filtration, washed with n-hexane and dried in vacuo to give an imidazole intermediate of formula IV.

### Imidazole VI-1 N-(1-(1H-Imidazol-1-yl)ethylidene)-4-chloroaniline

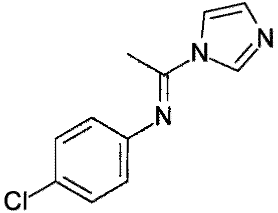

The title compound was obtained as white solid in 76% yield according to the General Procedure 5.
Amide: N-(4-chlorophenyl)acetamide
MS m/e: 220 (M+H⁺)

### Imidazole VI-2 N-(1-(1H-Imidazol-1-yl)ethylidene)-4-fluoroaniline

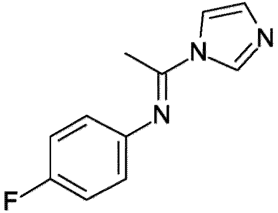

The title compound was obtained as off-white solid in 99% yield according to the General Procedure 5.
Amide: N-(4-fluorophenyl)acetamide
MS m/e: 204 (M+H⁺)

### Imidazole VI-3 N-(1-(1H-Imidazol-1-yl)ethylidene)aniline

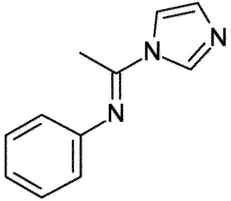

The title compound was obtained as off-white solid in 92% yield according to the General Procedure 5.
Amide: acetanilide
MS m/e: 186 (M+H⁺)

### Imidazole VI-4 N-(1-(1H-Imidazol-1-yl)ethylidene)-4-methylaniline

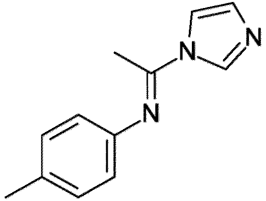

The title compound was obtained as white solid in 78% yield according to the General Procedure 5.
Amide: 4'-Methylacetaniline
MS m/e: 200 (M+H⁺)

### Imidazole VI-5 N-(1-(1H-Imidazol-1-yl)ethylidene)-4-(difluoromethyl)aniline

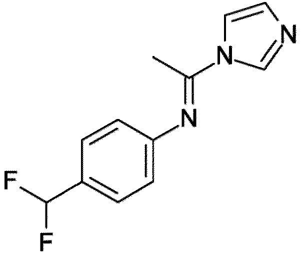

The title compound was obtained as brown solid in 66% yield according to the General Procedure 5.
Amide: N-(4-(difluoromethyl)phenyl)acetamide
MS m/e: 236 (M+H⁺)

### Imidazole VI-6 N-(1-(1H-Imidazol-1-yl)ethylidene)-4-cyanoaniline

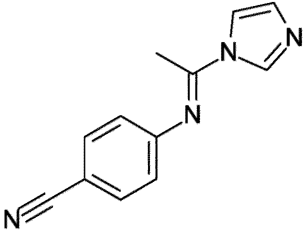

The title compound was obtained as off-white solid in 62% yield according to the General Procedure 5.
Amide: N-(4-cyanophenyl)acetamide
MS m/e: 211 (M+H⁺)

### Imidazole VI-7 N-(1-(1H-Imidazol-1-yl)ethylidene)-4-bromoaniline

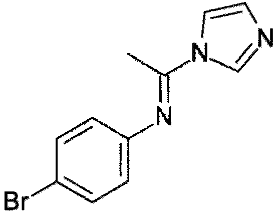

The title compound was obtained as off-white solid in 94% yield according to the General Procedure 5.
Amide: N-(4-bromophenyl)acetamide
MS m/e: 264; 266 (M+H⁺)

### Imidazole VI-8 N-(1-(1H-Imidazol-1-yl)ethylidene)-4-chloro-2-methylaniline

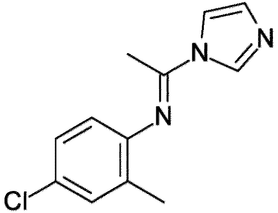

The title compound was obtained as off-white solid in 96% yield according to the General Procedure 5.
Amide: N-(4-chloro-2-methylphenyl)acetamide
MS m/e: 233 (M+H⁺)

### Imidazole VI-9 N-(4-Chlorophenyl)-1-(1H-imidazol-1-yl)-2-(methylthio)ethan-1-imine

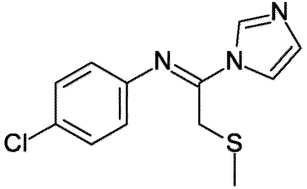

The title compound was obtained as yelow oil in 63% yield according to the General Procedure 5.
Amide: N-(4-chlorophenyl)-2-(methylthio)acetamide (CAS# 1252439-07-8)
MS m/e: 266 (M+H⁺)

### Imidazole VI-10 N-(1-(1H-Imidazol-1-yl)-2-methoxyethylidene)-4-chloroaniline

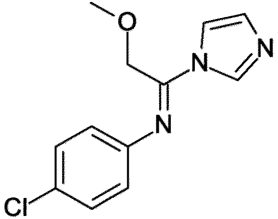

The title compound was obtained as light yellow solid in 51% yield according to the General Procedure 5.
Amide: N-(4-chlorophenyl)-2-methoxyacetamide
MS m/e: 248; 250 (M+H⁺)

### Imidazole VI-11 N-(1-(1H-Imidazol-1-yl)ethylidene)-4-chloro-3-fluoroaniline

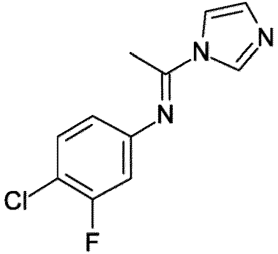

The title compound was obtained as light yellow solid in 56% yield according to the General Procedure 5.
Amide: N-(4-chloro-3-fluorophenyl)acetamide
MS m/e: 239 (M+H⁺)

### Imidazole VI-12 N-(1-(1H-Imidazol-1-yl)propylidene)-4-chloroaniline

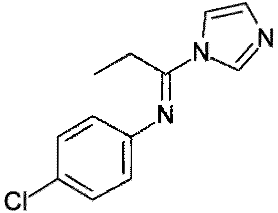

The title compound was obtained as white solid in 37% yield according to the General Procedure 5.
Amide: N-(4-chlorophenyl)propionamide
MS m/e: 233 (M+H⁺)

### Imidazole VI-13 N-(1-(1H-Imidazol-1-yl) ethylidene)-4-methoxyaniline

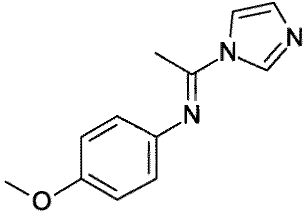

The title compound was obtained as white solid in 90% yield according to the General Procedure 5.
Amide: N-(4-methoxyphenyl)acetamide
MS m/e: 216 (M+H⁺)

### Imidazole VI-14 N-(1-(1H-Imidazol-1-yl) ethylidene)-3-chloroaniline

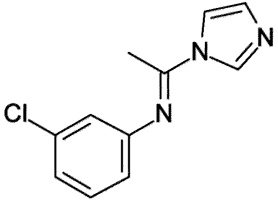

The title compound was obtained as white solid in 97% yield according to the General Procedure 5.
Amide: N-(3-chlorophenyl)acetamide
MS m/e: 220 (M+H⁺)

### Imidazole VI-15 N-(1-(1H-Imidazol-1-yl) ethylidene)-4-chloro-2-fluoroaniline

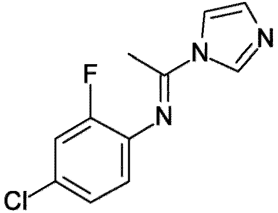

The title compound was obtained as white solid in 97% yield according to the General Procedure 5.
Amide: N-(4-chloro-2-fluorophenyl)acetamide
MS m/e: 238 (M+H⁺)

### Imidazole VI-16 N-(1-(1H-Imidazol-1-yl) ethylidene)-3-fluoro-4-methylaniline

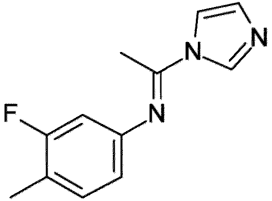

The title compound was obtained as an off-white solid in 73% yield according to the General Procedure 5.
Amide: N-(3-fluoro-4-methylphenyl)acetamide
MS m/e: 218 (M+H⁺)

### Imidazole VI-17 N-(1-(1H-Imidazol-1-yl)ethylidene)-5-chloropyridin-2-amine

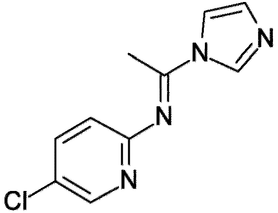

The title compound was obtained as yellow solid in 29% yield according to the General Procedure 5.
Amide: N-(5-chloropyridin-2-yl)acetamide
MS m/e: 221 (M+H⁺)

### Imidazole VI-18 N-(1-(1H-Imidazol-1-yl)ethylidene)-5-chloro-3-fluoropyridin-2-amine

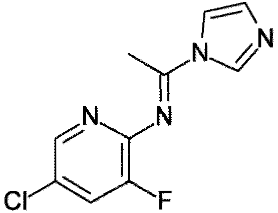

The title compound was obtained as an off-white solid in 59% yield according to the General Procedure 5.
Amide: N-(5-chloro-3-fluoropyridin-2-yl)acetamide
MS m/e: 239 (M+H⁺)

### Imidazole VI-19 N-(1-(1H-Imidazol-1-yl)ethylidene)-5-chlorothiophen-2-amine

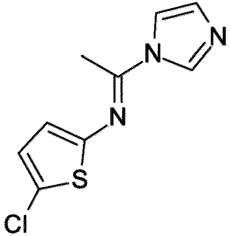

The title compound was obtained as yellow solid in 62% yield according to the General Procedure 5.
Amide: N-(5-chlorothiophen-2-yl)acetamide
MS m/e: 226 (M+H⁺)

### Imidazole VI-20 N-(1-(1H-Imidazol-1-yl)-2-methylprop-1-en-1-yl)-4-chloroaniline

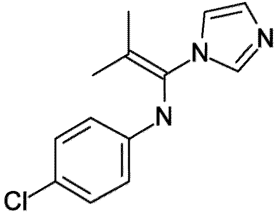

The title compound was obtained as yellow oil in 13% yield according to the General Procedure 5.
Amide: N-(4-chlorophenyl)isobutyramide
MS m/e: 248 (M+H⁺)

### Imidazole VI-21 N'-(4-Chlorophenyl)-N,N-dimethyl-1H-imidazole-1-carboximidamide

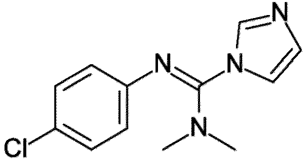

The title compound was obtained as light yellow solid in 90% yield according to the General Procedure 5.
Amide: 3 -(4-chlorophenyl)-1,1 -dimethylurea
MS m/e: 249 (M+H⁺)

### Imidazole VI-22 1-(1H-Imidazol-1-yl)-2-methoxy-N-(p-tolyl)ethan-1-imine

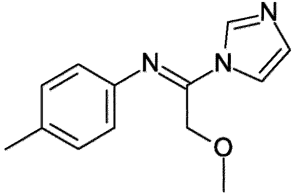

The title compound was obtained as yellow oil in 28% yield according to the General Procedure 5.
Amide: 2-methoxy-N-(p-tolyl)acetamide
MS m/e: 230 (M+H⁺)

### S-Methylthioimidate Intermediate VII-1 trans-Methyl N-(4-chlorophenyl)-4-(pyridin-2-yloxy)cyclohexane-1-carbimidothioate

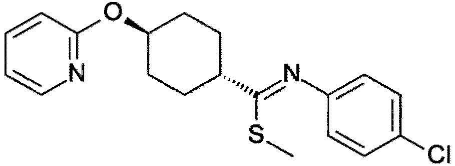

### a) trans-4-(Pyridin-2-yloxy)cyclohexanecarboxylic acid

To a solution of sodium tert-pentoxide (17,3 g, 157 mmol, eq: 2.44) in N-methyl-2-pyrrolidinone (100 ml) at 90 °C was added trans-4-hydroxycyclohexanecarboxylic acid (CAS 3685-26-5; 9.3 g, 64,5 mmol, eq: 1,00) dissolved in N-methyl-2-pyrrolidinone (30 ml) in approximately 20 min resulting in a thick suspension. 2-Chloropyridine (8,79 g, 7,26 ml, 77,4 mmol, eq: 1.2) was added, and the reaction mixture was stirred for 96 h at 90 °C. After cooling the reaction mixture was poured into water and set to pH 4-5 by addition of hydrochloric acid (2M, 55.5 ml, 111 mmol, eq: 1.72). The suspension was stirred for 2 h, then the precipitate was collected by filtration, washed with ice/water (200 ml, in 3 portions) and dried in vacuo to give 9.11 g (64%) of the title compound as off-white solid. MS m/e: 220 (M-H⁺)

### b) trans-N-(4-Chlorophenyl)-4-(pyridin-2-yloxy)cyclohexanecarboxamide

To a solution of trans-4-(pyridin-2-yloxy)cyclohexane-1-carboxylic acid (5 g, 22.6 mmol, eq: 1) in dichloromethane (200 ml) was added oxalyl chloride (3.44 g, 2.37 ml, 27.1 mmol, eq: 1.20) at 0-5 °C and subsequently a catalytic amount of DMF. The cooling bath was removed after 30 min, and stirring was continued for 2 h. The reaction mixture was concentrated in vacuo, and the residue was dissolved in dichloromethane (200 ml). At 0-5 °C 4-chloroaniline (2.85 g, 22.4 mmol, eq: 0.99) was added in one portion and subsequently triethylamine (4.8 g, 6.58 ml, 47.5 mmol, eq: 2.1). A catalytic amount of DMAP was added and the reaction mixture was stirred for 30 min at 0-5 °C. The ice bath was removed and stirring was continued at room temperature for 1 h. The reaction mixture was partitioned between dichloromethane (200 ml) and 0.5 M aqueous sodium hydroxide solution (200 ml and the layers were separated. The aqueous layer was extracted with three 150-ml portions of dichloromethane. The combined organic layers were washed with one 200-ml portion of saturated ammonium chloride solution, two 150-ml portions of water and one 200-ml portion of brine, dried over anhydrous sodium sulfate, filtered and concentrated in vacuo. The residue was triturated in n-heptane/ethyl acetate 9:1 (300-ml). The solids were collected by filtration, washed with n-heptane and dried in vacuo to give 6.76 g (86%) of the title compound as off-white solid. MS m/e: 331 (M+ H⁺)

### c) trans-N-(4-Chlorophenyl)-4-(pyridin-2-yloxy)cyclohexanecarbothioamide

A solution of trans-N-(4-chlorophenyl)-4-(pyridin-2-yloxy)cyclohexane-1-carboxamide (6.5 g, 19.6 mmol, eq: 1) and Lawesson's reagent (4.77 g, 11.8 mmol, eq: 0.6) in tetrahydrofuran (100 ml) was heated at reflux and stirred for 3 h. After cooling to room temperature the reaction mixture was concentrated in vacuo and the residue was purified by flash chromatography (silica, 0-100% ethyl acetate in heptane) to yield 4.4 g (61%) of the title compound as off-white solid. MS m/e: 347 (M+ H⁺)

### d) trans-Methyl N-(4-chlorophenyl)-4-(pyridin-2-yloxy)cyclohexane-1-carbimidothioate

To a solution of trans-N-(4-chlorophenyl)-4-(pyridin-2-yloxy)cyclohexane-1-carbothioamide (4.40 g, 12.7 mmol, eq: 1.00) in tetrahydrofuran (180 ml) was added potassium tert-butoxide (1.45 g, 12.9 mmol, eq: 1.02) at room temperature. The reaction mixture was stirred for 10 min. Methyl 4-methylbenzenesulfonate (2.42 g, 13 mmol, eq: 1.02) was added to give a suspension. Stirring was continued at room temperature for 1 h. For work up the reaction mixture was concentrated in vacuo. The residue was partitioned between dichloromethane (200 ml) and water (150 ml), and the layers were separated. The aqueous layer was extracted with one 200-ml portion of dichloromethane. The combined organic layers were washed with one 150-ml portion of water and one 125-ml portion of brine, dried over anhydrous sodium sulfate, filtered and concentrated in vacuo. The residue was purified by flash chromatography (silica, 0-15% ethyl acetate in heptane) to give 4.2 g (87%) of the title compound as white solid. MS m/e: 361 (M+H⁺)

### Examples

### General Procedure I:

To a solution of a corresponding thioamide (1 eq) in DMF (0.3 M) potassium carbonate (2 eq) is added, followed by iodomethane (1 eq) within 10 minutes at room temperature. After stirring over night methyl tert-butyl ether and water are added. The layers are separated and the aqueouos layer is extracted with two portions of methyl tert-butyl ether. The combined organic layers are dried over potassium sulfate and concentrated in vacuo. The residue is dissolved in butanol (10 ml for 1 mmol reagent), a hydrazide of formula V (1 eq) is added and the mixture is heated to 120 °C over night. For work-up toluene is added and the solvents are removed in vacuo. The residue is purified by column chromatography to yield a compound of formula I.

### General Procedure II:

To a 0.25 M suspension of a hydrazide of forumla V (1 eq) and an imidazole of formula **VI** (1.2 eq) in dry tetrahydrofuran is added dropwise trifluoroacetic acid (2 eq) at 0-5 °C. The ice bath is removed after 30 min, and stirring is continued for 16 h. For work up the reaction mixture is concentrated in vacuo, and the residue is partitioned between ethyl acetate and 1 M aqueous sodium hydroxide solution. The layers are separated. The aqueous layer is extracted with two portions of ethyl acetate. The combined organic layers are washed with one portion of 1 M aqueous sodium hydroxide solution, one portion of saturated ammonium chloride solution, and one portion of brine. The combined organig layers are dried over anhydrous sodium sulfate, filtered and concentrated in vacuo. The crude product is dissolved in ethyl acetate at reflux. The solution is allowed to cool to room temperature and stirred at room temperature for 3 h. The precipitate is collected by filtration, washed with n-hexane and dried in vacuo to give a compound of formula **I.**

### General Procedure III:

A mixture of a hydrazide of formula V (1 eq) and a corresponding isothiocyanate (1 eq) in ethanol (0.3M) is heated at reflux for 1 hr. After cooling the solid is filtered off, washed with ethanol and dried. The residue is dissolved in 1N sodium hydroxide solution and heated at reflux for 1 h. After cooling the mixture is acidfied to pH=6 with 1N HCl. The precipitate formed is washed with water and dried. The solid is dissolved in dichloromethane. At 0 °C a 1.9 M solution of hydrogen peroxide (2.5 eq) in acetic acid is added dropwise, and the mixture is stirred at room temperature for 1 h. For workup it is diluted with dichloromethane and washed with 1 N sodium hydroxide solution. The organic phase is dried with sodium sulfate and concentrated in vacuo. The crude product obtained is washed with ethylacetate and dried in vacuo to give a compound of formula **I.**

### General Procedure IV:

To a mixture of a hydrazide of formula V (1 eq), N-methylmorpholine (1.1 eq) and dichloromethane (0.5M) at 0 °C is added dropwise a carboxylic acid anhydride or an acid chloride (1.05 eq). The mixture is allowed to stir over night at room temperature. The solid is filtered off, and the filtrate is concentrated in vacuo to yield the crude N'-carboxyhydrazide intermediate. After chromatographic purification this corresponding intermediate (1 eq) is dissolved in dichloromethane (0.2M), followed by dropwise addition of pyridine (4 eq) and trifluoromethanesulfonic anhydride (2.5 eq) at 0 °C. The reaction mixture is stirred at room temperature over night, diluted with dichloromethane and successively washed with 2N HCl and saturated sodium bicarbonate solution. The organic layer is dried and concentrated in vacuo to give a corresponding oxadiazole intermediate which is purified by chromatography before the next step. After dissolving this corresponding intermediate (1 eq) in toluene (0.1M), an aromatic amine (1.5 eq) and trifluoroacetic acid (0.5 eq) are added, and the mixture is heated at 130 °C in a closed tube for 5 to 24 h. For workup the solvent is evaporated and the residue is purified by column chromatogaphy to yield a compound of formula **I.**

### General Procedure V:

To a 0.7 M solution of an alcohol (3 eq) in tetrahydrofuran (0.6 ml) is added sodium hydride (60% in mineral oil, 2.5 eq) and the reaction mixture is stirred at room temperature for 15 min. After adding trans-2-((4-(4-(4-chlorophenyl)-5-(methylsulfonyl)-4H-1,2,4-triazol-3-yl)cyclohexyl)oxy)pyridine (1 eq) the reaction mixture is stirred at room temperature for 3 hours. The mixture is diluted with ethyl acetate and washed with water. The aqueous layer is extracted with ethylaceate. The combined organic layers are dried over sodium sulfate, filtered and concentrated in vacuo. The crude product is purified by chromatography (silica gel, dichloromethane/methanol 10%) to yield a compound of formula I.

### General Procedure VI:

To a solution of a carbimidothioate of formula **VII** (eq: 1) and a corresponding hydrazide (eq: 1.2) in n-butanol (1 ml) is added acetic acid (eq: 1.55) at 50 °C. The reaction mixture is heated at 120 °C for 15 h. After cooling the reaction mixture is concentrated in vacuo and purified by column chromatography (silica, 0-20% isopropanol in ethyl acetate) to yield a compound of formula **I.**

### Example 1

### trans-4-(4-Chloro-phenyl)-3-methyl-5-(4-phenoxy-cyclohexyl)-4H-[1,2,4]triazole

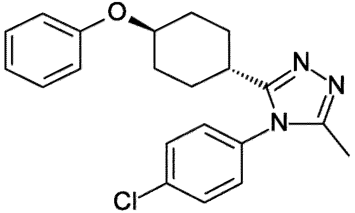

The title compound was obtained as light brown solid in 80% yield according to General Procedure I.
Hydrazide: trans-4-Phenoxy-cyclohexanecarboxylic acid hydrazide
Thioamide: N-(4-chlorophenyl)thioacetamide
MS m/e: 368 (M+H⁺)

### Example 2

### cis-4-(4-Chloro-2-methoxy-phenyl)-3-methyl-5-(4-phenoxy-cyclohexyl)-4H-[1,2,4]triazole

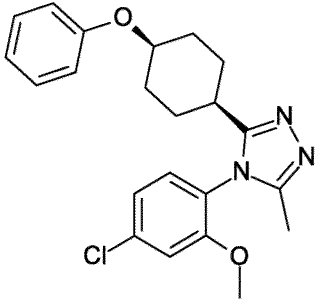

The title compound was obtained as colourless amorphous material in 29% yield according to General Procedure I.
Hydrazide: cis-4-phenoxy-cyclohexanecarboxylic acid hydrazide
Thioamide: N-(4-chloro-2-methoxyphenyl)thioacetamide
MS m/e: 398 (M+H⁺)

### Example 3

### trans-2-(4-(4-(4-Chlorophenyl)-5-methyl-4H-1,2,4-triazol-3-yl)cyclohexyloxy)pyridine

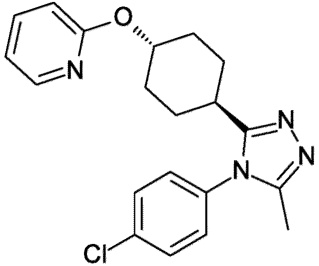

The title compound was obtained as white solid in 73% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-4-chloroaniline
MS m/e: 369 (M+H⁺)

### Example 4

### trans-2-((4-(4-(4-Fluorophenyl)-5-methyl-4H-1,2,4-triazol-3-yl)cyclohexyl)oxy)pyridine

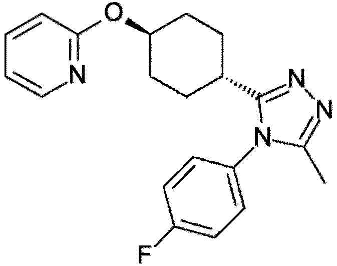

The title compound was obtained as white solid in 53% yield according to General Procedure II.
Hydrazide: trans-4-(pyridin-2-yloxy)-cyclohexanecarboxylic acid hydrazide
Imidazole: N-(1-(1H-Imidazol-1-yl)ethylidene)-4-fluoroaniline
MS m/e: 353 (M+H⁺)

### Example 5

### trans-2-((4-(5-Methyl-4-phenyl-4H-1,2,4-triazol-3-yl)cyclohexyl)oxy)pyridine

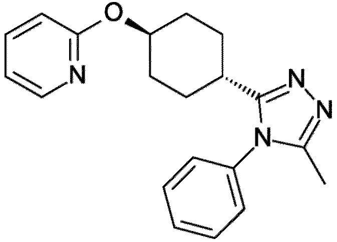

The title compound was obtained as white solid in 37% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)aniline
MS m/e: 333 (M+H⁺)

### Example 6

### trans-2-((4-(5-Methyl-4-(p-tolyl)-4H-1,2,4-triazol-3-yl)cyclohexyl)oxy)pyridine

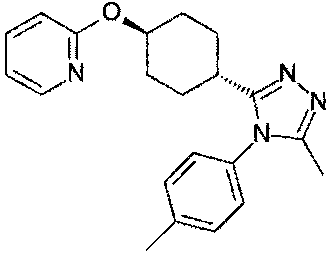

The title compound was obtained as white solid in 56% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-4-methylaniline
MS m/e: 349 (M+H⁺)

### Example 7

### trans-2-((4-(4-(4-(Difluoromethyl)phenyl)-5-methyl-4H-1,2,4-triazol-3-yl)cyclohexyl)oxy)pyridine

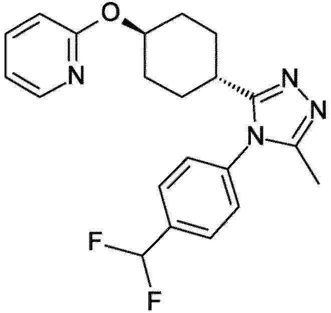

The title compound was obtained as white solid in 46% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-4-(difluoromethyl)aniline
MS m/e: 385 (M+H⁺)

### Example 8

### trans-4-(3-Methyl-5-((1r,4r)-4-(pyridin-2-yloxy)cyclohexyl)-4H-1,2,4-triazol-4-yl)benzonitrile

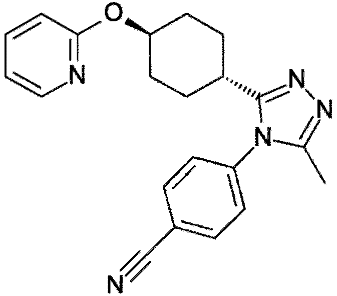

The title compound was obtained as white solid in 30% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-4-cyanoaniline
MS m/e: 360 (M+H⁺)

### Example 9

### trans-2-((4-(4-(4-Bromophenyl)-5-methyl-4H-1,2,4-triazol-3-yl)cyclohexyl)oxy)pyridine

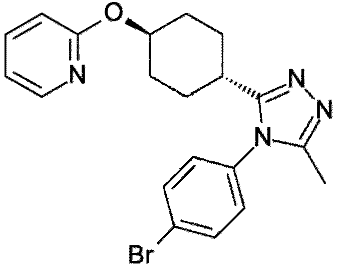

The title compound was obtained as white solid in 78% yield according to General Procedure II.
Hydrazide: trans-4-(pyridin-2-yloxy)-cyclohexanecarboxylic acid hydrazide
Imidazole: N-(1-(1H-Imidazol-1-yl)ethylidene)-4-bromoaniline
MS m/e: 413; 415 (M+H⁺)

### Example 10

### trans-2-((4-(4-(4-Methoxyphenyl)-5-methyl-4H-1,2,4-triazol-3-yl)cyclohexyl)oxy)pyridine

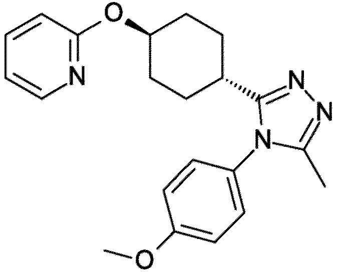

The title compound was obtained as white solid in 47% yield according to General Procedure II.
Hydrazide: trans-4-(pyridin-2-yloxy)-cyclohexanecarboxylic acid hydrazide
Imidazole: N-(1-(1H-Imidazol-1-yl)ethylidene)-4-methoxyaniline
MS m/e: 365 (M+H⁺)

### Example 11

### trans-2-((4-(4-(3-Chlorophenyl)-5-methyl-4H-1,2,4-triazol-3-yl)cyclohexyl)oxy)pyridine

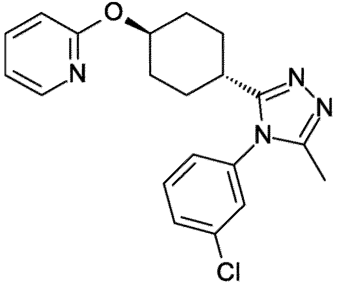

The title compound was obtained as white solid in 14% yield according to General Procedure II.
Hydrazide: trans-4-(pyridin-2-yloxy)-cyclohexanecarboxylic acid hydrazide
Imidazole: N-(1-(1H-Imidazol-1-yl)ethylidene)-3-chloroaniline
MS m/e: 369 (M+H⁺)

### Example 12

### trans-2-((4-(4-(4-Chloro-2-fluorophenyl)-5-methyl-4H-1,2,4-triazol-3-yl)cyclohexyl)oxy)pyridine

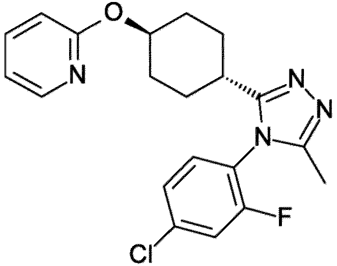

The title compound was obtained as white solid in 54% yield according to General Procedure II.
Hydrazide: trans-4-(pyridin-2-yloxy)-cyclohexanecarboxylic acid hydrazide
Imidazole: N-(1-(1H-Imidazol-1-yl)ethylidene)-4-chloro-2-fluoroaniline
MS m/e: 387 (M+H⁺)

### Example 13

### trans-2-((4-(4-(4-Chloro-2-methylphenyl)-5-methyl-4H-1,2,4-triazol-3-yl)cyclohexyl)oxy)pyridine

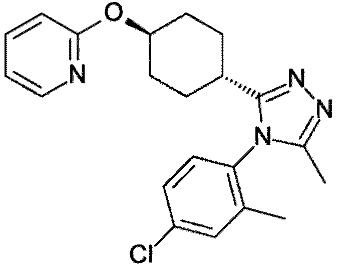

The title compound was obtained as white solid in 37% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-4-chloro-2-methylaniline
MS m/e: 383 (M+H⁺)

### Example 14

### trans-2-[4-[4-(4-Chloro-2-methylsulfanylphenyl)-5-methyl-1,2,4-triazol-3-yl] cyclohexyl]oxypyridine

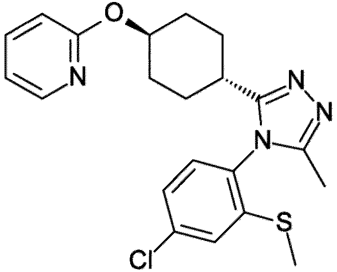

The title compound was obtained as a light brown oil in 43% yield according to General Procedure IV.
Hydrazide: trans-4-(pyridin-2-yloxy)-cyclohexanecarboxylic acid hydrazide
Acid chloride: acetylchloride
Aromatic amine: 4-chloro-2-(methylthio)aniline
MS m/e: 415 (M+H⁺)

### Example 15

### trans-1-[5-Chloro-2-[3-methyl-5-(4-pyridin-2-yloxycyclohexyl)-1,2,4-triazol-4-yl] phenyl]-N,N-dimethylmethanamine

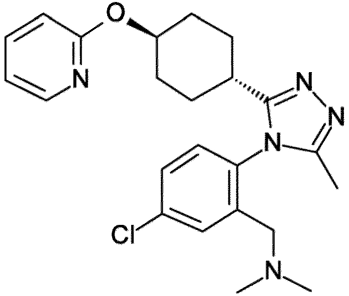

The title compound was obtained as a white solid in 23% yield according to General Procedure IV.
Hydrazide: trans-4-(pyridin-2-yloxy)-cyclohexanecarboxylic acid hydrazide
Acid chloride: acetylchloride
Aromatic amine: 4-chloro-2-((dimethylamino)methyl)aniline
MS m/e: 426 (M+H⁺)

### Example 16

### trans-2-((4-(4-(2,4-Dimethylphenyl)-5-methyl-4H-1,2,4-triazol-3-yl)cyclohexyl)oxy)pyridine

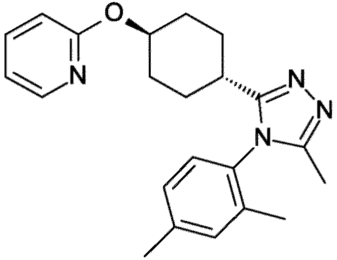

The title compound was obtained as off-white solid in 10% yield according to General Procedure IV.
Hydrazide: trans-4-(pyridin-2-yloxy)-cyclohexanecarboxylic acid hydrazide
Acid chloride: acetylchloride
Aromatic amine: 2,4-dimethylaniline
MS m/e: 363.1 (M+H⁺)

### Example 17

### trans 2-((4-(4-(2-Fluoro-4-methylphenyl)-5-methyl-4H-1,2,4-triazol-3-yl)cyclohexyl)oxy)pyridine

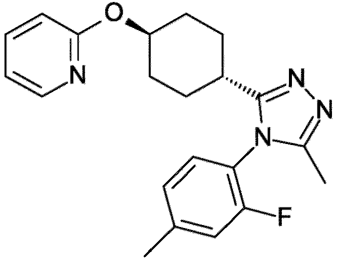

The title compound was obtained as off-white solid in 28% yield according to General Procedure IV.
Hydrazide: trans-4-(pyridin-2-yloxy)-cyclohexanecarboxylic acid hydrazide
Acid chloride: acetylchloride
Aromatic amine: 2-fluoro-4-methylaniline
MS m/e: 367.0 (M+H⁺)

### Example 18

### trans-2-((4-(4-(2-Chloro-4-methylphenyl)-5-methyl-4H-1,2,4-triazol-3-yl)cyclohexyl)oxy)pyridine

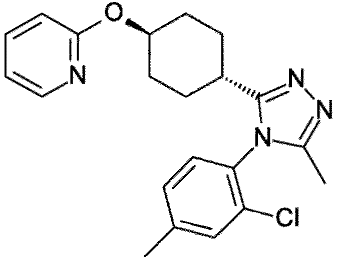

The title compound was obtained as white solid in 44% yield according to General Procedure IV.
Hydrazide: trans-4-(pyridin-2-yloxy)-cyclohexanecarboxylic acid hydrazide
Acid chloride: acetylchloride
Aromatic amine: 2-chloro-4-methylaniline
MS m/e: 383 (M+H⁺)

### Example 19

### trans-2-((4-(4-(4-Chloro-3-fluorophenyl)-5-methyl-4H-1,2,4-triazol-3-yl)cyclohexyl)oxy)pyridine

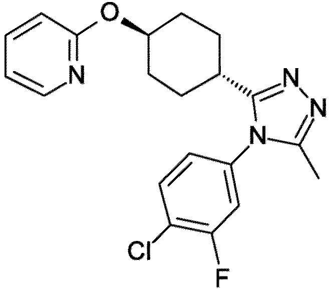

The title compound was obtained as white solid in 55% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-4-chloro-3-fluoroaniline
MS m/e: 387.1 (M+H⁺)

### Example 20

### trans-2-((4-(4-(3-Fluoro-4-methylphenyl)-5-methyl-4H-1,2,4-triazol-3-yl)cyclohexyl)oxy)pyridine

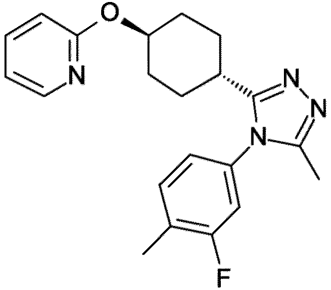

The title compound was obtained as white solid in 44% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-3-fluoro-4-methylaniline
MS m/e: 367 (M+H⁺)

### Example 21

### trans-2-((4-(4-(3,4-Difluorophenyl)-5-methyl-4H-1,2,4-triazol-3-yl)cyclohexyl)oxy)pyridine

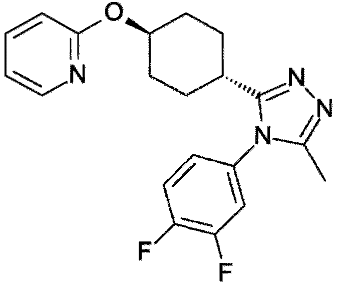

The title compound was obtained as white solid in 47% yield according to General Procedure IV.
Hydrazide: trans-4-(pyridin-2-yloxy)-cyclohexanecarboxylic acid hydrazide
Acid chloride: acetylchloride
Aromatic amine: 3,4-difluoroaniline
MS m/e: 371 (M+H⁺)

### Example 22

### trans-2-[4-[5-Methyl-4-(2,4,6-trimethylphenyl)-1,2,4-triazol-3-yl]cyclohexyl]oxypyridine

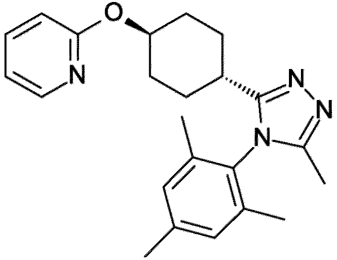

The title compound was obtained as white solid in 26% yield according to General Procedure IV.
Hydrazide: trans-4-(pyridin-2-yloxy)-cyclohexanecarboxylic acid hydrazide
Acid chloride: acetylchloride
Aromatic amine: 2,4,6-trimethylaniline
MS m/e: 377 (M+H⁺)

### Example 23

### trans-5-Chloro-2-(3-methyl-5-(4-(pyridin-2-yloxy)cyclohexyl)-4H-1,2,4-triazol-4-yl)pyridine

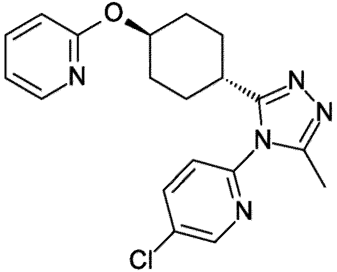

The title compound was obtained as white solid in 51% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-5-chloropyridin-2-amine
MS m/e: 370 (M+H⁺)

### Example 24

### trans-5-Chloro-3-fluoro-2-(3-methyl-5-(4-(pyridin-2-yloxy)cyclohexyl)-4H-1,2,4-triazol-4-yl)pyridine

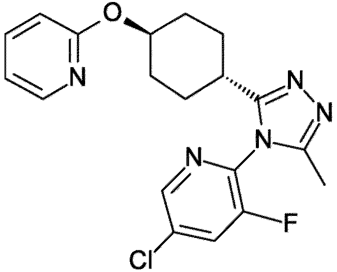

The title compound was obtained as white solid in 47% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-5-chloro-3-fluoropyridin-2-amine
MS m/e: 388 (M+H⁺)

### Example 25

### trans-2-((4-(4-(5-Chlorothiophen-2-yl)-5-methyl-4H-1,2,4-triazol-3-yl)cyclohexyl)oxy)pyridine

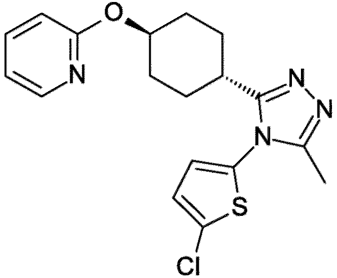

The title compound was obtained as an off-white solid in 30% yield according to General Procedure II.
Hydrazide: trans-4-(pyridin-2-yloxy)-cyclohexanecarboxylic acid hydrazide
Imidazole: N-(1-(1H-imidazol-1-yl)ethylidene)-5-chlorothiophen-2-amine
MS m/e: 375 (M+H⁺)

### Example 26

### trans-2-((4-(4-(4-Chlorophenyl)-5-ethyl-4H-1,2,4-triazol-3-yl)cyclohexyl)oxy)pyridine

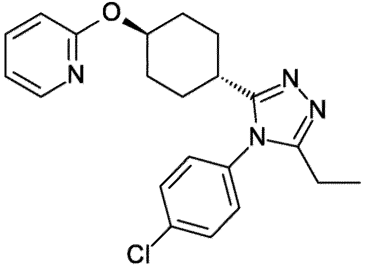

The title compound was obtained as white solid in 60% yield according to General Procedure II.
Hydrazide: trans-4-(pyridin-2-yloxy)-cyclohexanecarboxylic acid hydrazide
Imidazole: N-(1-(1H-imidazol-1-yl)propylidene)-4-chloroaniline
MS m/e: 383 (M+H⁺)

### Example 27

### trans-2-((4-(5-Ethyl-4-(p-tolyl)-4H-1,2,4-triazol-3-yl)cyclohexyl)oxy)pyridine

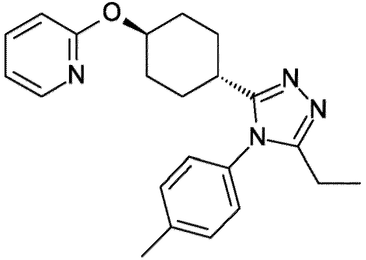

The title compound was obtained as off-white solid in 6% yield according to General Procedure IV.
Hydrazide: trans-4-(pyridin-2-yloxy)-cyclohexanecarboxylic acid hydrazide
Acid chloride: propionyl chloride
Aromatic amine: 4-methylaniline
MS m/e: 363.1 (M+H⁺)

### Example 28

### trans-2-((4-(5-Ethyl-4-(4-fluorophenyl)-4H-1,2,4-triazol-3-yl)cyclohexyl)oxy)pyridine

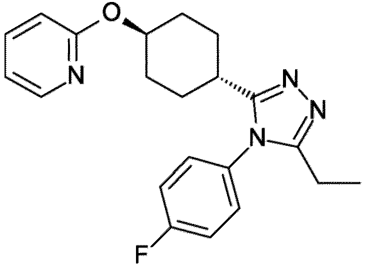

The title compound was obtained as off-white solid in 9% yield according to General Procedure IV.
Hydrazide: trans-4-(pyridin-2-yloxy)-cyclohexanecarboxylic acid hydrazide
Acid chloride: propionyl chloride
Aromatic amine: 4-fluoroaniline
MS m/e: 367.0 (M+H⁺)

### Example 29

### trans-2-((4-(4-(4-Chlorophenyl)-5-isopropyl-4H-1,2,4-triazol-3-yl)cyclohexyl)oxy)pyridine

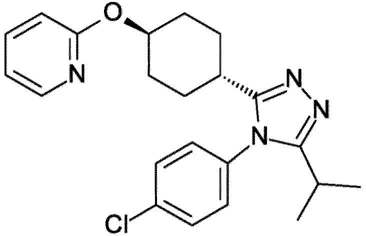

The title compound was obtained as white solid in 7% yield according to General Procedure II.
Hydrazide: trans-4-(pyridin-2-yloxy)-cyclohexanecarboxylic acid hydrazide
Imidazole: N-(1-(1H-imidazol-1-yl)-2-methylprop-1-en-1-yl)-4-chloroaniline
MS m/e: 397 (M+H⁺)

### Example 30

### trans-2-[4-[4-(4-Chlorophenyl)-5-cyclopropyl-1,2,4-triazol-3-yl]cyclohexyl]oxypyridine

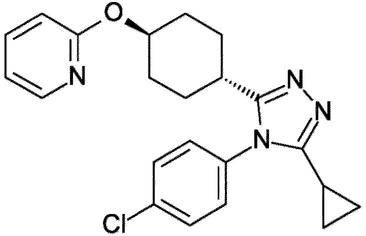

The title compound was obtained as a white solid in 36% yield according to General Procedure IV.
Hydrazide: trans-4-(pyridin-2-yloxy)-cyclohexanecarboxylic acid hydrazide
Acid chloride: cyclopropanecarbonyl chloride
Aromatic amine: 4-chloroaniline
MS m/e: 395 (M+H⁺)

### Example 31

### trans-2-((4-(5-(Difluoromethyl)-4-(p-tolyl)-4H-1,2,4-triazol-3-yl)cyclohexyl)oxy)pyridine

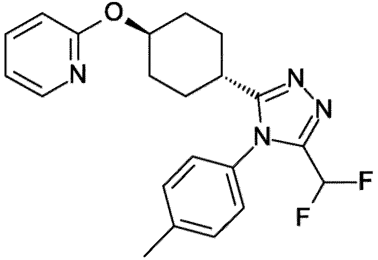

The title compound was obtained as off-white solid in 2% yield according to General Procedure IV.
Hydrazide: trans-4-(pyridin-2-yloxy)-cyclohexanecarboxylic acid hydrazide
Carboxylic acid anhydride: 2,2-difluoroacetic anhydride
Aromatic amine: 4-methylaniline
MS m/e: 385.0 (M+H⁺)

### Example 32

### trans-2-((4-(4-(4-Chlorophenyl)-5-(difluoromethyl)-4H-1,2,4-triazol-3-yl)cyclohexyl)oxy)pyridine

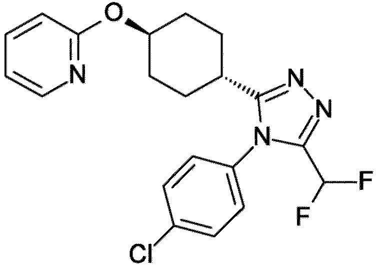

The title compound was obtained as off-white solid in 6% yield according to General Procedure IV.
Hydrazide: trans-4-(pyridin-2-yloxy)-cyclohexanecarboxylic acid hydrazide
Carboxylic acid anhydride: 2,2-difluoroacetic anhydride
Aromatic amine: 4-chloroaniline
MS m/e: 405 (M+H⁺)

### Example 33

### trans-2-((4-(4-(4-Chlorophenyl)-5-(trifluoromethyl)-4H-1,2,4-triazol-3-yl)cyclohexyl)oxy)pyridine

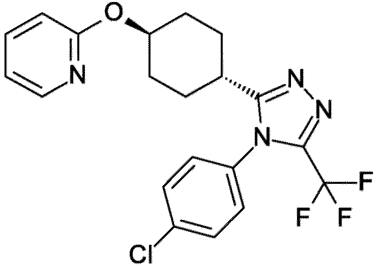

The title compound was obtained as off-white solid in 6% yield according to General Procedure IV.
Hydrazide: trans-4-(pyridin-2-yloxy)-cyclohexanecarboxylic acid hydrazide
Carboxylic acid anhydride: trifluoroacetic anhydride
Aromatic amine: 4-chloroaniline
MS m/e: 423 (M+H⁺)

### Example 34

### trans-(4-(4-Chlorophenyl)-5-(4-(pyridin-2-yloxy)cyclohexyl)-4H-1,2,4-triazol-3-yl)methanol

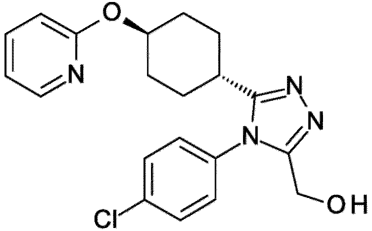

The title compound was obtained as a white solid in 36% yield according to General Procedure IV with the following modification: to remove the acetyl protecting group the crude product of the final cyclisation was stirred with 1 eq. of potassium carbonate (0.1 M in methanol) at 65 °C for 45 min, followed by evaporation and final column chromatogaphy.
Hydrazide: trans-4-(pyridin-2-yloxy)-cyclohexanecarboxylic acid hydrazide
Acid chloride: 2-chloro-2-oxoethyl acetate
Aromatic amine: 4-chloroaniline
MS m/e: 385 (M+H⁺)

### Example 35

### trans-2-(4-(4-(4-Chlorophenyl)-5-(methoxymethyl)-4H-1,2,4-triazol-3-yl)cyclohexyloxy)pyridine

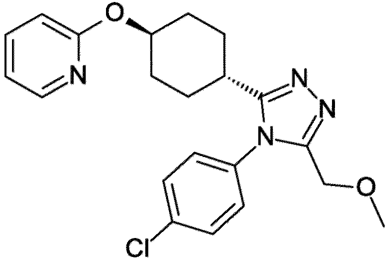

The title compound was obtained as white solid in 27% yield according to General Procedure II.
Hydrazide: trans-4-(pyridin-2-yloxy)-cyclohexanecarboxylic acid hydrazide
Imidazole: N-(1-(1H-imidazol-1-yl)-2-methoxyethylidene)-4-chloroaniline
MS m/e: 399 (M+H⁺)

### Example 36

### trans-2-[4-[4-(4-Chlorophenyl)-5-(methylsulfanylmethyl)-1,2,4-triazol-3-yl]cyclohexyl]oxypyridine

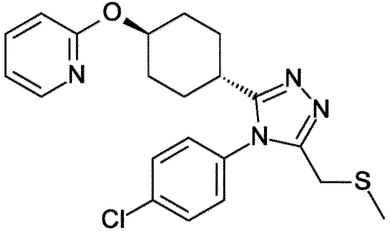

The title compound was obtained as off-white solid in 37% yield according to General Procedure II.
Hydrazide: trans-4-(pyridin-2-yloxy)-cyclohexanecarboxylic acid hydrazide
Imidazole: N-(4-chlorophenyl)-1-(1H-imidazol-1-yl)-2-(methylthio)ethan-1-imine
MS m/e: 415 (M+H⁺)

### Example 37

### trans-(rac)-2-((4-(4-(4-Chlorophenyl)-5-((methylsulfinyl)methyl)-4H-1,2,4-triazol-3-yl)cyclohexyl)oxy)pyridine

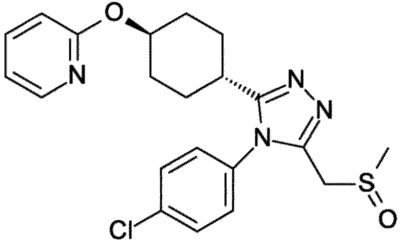

To a solution of trans-2-[4-[4-(4-chlorophenyl)-5-(methylsulfanylmethyl)-1,2,4-triazol-3-yl]cyclohexyl]oxypyridine (400 mg, 964 µmol, eq: 1.00) in dichloromethane (8.2 ml) was added 3-chloroperoxybenzoic acid (205 mg, 916 µmol, eq: 0.95), and the reaction mixture was stirred at room temperature for 16 hours. The reaction mixture was partitioned between dichloromethane (20 ml) and 10% aqueous sodium thiosulfate solution (10 ml). The layers were separated. The aqueous layer was extracted with one 50-ml portion of dichloromethane. The combined organic layers were washed with one 10-ml portion of 10% sodium thiosulfate solution, one 10-ml portion of 1 M sodium carbonate solution, and one 10-ml portion of brine, dried over anhydrous sodium sulfate, filtered and concentrated in vacuo. The residue was purified by chromatography (silica gel, 0-5% methanol in dichloromethane) to yield 338 mg (81%) of the title compound as a white solid.
MS m/e: 431 (M+H⁺)

### Example 38

### trans-(-)-2-((4-(4-(4-Chlorophenyl)-5-((methylsulfinyl)methyl)-4H-1,2,4-triazol-3-yl)cyclohexyl)oxy)pyridine

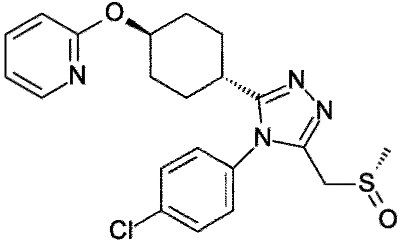

The title compound was obtained by chiral separation (Chiralcel OD, 2-propanol in hexane) of 326 mg of trans-(rac)-2-((4-(4-(4-chlorophenyl)-5-((methylsulfinyl)methyl)-4H-1,2,4-triazol-3-yl)cyclohexyl)oxy)pyridine, (-)-isomer, 118 mg (36%), light yellow solid.
MS m/e: 431 (M+H⁺)

### Example 39

### trans-(+)-2-((4-(4-(4-Chlorophenyl)-5-((methylsulfinyl)methyl)-4H-1,2,4-triazol-3-yl)cyclohexyl)oxy)pyridine

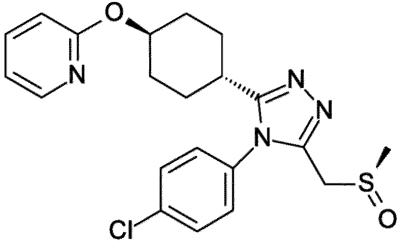

The title compound was obtained by chiral separation (Chiralcel OD, 2-propanol in hexane) of 326 mg of trans-(rac)-2-((4-(4-(4-chlorophenyl)-5-((methylsulfinyl)methyl)-4H-1,2,4-triazol-3-yl)cyclohexyl)oxy)pyridine, (+)-isomer, 135 mg (41%), light yellow solid.
MS m/e: 431 (M+H⁺)

### Example 40

### trans-2-((4-(4-(4-Chlorophenyl)-5-((methylsulfonyl)methyl)-4H-1,2,4-triazol-3-yl)cyclohexyl)oxy)pyridine

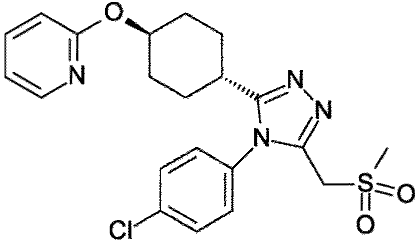

To a solution of trans-2-[4-[4-(4-chlorophenyl)-5-(methylsulfanylmethyl)-1,2,4-triazol-3-yl]cyclohexyl]oxypyridine (200 mg, 482 µmol, eq: 1.00) in dichloromethane (4.09 ml) was added 3-chloroperoxybenzoic acid (211 mg, 940 µmol, eq: 1.95). After stirring at room temperature for 1 hour the reaction mixture was partitioned between dichloromethane (20 ml) and 10% aqueous sodium thiosulfate solution (10 ml). The layers were separated and the aqueous layer was extracted with one 10-ml portion of dichloromethane. The combined organic layers were dried over anhydrous sodium sulfate, filtered and concentrated in vacuo. The residue was purified by chromatography (silica gel, 0-10% methanol in dichloromethane) to yield 66 mg (31%) of the title compound as white solid.
MS m/e: 447 (M+H⁺)

### Example 41

### trans-tert-butyl ((4-(4-Chlorophenyl)-5-(4-(pyridin-2-yloxy)cyclohexyl)-4H-1,2,4-triazol-3-yl)methyl)(methyl)carbamate

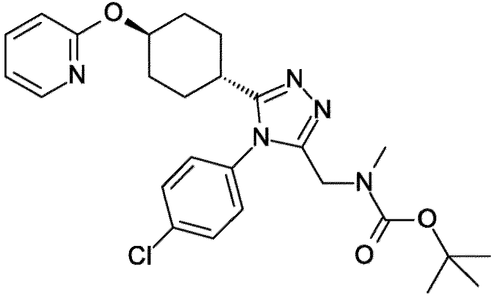

The title compound was obtained as white solid in 36% yield according to General Procedure VI.
Hydrazide: tert-butyl (2-hydrazinyl-2-oxoethyl)(methyl)carbamate (CAS 633328-19-5)
MS m/e: 498 (M+H⁺)

### Example 42

### trans-1-(4-(4-Chlorophenyl)-5-(4-(pyridin-2-yloxy)cyclohexyl)-4H-1,2,4-triazol-3-yl)-N-methylmethanamine

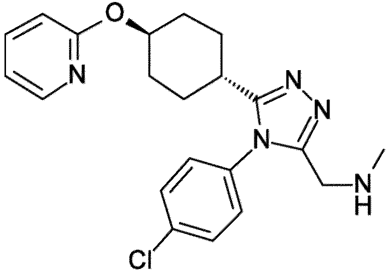

To a solution of trans-tert-butyl ((4-(4-chlorophenyl)-5-(4-(pyridin-2-yloxy)cyclohexyl)-4H-1,2,4-triazol-3-yl)methyl)(methyl)carbamate (350 mg, 703 µmol, eq: 1.00) in ethyl acetate (6 ml) was added 4M hydrogen chloride solution in 1,4-dioxane (1.76 ml, 7.03 mmol, eq: 10.0) at room temperature to give a white suspenison. The reaction mixture was stirred at room temperature for 2.5 h. The solids were collected by filtration and washed with ethyl acetate. Then the solids were partitioned between ethyl acetate (40 ml) and 0.5 M aqueous sodium hydroxide solution (20 ml). The layers were separated, and the aqueous layer was extracted with two 40-ml portions of ethyl acetate. The combined organic layers were dried over anhydrous sodium sulfate, filtered and concentrated in vacuo to give 250 mg (85%) of the title compound as white solid.
MS m/e: 398 (M+H⁺)

### Example 43

### trans-1-(4-(4-Chlorophenyl)-5-(4-(pyridin-2-yloxy)cyclohexyl)-4H-1,2,4-triazol-3-yl)-N,N-dimethylmethanamine

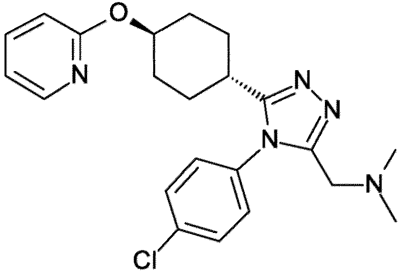

To a solution of trans-1-(4-(4-chlorophenyl)-5-(4-(pyridin-2-yloxy)cyclohexyl)-4H-1,2,4-triazol-3-yl)-N-methylmethanamine (70mg, 176 µmol, eq: 1), acetic acid (21.1 mg, 20.1 µl, 352 µmol, eq: 2.00) and sodium acetate anhydrous (28.9 mg, 352 µmol, eq: 2.00) in dichloromethane (9 ml) was added formaldehyde (36 % solution in water, 41.1 mg, 37.7 µl, 493 µmol, eq: 2.80) at room temperature. The reaction mixture was stirred at room temperature for 1 h. Then sodium triacetoxyborohydride (119 mg, 563 µmol, eq: 3.20) was added in one portion at 0-5 °C. The cooling bath was removed after 10 min, and stirring was continued for 24 h at room temperature. The reaction mixture was partitioned between dichloromethane (20 ml) and saturated sodium bicarboante solution (10 ml), and the layers were separated. The aqueous layer was extracted with two 20 ml portions of dichloromethane. The combined organic layers were dried over anhydrous sodium sulfate, filtered and concentrated in vacuo to give 57 mg (71%) of the title compound as white solid.
MS m/e: 412 (M+H⁺)

### Example 44

### trans-N-((4-(4-Chlorophenyl)-5-(4-(pyridin-2-yloxy)cyclohexyl)-4H-1,2,4-triazol-3-yl)methyl)-N-methylacetamide

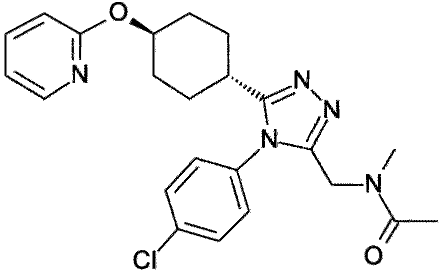

To a solution of trans-1-(4-(4-chlorophenyl)-5-(4-(pyridin-2-yloxy)cyclohexyl)-4H-1,2,4-triazol-3-yl)-N-methylmethanamine (40 mg, 101 µmol, eq: 1) and triethylamine (20.3 mg, 27.9 µl, 201 µmol, eq: 2.0) in dichloromethane (1 ml) was added acetyl chloride (15.8 mg, 14.3 µl, 201 µmol, eq: 2.0), and the reaction mixture was stirred for 2 h at room temperature. The reaction mixture was quenched with methanol (0.5 ml) at room temperature, stirred for 5 min and concentrated in vacuo. The residue was partitioned between ethyl acetate (20 ml) and water (10 ml). The layers were separated. The aqueous layer was extracted with one 20-ml portion of ethyl acetate. The combined organic layers were washed with one 10-ml portion of brine, dried over anhydrous sodium sulfate, filtered and concentrated in vacuo. The crude product was triturated in ethyl acetate (1 ml) and n-heptane (3 ml). The solids were collected by filtration, washed with n-heptane and dried in vacuo to give 39 mg (79%) of the title compound as white solid.
MS m/e: 440 (M+H⁺)

### Example 45

### trans-2-[4-[4-(4-Chlorophenyl)-5-(2-phenylmethoxyethyl)-1,2,4-triazol-3-yl] cyclohexyl] oxypyridine

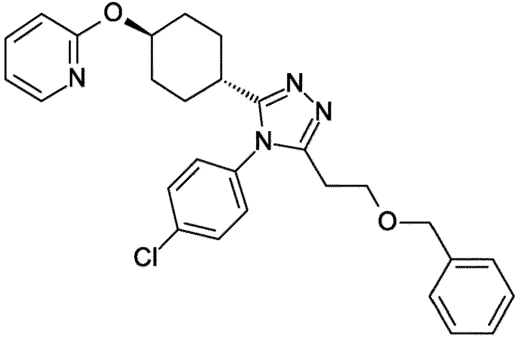

The title compound was obtained as white solid in 38% yield according to General Procedure VI.
Hydrazide: 3-(benzyloxy)propanehydrazide (CAS 37952-35-5)
MS m/e: 489 (M+H⁺)

### Example 46

### trans-2-((4-(4-(4-Chlorophenyl)-5-(2-(methylthio)ethyl)-4H-1,2,4-triazol-3-yl)cyclohexyl)oxy)pyridine

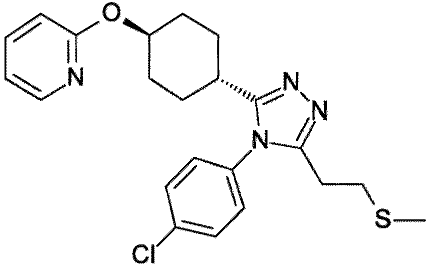

The title compound was obtained as white solid in 4% yield according to General Procedure VI as a by-product in the synthesis of trans-2-[4-(4-chlorophenyl)-5-(4-pyridin-2-yloxycyclohexyl)-1,2,4-triazol-3-yl]-N,N-dimethylethanamine.
Hydrazide: 3-(dimethylamino)propanehydrazide (CAS 22636-79-9)
MS m/e: 429 (M+H⁺)

### Example 47

### trans-2-[4-(4-Chlorophenyl)-5-(4-pyridin-2-yloxycyclohexyl)-1,2,4-triazol-3-yl]-N,N-dimethylethanamine

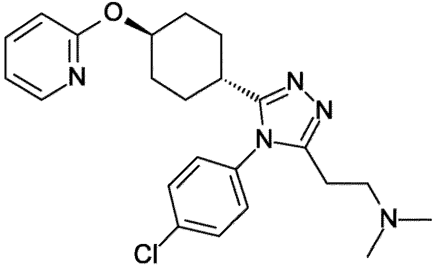

The title compound was obtained as white solid in 7% yield according to General Procedure VI.
Hydrazide: 3-(dimethylamino)propanehydrazide (CAS 22636-79-9)
MS m/e: 426 (M+H⁺)

### Example 48

### trans-2-((4-(4-(4-Chlorophenyl)-5-(methylsulfonyl)-4H-1,2,4-triazol-3-yl)cyclohexyl)oxy)pyridine

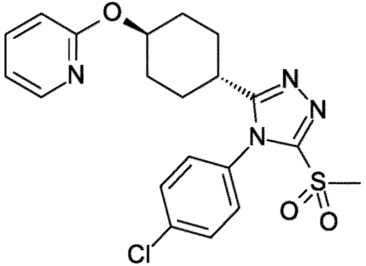

### a) trans-4-(4-Chlorophenyl)-3-(4-(pyridin-2-yloxy)cyclohexyl)-1H-1,2,4-triazole-5(4H)-thione

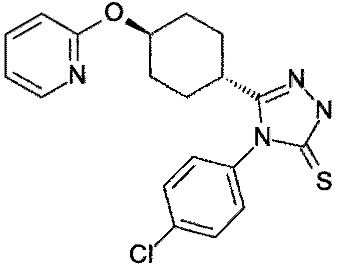

A mixture of trans-4-(pyridin-2-yloxy)cyclohexanecarbohydrazide (200 mg, 0.85 mmol, 1 eq) and 1-chloro-4-isothiocyanatobenzene (146 mg, 850 µmol, eq: 1) in ethanol (3 ml) was heated at reflux for 1 hr. After cooling the solid was filtered off, washed with ethanol and dried. Then the solid was dissolved in 1N aq. sodium hydroxide solution (2 ml) and heated at reflux for 1 h. After cooling the mixture was acidfied to pH=6 with 1N aq. HCl solution, and the precipitate formed was washed with water and dried to obtain 275 mg (84%) of trans-4-(4-chlorophenyl)-3-(4-(pyridin-2-yloxy)cyclohexyl)-1H-1,2,4-triazole-5(4H)-thione as a white solid.
MS m/e: 386.9 (M+H⁺)

### b) trans-2-((4-(4-(4-Chlorophenyl)-5-(methylthio)-4H-1,2,4-triazol-3-yl)cyclohexyl)oxy)pyridine

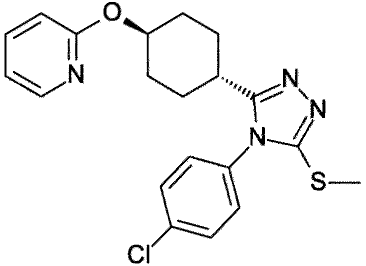

To a solution of trans-4-(4-chlorophenyl)-3-(4-(pyridin-2-yloxy)cyclohexyl)-1H-1,2,4-triazole-5(4H)-thione (100 mg, 258 µmol, 1 eq) in DMF (2 ml) was added potassium carbonate (89.3 mg, 646 µmol, 2.5 eq) and iodomethane (74.1 mg, 32.5 µl, 517 µmol, 2 eq). After stirring at room temperature over night the reaction mixture was concentrated in vacuo, and the residue was partitioned between ethylacetate and water. The combined organic layers were dried over sodium sulfate, filtered and concentrated in vacuo. The crude product was purified by chromatography (silica gel, dichloromethane/ methanol 5%) to yield trans-2-((4-(4-(4-chlorophenyl)-5-(methylthio)-4H-1,2,4-triazol-3-yl)cyclohexyl)oxy)pyridine (79 mg, 76%) as a white solid.
MS m/e: 401.1 (M+H⁺)

### c) trans-2-((4-(4-(4-Chlorophenyl)-5-(methylsulfonyl)-4H-1,2,4-triazol-3-yl)cyclohexyl)oxy)pyridine

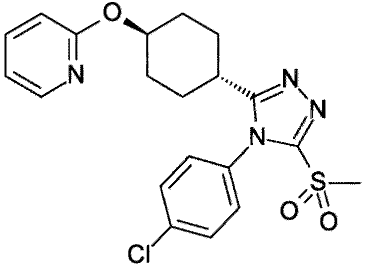

To a solution of 2-((4-(4-(4-chlorophenyl)-5-(methylthio)-4H-1,2,4-triazol-3-yl)cyclohexyl)oxy)pyridine (880 mg, 2.19 mmol, 1 eq) in dichloromethane (18 ml) was added 3-chloroperoxybenzoic acid (1.52 g, 8.78 mmol, 4 eq), and the reaction mixture was stirred at room temperature over night. The reaction mixture was diluted with 40 ml of dichloromethane and washed with 0.1 N sodium thiosulfate solution (20 ml) and saturated sodiumbicarbonate solution (20 ml). The organic layer was dried over sodium sulfate, filtered and concentrated in vacuo to give a yellow foam. This crude product was purified by chromatography (silica gel, dichloromethane/methanol 10%) to yield the title compound (158 mg, 17%) as a white solid.
MS m/e: 433 (M+H⁺)

### Example 49

### trans-2-((4-(4-(4-Chlorophenyl)-5-methoxy-4H-1,2,4-triazol-3-yl)cyclohexyl)oxy)pyridine

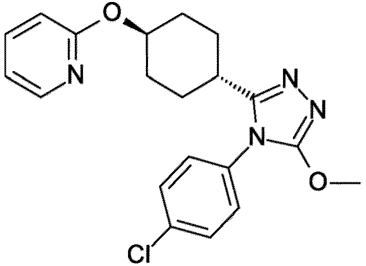

The title compound was obtained as white solid in 66% yield according to General Procedure V.
Alcohol: methanol
MS m/e: 385 (M+H⁺)

### Example 50

### trans-2-((4-(4-(4-Chlorophenyl)-5-ethoxy-4H-1,2,4-triazol-3-yl)cyclohexyl)oxy)pyridine

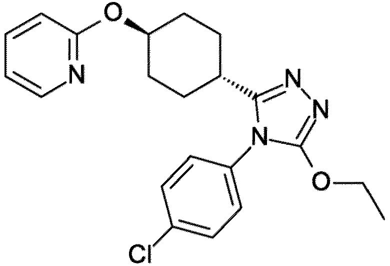

The title compound was obtained as white solid in 62% yield according to General Procedure V.
Alcohol: ethanol
MS m/e: 399 (M+H⁺)

### Example 51

### trans-2-[[4-(4-Chlorophenyl)-5-(4-pyridin-2-yloxycyclohexyl)-1,2,4-triazol-3-yl]oxy]-N,N-dimethylethanamine

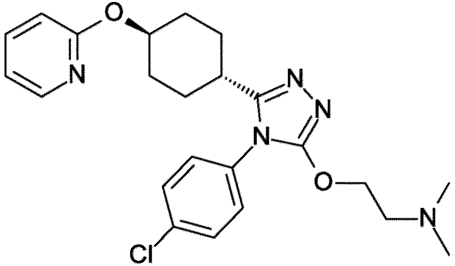

The title compound was obtained as white solid in 59% yield according to General Procedure V.
Alcohol: dimethylaminoethanol
MS m/e: 442 (M+H⁺)

### Example 52

### trans-2-((4-(4-(4-Chlorophenyl)-4H-1,2,4-triazol-3-yl)cyclohexyl)oxy)pyridine

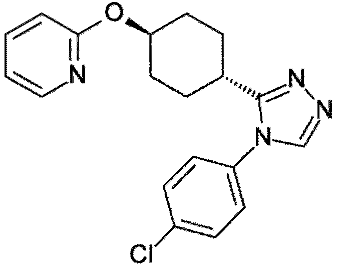

The title compound was obtained as white solid in 44% yield according to General Procedure III.
Hydrazide: trans-4-(pyridin-2-yloxy)-cyclohexanecarboxylic acid hydrazide
Isothiocyanate: 1-chloro-4-isothiocyanatobenzene
MS m/e: 355.5 (M+H⁺)

### Example 53

### trans-2-((4-(4-(4-Chloro-2-methylphenyl)-4H-1,2,4-triazol-3-yl)cyclohexyl)oxy)pyridine

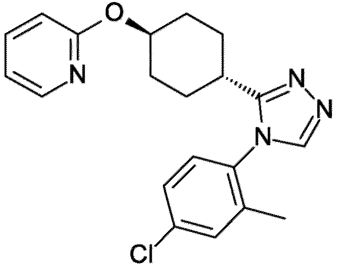

The title compound was obtained as off-white solid in 29% yield according to General Procedure III.
Hydrazide: trans-4-(pyridin-2-yloxy)-cyclohexanecarboxylic acid hydrazide
Isothiocyanate: 4-chloro-1-isothiocyanato-2-methylbenzene
MS m/e: 369.5 (M+H⁺)

### Example 54

### trans-2-((4-(4-(2,4-Dimethylphenyl)-4H-1,2,4-triazol-3-yl)cyclohexyl)oxy)pyridine

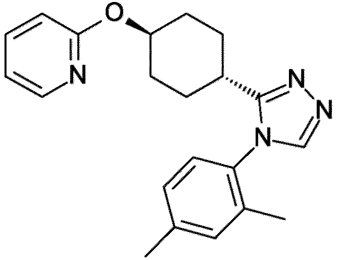

The title compound was obtained as off-white solid in 64% yield according to General Procedure III.
Hydrazide: trans-4-(pyridin-2-yloxy)-cyclohexanecarboxylic acid hydrazide
Isothiocyanate: 1-isothiocyanato-2,4-dimethylbenzene
MS m/e: 349 (M+H⁺)

### Example 55

### trans-4-(4-Chlorophenyl)-N,N-dimethyl-5-(4-(pyridin-2-yloxy)cyclohexyl)-4H-1,2,4-triazol-3-amine

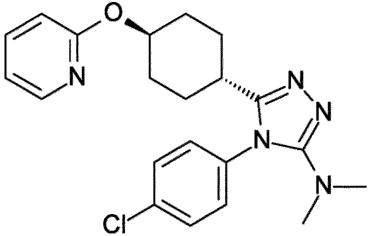

The title compound was obtained as white solid in 17% yield according to General Procedure II.
Hydrazide: trans-4-((5-methylpyridin-2-yl)oxy)-cyclohexanecarboxylic acid hydrazide
Imidazole: N'-(4-chlorophenyl)-N,N-dimethyl-1H-imidazole-1-carboximidamide
MS m/e: 398 (M+H⁺)

### Example 56

### trans-2-((4-(4-(4-Chlorophenyl)-5-methyl-4H-1,2,4-triazol-3-yl)cyclohexyl)oxy)-5-methylpyridine

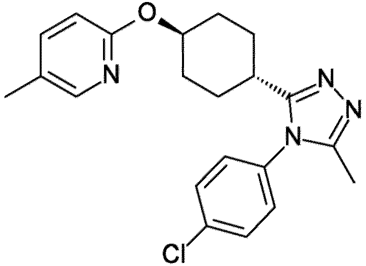

The title compound was obtained as white solid in 60% yield according to General Procedure II.
Hydrazide: trans-4-((5-methylpyridin-2-yl)oxy)-cyclohexanecarboxylic acid hydrazide
Imidazole: N-(1-(1H-imidazol-1-yl)ethylidene)-4-chloroaniline
MS m/e: 383 (M+H⁺)

### Example 57

### trans-5-Methyl-2-[4-[5-methyl-4-(4-methylphenyl)-1,2,4-triazol-3-yl]cyclohexyl]oxypyridine

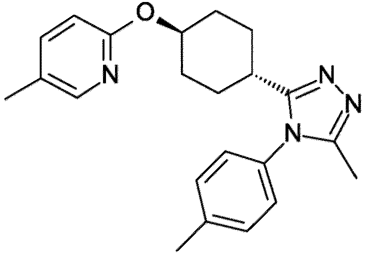

The title compound was obtained as white solid in 56% yield according to General Procedure II.
Hydrazide: trans-4-((5-methylpyridin-2-yl)oxy)-cyclohexanecarboxylic acid hydrazide
Imidazole: N-(1-(1H-imidazol-1-yl)ethylidene)-4-methylaniline
MS m/e: 363 (M+H⁺)

### Example 58

### trans-2- [4- [4-(4-Chlorophenyl)-5-(methoxymethyl)-1,2,4-triazol-3-yl] cyclohexyl] oxy-5-methylpyridine

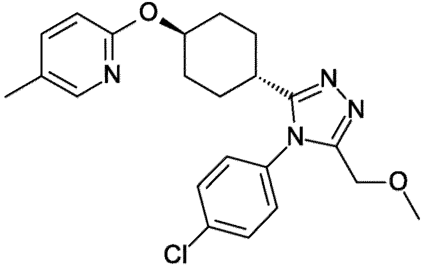

The title compound was obtained as white solid in 47% yield according to General Procedure II.
Hydrazide: trans-4-((5-methylpyridin-2-yl)oxy)-cyclohexanecarboxylic acid hydrazide
Imidazole: N-(1-(1H-imidazol-1-yl)-2-methoxyethylidene)-4-chloroaniline
MS m/e: 413 (M+H⁺)

### Example 59

### trans-2-[4-[5-(Methoxymethyl)-4-(4-methylphenyl)-1,2,4-triazol-3-yl]cyclohexyl]oxy-5-methylpyridine

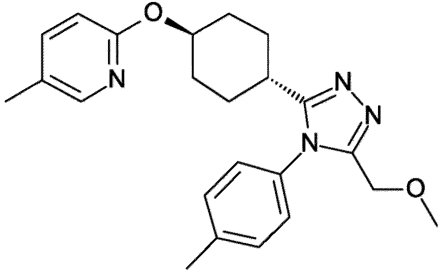

The title compound was obtained as white solid in 44% yield according to General Procedure II.
Hydrazide: trans-4-((5-methylpyridin-2-yl)oxy)-cyclohexanecarboxylic acid hydrazide
Imidazole: 1-(1H-imidazol-1-yl)-2-methoxy-N-(p-tolyl)ethan-1-imine
MS m/e: 393 (M+H⁺)

### Example 60

### trans-2-((4-(4-(4-Chlorophenyl)-5-methyl-4H-1,2,4-triazol-3-yl)cyclohexyl)oxy)-5-(difluoromethyl)pyridine

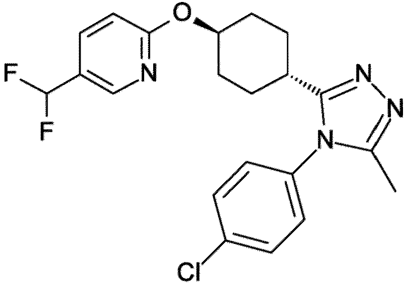

The title compound was obtained as white solid in 26% yield according to General Procedure II.
Hydrazide: trans-4-((5-difluoromethylpyridin-2-yl)oxy)-cyclohexanecarboxylic acid hydrazide
Imidazole: N-(1-(1H-imidazol-1-yl)ethylidene)-4-chloroaniline
MS m/e: 419 (M+H⁺)

### Example 61

### trans-5-Bromo-2-(4-(4-(4-chlorophenyl)-5-methyl-4H-1,2,4-triazol-3-yl)cyclohexyloxy)pyridine

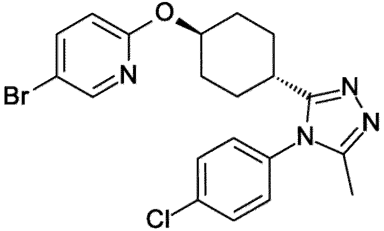

The title compound was obtained as white solid in 59% yield according to General Procedure II.
Hydrazide: trans-4-(5-Bromopyridin-2-yloxy)cyclohexanecarboxylic acid hydrazide
Imidazole: N-(1-(1H-imidazol-1-yl)ethylidene)-4-chloroaniline
MS m/e: 447, 449 (M+H⁺)

### Example 62

### trans-2-(4-(4-(4-Chlorophenyl)-5-methyl-4H-1,2,4-triazol-3-yl)cyclohexyloxy)-5-methoxypyridine

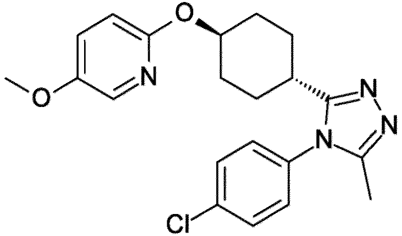

To a mixture of trans-5-bromo-2-(4-(4-(4-chlorophenyl)-5-methyl-4H-1,2,4-triazol-3-yl)cyclohexyloxy)pyridine (0.255 g, 570 µmol, eq: 1.00), 3,4,7,8-tetramethyl-1,10-phenanthroline (0.0538 g, 228 µmol, eq: 0.400), copper(I)-iodide (0.0217 g, 114 µmol, eq: 0.200) and cesium carbonate (0.557 g, 1.71 mmol, eq: 3.00) in toluene (2 ml) was added methanol (182 mg, 0.230 ml, 5.69 mmol, eq: 9.98) at room temperature. The reaction vial was sealed with a screw cap, heated to 85 °C and stirred for 20 h. Because the reaction was not finished, more methanol (182 mg, 0.230 ml, 5.69 mmol, eq: 9.98) was added, and heating at 85 °C was continued for another 25 h. The reaction mixture was diluted with methanol, and the solids were removed by filtration over decalite and washed with methanol. The filtrate was concentrated in vacuo, and the residue was partitioned between ethyl acetate (50 ml) and 1 M aqueous hydrogen chloride solution (50 ml). The layers were the separated and the aqueous layer was extracted with one 50-ml portion of ethyl acetate. The aqueous layer was basified to pH 12 with one 60-ml portion of 2 M aqueous sodium hydroxide solution and extracted with three 50-ml portions of ethyl acetate. The combined organic layers were washed with one 30-ml portion of brine, dried over anhydrous sodium sulfate and concentrated in vacuo. The residue was purified by flash chromatography (silica, 0-30% isopropanol in n-heptane) to give 78 mg (34%) of the title compound as white solid.
MS m/e: 399 (M+H⁺)

### Example 63

### trans-6-(4-(4-(4-Chlorophenyl)-5-methyl-4H-1,2,4-triazol-3-yl)cyclohexyloxy)pyridin-3-ol

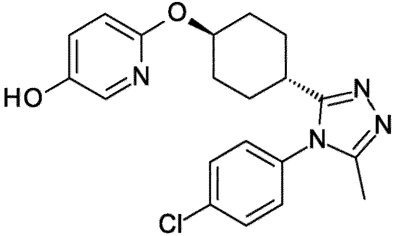

To a solution of trans-2-(4-(4-(4-chlorophenyl)-5-methyl-4H-1,2,4-triazol-3-yl)cyclohexyloxy)-5-methoxypyridine (0.075 g, 188 µmol, eq: 1.00) in dichloromethane (1.88 ml) was added boron tribromide in dichloromethane (1M, 0.94 ml, 940 µmol, eq: 5.00) at 0-5 °C. The ice-water bath was removed after 5 min, and stirring was continued for 90 min. The reaction mixture was quenched with methanol (0.5 ml) at 0-5 °C and stirred for 10 min. The mixture was partitioned between ethyl acetate (30-ml) and saturated ammonium chloride solution (30-ml), and the layers were separated. The aqueous layer (pH 5-6) was extracted with two 30-ml portions of ethyl acetate. The combined organic layers were washed with one 30-ml portion of brine, dried over anhydrous sodium sulfate and concentrated in vacuo. The residue was purified by RP-HPLC on a Gemini NX 3u 50x4.6mm column with water/acetonitrile/formic acid to give 0.02 g (28%) of the title compound as light brown solid.
MS m/e: 385 (M+H⁺)

### Example 64

### trans-5-[4-[4-(4-Chlorophenyl)-5-methyl-1,2,4-triazol-3-yl]cyclohexyl]oxy-2-methylpyridine

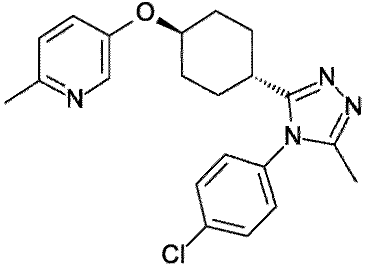

The title compound was obtained as white solid in 59% yield according to General Procedure II.
Hydrazide: trans-4-((6-methylpyridin-3-yl)oxy)-cyclohexanecarboxylic acid hydrazide
Imidazole: N-(1-(1H-imidazol-1-yl)ethylidene)-4-chloroaniline
MS m/e: 383 (M+H⁺)

### Example 65

### trans-2-Methyl-5-[4-[5-methyl-4-(4-methylphenyl)-1,2,4-triazol-3-yl]cyclohexyl]oxypyridine

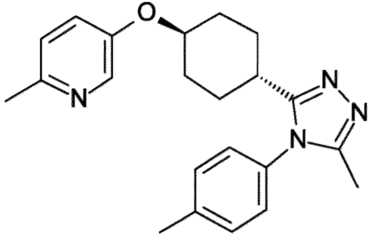

The title compound was obtained as white solid in 52% yield according to General Procedure II.
Hydrazide: trans-4-((6-methylpyridin-3-yl)oxy)-cyclohexanecarboxylic acid hydrazide
Imidazole: N-(1-(1H-Imidazol-1-yl)ethylidene)-4-methylaniline
MS m/e: 363 (M+H⁺)

### Example 66

### trans-5-[4-[5-(Methoxymethyl)-4-(4-methylphenyl)-1,2,4-triazol-3-yl]cyclohexyl]oxy-2-methylpyridine

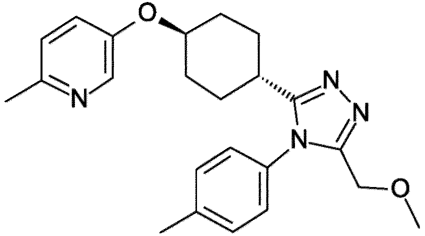

The title compound was obtained as light yellow solid in 25% yield according to General Procedure II.
Hydrazide: trans-4-((6-methylpyridin-3-yl)oxy)-cyclohexanecarboxylic acid hydrazide
Imidazole: 1-(1H-imidazol-1-yl)-2-methoxy-N-(p-tolyl)ethan-1-imine
MS m/e: 393 (M+H⁺)

### Example 67

### trans-5-[4-[4-(4-Chlorophenyl)-5-(methoxymethyl)-1,2,4-triazol-3-yl]cyclohexyl]oxy-2-methylpyridine

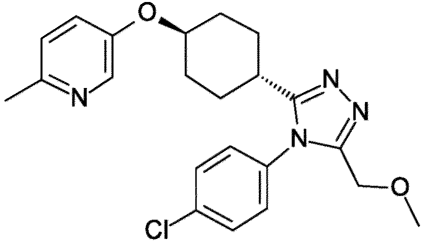

The title compound was obtained as white solid in 28% yield according to General Procedure II.
Hydrazide: trans-4-((6-methylpyridin-3-yl)oxy)-cyclohexanecarboxylic acid hydrazide
Imidazole: N-(1-(1H-imidazol-1-yl)-2-methoxyethylidene)-4-chloroaniline
MS m/e: 413 (M+H⁺)

### Example 68

### trans-2-[4-[5-(Methoxymethyl)-4-(4-methylphenyl)-1,2,4-triazol-3-yl]cyclohexyl]oxypyrazine

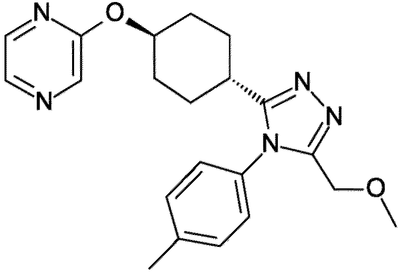

The title compound was obtained as white solid in 39% yield according to General Procedure II.
Hydrazide: trans-4-(pyrazin-2-yloxy)-cyclohexanecarboxylic acid hydrazide
Imidazole: 1-(1H-imidazol-1-yl)-2-methoxy-N-(p-tolyl)ethan-1-imine
MS m/e: 380 (M+H⁺)

### Example 69

### trans-2-[4-[4-(4-Chlorophenyl)-5-(methoxymethyl)-1,2,4-triazol-3-yl]cyclohexyl]oxypyrazine

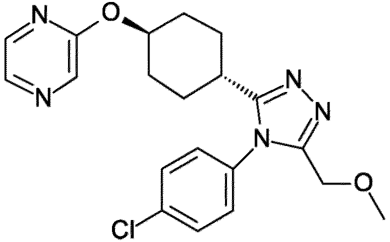

The title compound was obtained as light yellow solid in 38% yield according to General Procedure II.
Hydrazide: trans-4-(pyrazin-2-yloxy)-cyclohexanecarboxylic acid hydrazide
Imidazole: N-(1-(1H-imidazol-1-yl)-2-methoxyethylidene)-4-chloroaniline
MS m/e: 400 (M+H⁺)

### Example 70

### trans-2-[4-[4-(4-Chlorophenyl)-5-methyl-1,2,4-triazol-3-yl] cyclohexyl] oxy-5-methylpyrazine

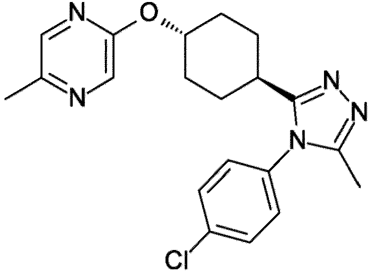

The title compound was obtained as white solid in 56% yield according to General Procedure II.
Hydrazide: trans-4-(5-Methylpyrazin-2-yl)oxycyclohexanecarboxylic acid hydrazide
Imidazole: N-(1-(1H-imidazol-1-yl)ethylidene)-4-chloroaniline
MS m/e: 384 (M+H⁺)

### Example 71

### trans-2-Methyl-5-[4-[5-methyl-4-(4-methylphenyl)-1,2,4-triazol-3-yl]cyclohexyl]oxypyrazine

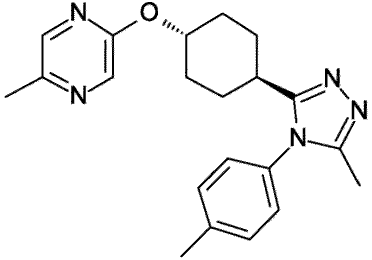

The title compound was obtained as white solid in 53% yield according to General Procedure II.
Hydrazide: trans-4-(5-Methylpyrazin-2-yl)oxycyclohexanecarboxylic acid hydrazide
Imidazole: N-(1-(1H-Imidazol-1-yl)ethylidene)-4-methylaniline
MS m/e: 364 (M+H⁺)

### Example 72

### trans-2- [4- [4-(4-Chlorophenyl)-5-(methoxymethyl)-1,2,4-triazol-3-yl] cyclohexyl] oxy-5-methylpyrazine

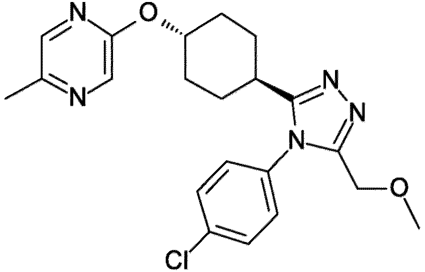

The title compound was obtained as white solid in 23% yield according to General Procedure II.
Hydrazide: trans-4-(5-Methylpyrazin-2-yl)oxycyclohexanecarboxylic acid hydrazide
Imidazole: N-(1-(1H-imidazol-1-yl)-2-methoxyethylidene)-4-chloroaniline
MS m/e: 414 (M+H

1 Robben, et al. (2006). Am J Physiol Renal Physiol. 291, F257-70, "Cell biological aspects of the vasopressin type-2 receptor and aquaporin 2 water channel in nephrogenic diabetes insipidus"
2 Neumann (2008). J Neuroendocrinol. 20, 858-65, "Brain oxytocin: a key regulator of emotional and social behaviours in both females and males"
3 Ebner, et al. (2002). Eur J Neurosci. 15, 384-8., "Forced swimming triggers vasopressin release within the amygdala to modulate stress-coping strategies in rats"
4 Kendler, et al. (2003). Arch Gen Psychiatry. 60, 789-96, "Life Event Dimensions of Loss, Humiliation, Entrapment, and Danger in the Prediction of Onsets of Major Depression and Generalized Anxiety"
5 Regier, et al. (1998). Br J Psychiatry Suppl. 24-8, "Prevalence of anxiety disorders and their comorbidity with mood and addictive disorders"
6 Bielsky, et al. (2004). Neuropsychopharmacology. 29, 483-93, "Profound impairment in social recognition and reduction in anxiety-like behavior in vasopressin V1a receptor knockout mice"
7 Landgraf, et al. (1995). Regul Pept. 59, 229-39., "V1 vasopressin receptor antisense oligodeoxynucleotide into septum reduces vasopressin binding, social discrimination abilities, and anxiety-related behavior in rats"
8 Yirmiya, et al. (2006). 11, 488-94, "Association between the arginine vasopressin 1a receptor (AVPR1a) gene and autism in a family-based study: mediation by socialization skills"
9 Thompson, et al. (2004). Psychoneuroendocrinology. 29, 35-48, "The effects of vasopressin on human facial responses related to social communication"
10 Raskind, et al. (1987). Biol Psychiatry. 22, 453-62, "Antipsychotic drugs and plasma vasopressin in normals and acute schizophrenic patients"
11 Altemus, et al. (1992). Arch Gen Psychiatry. 49, 9-20, "Abnormalities in the regulation of vasopressin and corticotropin releasing factor secretion in obsessive-compulsive disorder"
12 Genes, Brain and Behavior (2011) 10: 228-235
13 Curr. Opin. Neurobiol. 19, 231-234 (2009*)*
14 Kalsbeek, A., E. Fliers, M. A. Hofman, D. F. Swaab and R. M. Buijs. 2010. Vasopressin and the output of the hypothalamic biological clock.
15 Schwartz, W. J., R. J. Coleman and S. M. Reppert. 1983. A daily vasopressin rhythm in rat cerebrospinal fluid. Brain Res 263: 105-12
16 Groblewski, T. A., A. A. Nunez and R. M. Gold. 1981. Circadian rhythms in vasopressin deficient rats. Brain Res Bull 6: 125-30
17 Albers, H. E., C. F. Ferris, S. E. Leeman and B. D. Goldman. 1984. Avian pancreatic polypeptide phase shifts hamster circadian rhythms when microinjected into the suprachiasmatic region. Science 223: 833-5
18 Yoshiaki Yamaguchi, Toru Suzuki, Yasutaka Mizoro, Hiroshi Kori, Kazuki Okada, Yulin Chen, Jean-Michel Fustin, Fumiyoshi Yamazaki, Naoki Mizuguchi, Jing Zhang, Xin Dong, Gozoh Tsujimoto, Yasushi Okuno, Masao Doi, Hitoshi Okamura. Mice Genetically Deficient in Vasopressin V1a and V1b Receptors Are Resistant to Jet Lag. (2013) Science, 342: 85-90

## Claims

1. A compound of formula I 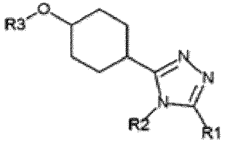 Wherein
R¹ is H, alkyl, cycloalkyl, haloalkyl, hydroxyalkyl, alkoxyalkyl, sulfanylalkyl, sulfinylalkyl, sulfonylalkyl, [tert-butoxycarbonyl-(methyl)amino]alkyl, aminoalkyl, acetyl(methyl)aminoalkyl, benzoxyalkyl, sulfonyl, alkoxy, aminoalkoxy, or amino;
R² is an unsubstituted aryl ring, a substituted aryl ring or a substituted heteroaryl ring wherein substituted aryl ring or substituted heteroaryl ring is substituted with one or more substituents independently selected from halogen, alkyl, alkoxy, sulfanyl, aminoalkyl, haloalkyl or cyano;
R³ is an unsubstituted aryl ring, an unsubstituted heteroaryl ring, or a substituted heteroaryl ring, wherein substituted heteroaryl ring is substituted with one or more substituents independently selected from alkyl, halogen, haloalkyl, alkoxy or hydroxy;
or a pharmaceutically acceptable salts thereof.

2. The compound according to Claim 1, or a pharmaceutically acceptable salt thereof, wherein R¹ is alkyl or alkoxyalkyl.

3. The compound according to any one of claims 1 to 2, or a pharmaceutically acceptable salt thereof, wherein R² is unsubstituted phenyl, substituted phenyl, a substituted 6-member nitrogen containing heteroaryl ring, or a substituted 5-member sulfur containing heteroaryl ring, wherein substituted phenyl, substituted 6-member nitrogen containing heteroaryl ring or substituted 5-member sulfur containing heteroaryl ring is substituted with one or more substituents independently selected from halogen, alkyl, alkoxy, sulfanyl, aminoalkyl, haloalkyl or cyano.

4. The compound according to any one of claims 1 to 3, or a pharmaceutically acceptable salt thereof, wherein R³ is an unsubstituted phenyl, an unsubstituted heteroaryl ring containing nitrogen, or a substituted heteroaryl ring containing nitrogen, wherein substituted heteroaryl ring containing nitrogen is substituted with alkyl, halogen, haloalkyl, alkoxy or hydroxy.

5. A compound according to claim 1, wherein:
R¹ is alkyl or alkoxyalkyl;
R² is phenyl substituted with one to three substituents independently selected from halogen or alkyl;
R³ is a 6-membered heteroaryl ring, unsubstituted or substituted with alkyl;
or a pharmaceutically acceptable salt thereof.

6. The compound according to claim 5, wherein
R¹ is methyl or methoxymethyl;
R² is a substituted phenyl ring substituted with methyl or chloro;
R³ is an unsubstituted pyridyl ring, an unsubstituted pyrazinyl ring, a substituted pyridyl ring or a substituted pyrazinyl ring, wherein substituted pyridyl ring or substituted pyrazinyl ring is substituted with methyl;
or a pharmaceutically acceptable salts thereof.

7. A compound according to any one of claims 1 to 6, selected from:
trans-4-(4-Chloro-phenyl)-3-methyl-5-(4-phenoxy-cyclohexyl)-4H-[1,2,4]triazole;
cis-4-(4-Chloro-2-methoxy-phenyl)-3-methyl-5-(4-phenoxy-cyclohexyl)-4H-[1,2,4]triazole;
trans-2-(4-(4-(4-chlorophenyl)-5-methyl-4H-1,2,4-triazol-3-yl)cyclohexyloxy)pyridine;
trans-2-((4-(4-(4-fluorophenyl)-5-methyl-4H-1,2,4-triazol-3-yl)cyclohexyl)oxy)pyridine;
trans-2-((4-(5-methyl-4-phenyl-4H-1,2,4-triazol-3-yl)cyclohexyl)oxy)pyridine;
trans-2-((4-(5-methyl-4-(p-tolyl)-4H-1,2,4-triazol-3-yl)cyclohexyl)oxy)pyridine;
trans-2-((4-(4-(4-(difluoromethyl)phenyl)-5-methyl-4H-1,2,4-triazol-3-yl)cyclohexyl)oxy)pyridine;
trans-4-(3-methyl-5-(4-(pyridin-2-yloxy)cyclohexyl)-4H-1,2,4-triazol-4-yl)benzonitrile;
trans-2-((4-(4-(4-bromophenyl)-5-methyl-4H-1,2,4-triazol-3-yl)cyclohexyl)oxy)pyridine;
trans-2-((4-(4-(4-methoxyphenyl)-5-methyl-4H-1,2,4-triazol-3-yl)cyclohexyl)oxy)pyridine;
trans-2-((4-(4-(3-chlorophenyl)-5-methyl-4H-1,2,4-triazol-3-yl)cyclohexyl)oxy)pyridine;
trans-2-((4-(4-(4-chloro-2-fluorophenyl)-5-methyl-4H-1,2,4-triazol-3-yl)cyclohexyl)oxy)pyridine;
trans-2-((4-(4-(4-chloro-2-methylphenyl)-5-methyl-4H-1,2,4-triazol-3-yl)cyclohexyl)oxy)pyridine;
trans-2-[4-[4-(4-chloro-2-methylsulfanylphenyl)-5-methyl-1,2,4-triazol-3-yl] cyclohexyl] oxypyridine;
trans-1-[5-chloro-2-[3-methyl-5-(4-pyridin-2-yloxycyclohexyl)-1,2,4-triazol-4-yl]phenyl]-N,N-dimethylmethanamine;
trans-2-((4-(4-(2,4-dimethylphenyl)-5-methyl-4H-1,2,4-triazol-3-yl)cyclohexyl)oxy)pyridine;
trans-2-((4-(4-(2-fluoro-4-methylphenyl)-5-methyl-4H-1,2,4-triazol-3-yl)cyclohexyl)oxy)pyridine;
trans-2-((4-(4-(2-chloro-4-methylphenyl)-5-methyl-4H-1,2,4-triazol-3-yl)cyclohexyl)oxy)pyridine;
trans-2-((4-(4-(4-chloro-3-fluorophenyl)-5-methyl-4H-1,2,4-triazol-3-yl)cyclohexyl)oxy)pyridine;
trans-2-((4-(4-(3-fluoro-4-methylphenyl)-5-methyl-4H-1,2,4-triazol-3-yl)cyclohexyl)oxy)pyridine;
trans-2-((4-(4-(3,4-difluorophenyl)-5-methyl-4H-1,2,4-triazol-3-yl)cyclohexyl)oxy)pyridine;
trans-2-[4-[5-methyl-4-(2,4,6-trimethylphenyl)-1,2,4-triazol-3-yl] cyclohexyl] oxypyridine;
trans-5-chloro-2-(3-methyl-5-(4-(pyridin-2-yloxy)cyclohexyl)-4H-1,2,4-triazol-4-yl)pyridine;
trans-5-chloro-3-fluoro-2-(3-methyl-5-(4-(pyridin-2-yloxy)cyclohexyl)-4H-1,2,4-triazol-4-yl)pyridine;
trans-2-((4-(4-(5-chlorothiophen-2-yl)-5-methyl-4H-1,2,4-triazol-3-yl)cyclohexyl)oxy)pyridine;
trans-2-((4-(4-(4-chlorophenyl)-5-ethyl-4H-1,2, 4-triazol-3-yl)cyclohexyl)oxy)pyridine;
trans-2-((4-(5-ethyl-4-(p-tolyl)-4H-1,2,4-triazol-3-yl)cyclohexyl)oxy)pyridine;
trans-2-((4-(5-ethyl-4-(4-fluorophenyl)-4H-1,2,4-triazol-3-yl)cyclohexyl)oxy)pyridine;
trans-2-((4-(4-(4-chlorophenyl)-5-isopropyl-4H-1,2,4-triazol-3-yl)cyclohexyl)oxy)pyridine;
trans-2-[4-[4-(4-chlorophenyl)-5-cyclopropyl-1,2,4-triazol-3-yl]cyclohexyl]oxypyridine;
trans-2-((4-(5-(difluoromethyl)-4-(p-tolyl)-4H-1,2,4-triazol-3-yl)cyclohexyl)oxy)pyridine;
trans-2-((4-(4-(4-chlorophenyl)-5-(difluoromethyl)-4H-1,2,4-triazol-3-yl)cyclohexyl)oxy)pyridine;
trans-2-((4-(4-(4-chlorophenyl)-5-(trifluoromethyl)-4H-1,2,4-triazol-3-yl)cyclohexyl)oxy)pyridine;
trans-(4-(4-chlorophenyl)-5-(4-(pyridin-2-yloxy)cyclohexyl)-4H-1,2,4-triazol-3-yl)methanol;
trans-2-(4-(4-(4-chlorophenyl)-5-(methoxymethyl)-4H-1,2,4-triazol-3-yl)cyclohexyloxy)pyridine;
trans-2-[4-[4-(4-chlorophenyl)-5-(methylsulfanylmethyl)-1,2,4-triazol-3-yl] cyclohexyl] oxypyridine;
trans-(rac)-2-((4-(4-(4-chlorophenyl)-5-((methylsulfinyl)methyl)-4H-1,2,4-triazol-3-yl)cyclohexyl)oxy)pyridine;
trans-(-)-2-((4-(4-(4-chlorophenyl)-5-((methylsulfinyl)methyl)-4H-1,2,4-triazol-3-yl)cyclohexyl)oxy)pyridine;
trans-(+)-2-((4-(4-(4-chlorophenyl)-5-((methylsulfinyl)methyl)-4H-1,2,4-triazol-3-yl)cyclohexyl)oxy)pyridine;
trans-2-((4-(4-(4-chlorophenyl)-5-((methylsulfonyl)methyl)-4H-1,2,4-triazol-3-yl)cyclohexyl)oxy)pyridine;
trans-tert-butyl ((4-(4-chlorophenyl)-5-(4-(pyridin-2-yloxy)cyclohexyl)-4H-1,2,4-triazol-3-yl)methyl)(methyl)carbamate;
trans-1-(4-(4-chlorophenyl)-5-(4-(pyridin-2-yloxy)cyclohexyl)-4H-1,2,4-triazol-3-yl)-N-methylmethanamine;
trans-1-(4-(4-chlorophenyl)-5-(4-(pyridin-2-yloxy)cyclohexyl)-4H-1,2,4-triazol-3-yl)-N,N-dimethylmethanamine;
trans-N-((4-(4-chlorophenyl)-5-(4-(pyridin-2-yloxy)cyclohexyl)-4H-1,2,4-triazol-3-yl)methyl)-N-methylacetamide;
trans-2-[4-[4-(4-chlorophenyl)-5-(2-phenylmethoxyethyl)-1,2,4-triazol-3-yl] cyclohexyl] oxypyridine;
trans-2-((4-(4-(4-chlorophenyl)-5-(2-(methylthio)ethyl)-4H-1,2,4-triazol-3-yl)cyclohexyl)oxy)pyridine;
trans-2-[4-(4-chlorophenyl)-5-(4-pyridin-2-yloxycyclohexyl)-1,2,4-triazol-3-yl]-N,N-dimethylethanamine;
trans-2-((4-(4-(4-chlorophenyl)-5-(methylsulfonyl)-4H-1,2,4-triazol-3-yl)cyclohexyl)oxy)pyridine;
trans-2-((4-(4-(4-chlorophenyl)-5-methoxy-4H-1,2,4-triazol-3-yl)cyclohexyl)oxy)pyridine;
trans-2-((4-(4-(4-chlorophenyl)-5-ethoxy-4H-1,2,4-triazol-3-yl)cyclohexyl)oxy)pyridine;
trans-2-[[4-(4-chlorophenyl)-5-(4-pyridin-2-yloxycyclohexyl)-1,2,4-triazol-3-yl]oxy]-N,N-dimethylethanamine;
trans-2-((4-(4-(4-chlorophenyl)-4H-1,2,4-triazol-3-yl)cyclohexyl)oxy)pyridine;
trans-2-((4-(4-(4-chloro-2-methylphenyl)-4H-1,2,4-triazol-3-yl)cyclohexyl)oxy)pyridine;
trans-2-((4-(4-(2,4-dimethylphenyl)-4H-1,2,4-triazol-3-yl)cyclohexyl)oxy)pyridine;
trans-4-(4-chlorophenyl)-N,N-dimethyl-5-(4-(pyridin-2-yloxy)cyclohexyl)-4H-1,2,4-triazol-3-amine;
trans-2-((4-(4-(4-chlorophenyl)-5-methyl-4H-1,2,4-triazol-3-yl)cyclohexyl)oxy)-5-methylpyridine;
trans-5-methyl-2-[4-[5-methyl-4-(4-methylphenyl)-1,2,4-triazol-3-yl] cyclohexyl] oxypyridine;
trans-2-((4-(4-(4-chlorophenyl)-5-(methoxymethyl)-4H-1,2,4-triazol-3-yl)cyclohexyl)oxy)-5-methylpyridine;
trans-2-[4-[5-(methoxymethyl)-4-(4-methylphenyl)-1,2,4-triazol-3-yl]cyclohexyl]oxy-5-methylpyridine;
trans-2-((4-(4-(4-chlorophenyl)-5-methyl-4H-1,2,4-triazol-3-yl)cyclohexyl)oxy)-5-(difluoromethyl)pyridine;
trans-5-bromo-2-(4-(4-(4-chlorophenyl)-5-methyl-4H-1,2,4-triazol-3-yl)cyclohexyloxy)pyridine;
trans-2-(4-(4-(4-chlorophenyl)-5-methyl-4H-1,2,4-triazol-3-yl)cyclohexyloxy)-5-methoxypyridine;
trans-6-(4-(4-(4-chlorophenyl)-5-methyl-4H-1,2,4-triazol-3-yl)cyclohexyloxy)pyridin-3-ol;
trans-5-[4-[4-(4-chlorophenyl)-5-methyl-1,2,4-triazol-3-yl]cyclohexyl]oxy-2-methylpyridine;
trans-2-methyl-5-[4-[5-methyl-4-(4-methylphenyl)-1,2,4-triazol-3-yl] cyclohexyl] oxypyridine;
trans-5-[4-[5-(methoxymethyl)-4-(4-methylphenyl)-1,2,4-triazol-3-yl]cyclohexyl]oxy-2-methylpyridine;
trans-5-[4-[4-(4-chlorophenyl)-5-(methoxymethyl)-1,2,4-triazol-3-yl]cyclohexyl]oxy-2-methylpyridine;
trans-2-[4-[5-(methoxymethyl)-4-(4-methylphenyl)-1,2,4-triazol-3-yl] cyclohexyl] oxypyrazine;
trans-2-[4-[4-(4-chlorophenyl)-5-(methoxymethyl)-1,2,4-triazol-3-yl] cyclohexyl] oxypyrazine;
trans-2-[4-[4-(4-chlorophenyl)-5-methyl-1,2,4-triazol-3-yl]cyclohexyl]oxy-5-methylpyrazine;
trans-2-methyl-5-[4-[5-methyl-4-(4-methylphenyl)-1,2,4-triazol-3-yl]cyclohexyl]oxypyrazine;
trans-2-[4-[4-(4-chlorophenyl)-5-(methoxymethyl)-1,2,4-triazol-3-yl]cyclohexyl]oxy-5-methylpyrazine;
or a pharmaceutically acceptable salt thereof.

8. The compound according to any of claims 1 to 7 selected from:
trans-2-(4-(4-(4-chlorophenyl)-5-methyl-4H-1,2,4-triazol-3-yl)cyclohexyloxy)pyridine;
trans-2-(4-(4-(4-chlorophenyl)-5-(methoxymethyl)-4H-1,2,4-triazol-3-yl)cyclohexyloxy)pyridine;
trans-2-((4-(4-(4-chlorophenyl)-5-methyl-4H-1,2,4-triazol-3-yl)cyclohexyl)oxy)-5-methylpyridine;
trans-5-methyl-2-[4-[5-methyl-4-(4-methylphenyl)-1,2,4-triazol-3-yl] cyclohexyl] oxypyridine;
trans-2-((4-(4-(4-chlorophenyl)-5-(methoxymethyl)-4H-1,2,4-triazol-3-yl)cyclohexyl)oxy)-5-methylpyridine;
trans-2-[4-[5-(methoxymethyl)-4-(4-methylphenyl)-1,2,4-triazol-3-yl]cyclohexyl]oxy-5-methylpyridine;
trans-5-[4-[4-(4-chlorophenyl)-5-methyl-1,2,4-triazol-3-yl]cyclohexyl]oxy-2-methylpyridine;
trans-2-methyl-5-[4-[5-methyl-4-(4-methylphenyl)-1,2,4-triazol-3-yl] cyclohexyl] oxypyridine;
trans-5-[4-[5-(methoxymethyl)-4-(4-methylphenyl)-1,2,4-triazol-3-yl]cyclohexyl]oxy-2-methylpyridine;
trans-5-[4-[4-(4-chlorophenyl)-5-(methoxymethyl)-1,2,4-triazol-3-yl]cyclohexyl]oxy-2-methylpyridine;
trans-2-[4-[4-(4-chlorophenyl)-5-(methoxymethyl)-1,2,4-triazol-3-yl]cyclohexyl]oxy-5-methylpyrazine;
or a pharmaceutically acceptable salt thereof.

9. A process for the preparation of a compound according to any one of claims 1 to 8, or a pharmaceutically acceptable salt thereof, comprising the reacting a compound of formula **VII** to a compound of formula **I**: 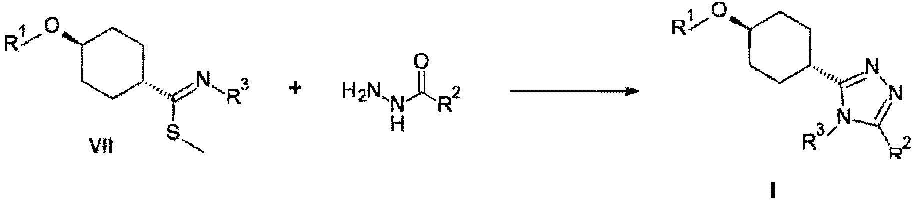 wherein R¹, R² and R³ are as defined hereinabove for formula **I**.

10. A process for the preparation of a compound according to any one of claims 1 to 8, or a pharmaceutically acceptable salt thereof, comprising reacting a compound of formula **V** to a compound of formula **I**: 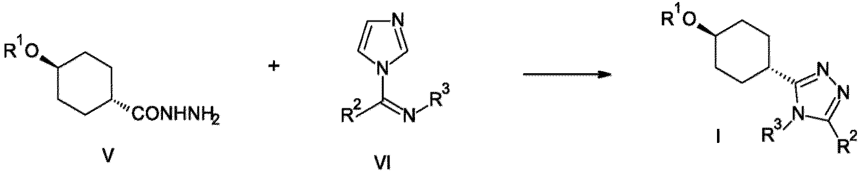 wherein R¹, R² and R³ are as defined hereinabove for formula **I**.

11. A compound according to any of claims 1 to 8, or a pharmaceutically acceptable salt thereof, for use as a therapeutically active substance.

12. A pharmaceutical composition comprising a compound according to any of claims 1 to 8, or a pharmaceutically acceptable salt thereof, and a pharmaceutically acceptable excipient.

13. A compound according to any one of claims 1 to 8, or a pharmaceutically acceptable salt thereof, for use in the therapeutic and/or prophylactic treatment of conditions of inappropriate secretion of vasopressin, anxiety, depressive disorders, obsessive compulsive disorder, autistic spectrum disorders, schizophrenia, aggressive behavior, social anxiety disorder, post-traumatic stress disorder (PTSD), brain edema in stroke or traumatic brain injury, and phase shift sleep disorders, in particular jetlag.

## Patentansprüche

1. Verbindung der Formel I 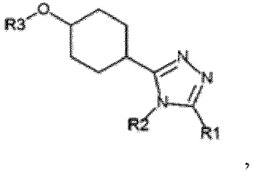 wobei
R¹ H, Alkyl, Cycloalkyl, Halogenalkyl, Hydroxyalkyl, Alkoxyalkyl, Sulfanylalkyl, Sulfinylalkyl, Sulfonylalkyl, [tert-Butoxycarbonyl-(methyl)amino]alkyl, Aminoalkyl, Acetyl(methyl)aminoalkyl, Benzoxyalkyl, Sulfonyl, Alkoxy, Aminoalkoxy oder Amino ist;
R² ein unsubstituierter Arylring, ein substituierter Arylring oder ein substituierter Heteroarylring ist, wobei der substituierte Arylring oder der substituierte Heteroarylring mit einem oder mehreren Substituenten substituiert ist, die unabhängig voneinander aus Halogen, Alkyl, Alkoxy, Sulfanyl, Aminoalkyl, Halogenalkyl oder Cyano ausgewählt sind;
R³ ein unsubstituierter Arylring, ein unsubstituierter Heteroarylring oder ein substituierter Heteroarylring ist, wobei der substituierte Heteroarylring mit einem oder mehreren Substituenten substituiert ist, die unabhängig voneinander aus Alkyl, Halogen, Halogenalkyl, Alkoxy oder Hydroxy ausgewählt sind;
oder ein pharmazeutisch unbedenkliches Salz davon.

2. Verbindung nach Anspruch 1 oder ein pharmazeutisch unbedenkliches Salz davon, wobei R¹ Alkyl oder Alkoxyalkyl ist.

3. Verbindung nach einem der Ansprüche 1 bis 2 oder ein pharmazeutisch unbedenkliches Salz davon, wobei R² unsubstituiertes Phenyl, substituiertes Phenyl, ein substituierter 6-gliedriger Stickstoff enthaltender Heteroarylring oder ein substituierter 5-gliedriger Schwefel enthaltender Heteroarylring ist, wobei das substituierte Phenyl, der substituierte 6-gliedrige Stickstoff enthaltende Heteroarylring oder der substituierte 5-gliedrige Schwefel enthaltende Heteroarylring mit einem oder mehreren Substituenten substituiert ist, die unabhängig voneinander aus Halogen, Alkyl, Alkoxy, Sulfanyl, Aminoalkyl, Halogenalkyl oder Cyano ausgewählt sind.

4. Verbindung nach einem der Ansprüche 1 bis 3 oder ein pharmazeutisch unbedenkliches Salz davon, wobei R³ ein unsubstituiertes Phenyl, ein unsubstituierter Stickstoff enthaltender Heteroarylring oder ein substituierter Stickstoff enthaltender Heteroarylring ist, wobei der substituierte Stickstoff enthaltende Heteroarylring mit Alkyl, Halogen, Halogenalkyl, Alkoxy oder Hydroxy substituiert ist.

5. Verbindung nach Anspruch 1, wobei:
R¹ Alkyl oder Alkoxyalkyl ist;
R² Phenyl ist, das mit einem bis drei Substituenten substituiert ist, die unabhängig voneinander aus Halogen oder Alkyl ausgewählt sind;
R³ ein 6-gliedriger Heteroarylring ist, der unsubstituiert oder mit Alkyl substituiert ist;
oder ein pharmazeutisch unbedenkliches Salz davon.

6. Verbindung nach Anspruch 5, wobei
R¹ Methyl oder Methoxymethyl ist;
R² ein substituierter Phenylring ist, der mit Methyl oder Chlor substituiert ist;
R³ ein unsubstituierter Pyridylring, ein unsubstituierter Pyrazinylring, ein substituierter Pyridylring oder ein substituierter Pyrazinylring ist, wobei der substituierte Pyridylring oder der substituierte Pyrazinylring mit Methyl substituiert ist;
oder ein pharmazeutisch unbedenkliches Salz davon.

7. Verbindung nach einem der Ansprüche 1 bis 6, ausgewählt aus:
trans-4-(4-Chlorphenyl)-3-methyl-5-(4-phenoxycyclohexyl)-4H-[1,2,4]triazol;
cis-4-(4-Chlor-2-methoxyphenyl)-3-methyl-S-(4-phenoxycyclohexyl)-4H-[1,2,4]triazol;
trans-2-(4-(4-(4-Chlorphenyl)-5-methyl-4H-1,2,4-triazol-3-yl)cyclohexyloxy)pyridin;
trans-2-((4-(4-(4-Fluorphenyl)-5-methyl-4H-1,2,4-triazol-3-yl)cyclohexyl)oxy)pyridin;
trans-2-((4-(5-Methyl-4-phenyl-4H-1,2,4-triazol-3-yl)cyclohexyl)oxy)pyridin;
trans-2-((4-(5-Methyl-4-(p-tolyl)-4H-1,2,4-triazol-3-yl)cyclohexyl)oxy)pyridin;
trans-2-((4-(4-(4-(Difluormethyl)phenyl)-5-methyl-4H-1,2,4-triazol-3-yl)cyclohexyl)oxy)pyridin;
trans-4-(3-Methyl-5-(4-(pyridin-2-yloxy)cyclohexyl)-4H-1,2,4-triazol-4-yl)benzonitril;
trans-2-((4-(4-(4-Bromphenyl)-5-methyl-4H-1,2,4-triazol-3-yl)cyclohexyl)oxy)pyridin;
trans-2-((4-(4-(4-Methoxyphenyl)-5-methyl-4H-l,2,4-triazol-3-yl)cyclohexyl)oxy)pyridin;
trans-2-((4-(4-(3-Chlorphenyl)-5-methyl-4H-1,2,4-triazol-3-yl)cyclohexyl)oxy)pyridin;
trans-2-((4-(4-(4-Chlor-2-fluorphenyl)-5-methyl-4H-1,2,4-triazol-3-yl)cyclohexyl)oxy)pyridin;
trans-2-((4-(4-(4-Chlor-2-methylphenyl)-5-methyl-4H-1,2,4-triazol-3-yl)cyclohexyl)oxy)pyridin;
trans-2-[4-[4-(4-Chlor-2-methylsulfanylphenyl)-5-methyl-1,2,4-triazol-3-yl]cyclohexyl]oxypyridin;
trans-1-[5-Chlor-2-[3-methyl-5-(4-pyridin-2-yloxycyclohexyl)-1,2,4-triazol-4-yl]phenyl]-N,N-dimethylmethanamin;
trans-2-((4-(4-(2,4-Dimethylphenyl)-5-methyl-4H-1,2,4-triazol-3-yl)cyclohexyl)oxy)pyridin;
trans-2-((4-(4-(2-Fluor-4-methylphenyl)-5-methyl-4H-1,2,4-triazol-3-yl)cyclohexyl)oxy)pyridin;
trans-2-((4-(4-(2-Chlor-4-methylphenyl)-5-methyl-4H-1,2,4-triazol-3-yl)cyclohexyl)oxy)pyridin;
trans-2-((4-(4-(4-Chlor-3-fluorphenyl)-5-methyl-4H-1,2,4-triazol-3-yl)cyclohexyl)oxy)pyridin;
trans-2-((4-(4-(3-Fluor-4-methylphenyl)-5-methyl-4H-1,2,4-triazol-3-yl)cyclohexyl)oxy)pyridin;
trans-2-((4-(4-(3,4-Difluorphenyl)-5-methyl-4H-1,2,4-triazol-3-yl)cyclohexyl)oxy)pyridin;
trans-2-[4-[5-Methyl-4-(2,4,6-trimethylphenyl)-1,2,4-triazol-3-yl]cyclohexyl]oxypyridin;
trans-5-Chlor-2-(3-methyl-5-(4-(pyridin-2-yloxy)cyclohexyl)-4H-1,2,4-triazol-4-yl)pyridin;
trans-5-Chlor-3-fluor-2-(3-methyl-5-(4-(pyridin-2-yloxy)cyclohexyl)-4H-1,2,4-triazol-4-yl)pyridin;
trans-2-((4-(4-(5-Chlorthiophen-2-yl)-5-methyl-4H-1,2,4-triazol-3-yl)cyclohexyl)oxy)pyridin;
trans-2-((4-(4-(4-Chlorphenyl)-5-ethyl-4H-1,2,4-triazol-3-yl)cyclohexyl)oxy)pyridin;
trans-2-((4-(5-Ethyl-4-(p-tolyl)-4H-1,2,4-triazol-3-yl)cyclohexyl)oxy)pyridin;
trans-2-((4-(5-Ethyl-4-(4-fluorphenyl)-4H-1,2,4-triazol-3-yl)cyclohexyl)oxy)pyridin;
trans-2-((4-(4-(4-Chlorphenyl)-5-isopropyl-4H-1,2,4-triazol-3-yl)cyclohexyl)oxy)pyridin;
trans-2-[4-[4-(4-Chlorphenyl)-5-cyclopropyl-1,2,4-triazol-3-yl]cyclohexyl]oxypyridin;
trans-2-((4-(5-(Difluormethyl)-4-(p-tolyl)-4H-1,2,4-triazol-3-yl)cyclohexyl)oxy)pyridin;
trans-2-((4-(4-(4-Chlorphenyl)-5-(difluormethyl)-4H-1,2,4-triazol-3-yl)cyclohexyl)oxy)pyridin;
trans-2-((4-(4-(4-Chlorphenyl)-5-(trifluormethyl)-4H-1,2,4-triazol-3-yl)cyclohexyl)oxy)pyridin;
trans-(4-(4-Chlorphenyl)-5-(4-(pyridin-2-yloxy)cyclohexyl)-4H-1,2,4-triazol-3-yl)methanol;
trans-2-(4-(4-(4-Chlorphenyl)-5-(methoxymethyl)-4H-1,2,4-triazol-3-yl)cyclohexyloxy)pyridin;
trans-2-[4-[4-(4-Chlorphenyl)-5-(methylsulfanylmethyl)-1,2,4-triazol-3-yl]cyclohexyl]oxypyridin;
trans-(rac)-2-((4-(4-(4-Chlorphenyl)-5-((methylsulfinyl)methyl)-4H-1,2,4-triazol-3-yl)cyclohexyl)oxy)pyridin;
trans-(-)-2-((4-(4-(4-Chlorphenyl)-5-((methylsulfinyl)methyl)-4H-1,2,4-triazol-3-yl)cyclohexyl)oxy)pyridin;
trans-(+)-2-((4-(4-(4-Chlorphenyl)-5-((methylsulfinyl)methyl)-4H-1,2,4-triazol-3-yl)cyclohexyl)oxy)pyridin;
trans-2-((4-(4-(4-Chlorphenyl)-5-((methylsulfonyl)methyl)-4H-1,2,4-triazol-3-yl)cyclohexyl)oxy)pyridin;
trans-tert-Butyl-((4-(4-chlorphenyl)-5-(4-(pyridin-2-yloxy)cyclohexyl)-4H-1,2,4-triazol-3-yl)methyl)(methyl)carbamat;
trans-1-(4-(4-Chlorphenyl)-5-(4-(pyridin-2-yloxy)cyclohexyl)-4H-1,2,4-triazol-3-yl)-N-methylmethanamin;
trans-1-(4-(4-Chlorphenyl)-5-(4-(pyridin-2-yloxy)cyclohexyl)-4H-1,2,4-triazol-3-yl)-N,N-dimethylmethanamin;
trans-N-((4-(4-Chlorphenyl)-5-(4-(pyridin-2-yloxy)cyclohexyl)-4H-1,2,4-triazol-3-yl)methyl)-N-methylacetamid;
trans-2-[4-[4-(4-Chlorphenyl)-5-(2-phenylmethoxyethyl)-1,2,4-triazol-3-yl]cyclohexyl]oxypyridin;
trans-2-((4-(4-(4-Chlorphenyl)-5-(2-(methylthio)ethyl)-4H-1,2,4-triazol-3-yl)cyclohexyl)oxy)pyridin;
trans-2-[4-(4-Chlorphenyl)-5-(4-pyridin-2-yloxycyclohexyl)-1,2,4-triazol-3-yl]-N,N-dimethylethanamin;
trans-2-((4-(4-(4-Chlorphenyl)-5-(methylsulfonyl)-4H-1,2,4-triazol-3-yl)cyclohexyl)oxy)pyridin;
trans-2-((4-(4-(4-Chlorphenyl)-5-methoxy-4H-1,2,4-triazol-3-yl)cyclohexyl)oxy)pyridin;
trans-2-((4-(4-(4-Chlorphenyl)-5-ethoxy-4H-1,2,4-triazol-3-yl)cyclohexyl)oxy)pyridin;
trans-2-[[4-(4-Chlorphenyl)-5-(4-pyridin-2-yloxycyclohexyl)-1,2,4-triazol-3-yl]oxy]-N,N-dimethylethanamin;
trans-2-((4-(4-(4-Chlorphenyl)-4H-1,2,4-triazol-3-yl)cyclohexyl)oxy)pyridin;
trans-2-((4-(4-(4-Chlor-2-methylphenyl)-4H-1,2,4-triazol-3-yl)cyclohexyl)oxy)pyridin;
trans-2-((4-(4-(2,4-Dimethylphenyl)-4H-1,2,4-triazol-3-yl)cyclohexyl)oxy)pyridin;
trans-4-(4-Chlorphenyl)-N,N-dimethyl-5-(4-(pyridin-2-yloxy)cyclohexyl)-4H-1,2,4-triazol-3-amin;
trans-2-((4-(4-(4-Chlorphenyl)-5-methyl-4H-1,2,4-triazol-3-yl)cyclohexyl)oxy)-5-methylpyridin;
trans-5-Methyl-2-[4-[5-methyl-4-(4-methylphenyl)-1,2,4-triazol-3-yl]cyclohexyl]oxypyridin;
trans-2-((4-(4-(4-Chlorphenyl)-5-(methoxymethyl)-4H-1,2,4-triazol-3-yl)cyclohexyl)oxy)-5-methylpyridin;
trans-2-[4-[5-(Methoxymethyl)-4-(4-methylphenyl)-1,2,4-triazol-3-yl]cyclohexyl]oxy-5-methylpyridin;
trans-2-((4-(4-(4-Chlorphenyl)-5-methyl-4H-1,2,4-triazol-3-yl)cyclohexyl)oxy)-5-(difluormethyl)pyridin;
trans-5-Brom-2-(4-(4-(4-chlorphenyl)-5-methyl-4H-1,2,4-triazol-3-yl)cyclohexyloxy)pyridin;
trans-2-(4-(4-(4-Chlorphenyl)-5-methyl-4H-1,2,4-triazol-3-yl)cyclohexyloxy)-5-methoxypyridin;
trans-6-(4-(4-(4-Chlorphenyl)-5-methyl-4H-1,2,4-triazol-3-yl)cyclohexyloxy)pyridin-3-ol;
trans-5-[4-[4-(4-Chlorphenyl)-5-methyl-1,2,4-triazol-3-yl]cyclohexyl]oxy-2-methylpyridin;
trans-2-Methyl-5-[4-[5-methyl-4-(4-methylphenyl)-1,2,4-triazol-3-yl]cyclohexyl]oxypyridin;
trans-5-[4-[5-(Methoxymethyl)-4-(4-methylphenyl)-1,2,4-triazol-3-yl]cyclohexyl]oxy-2-methylpyridin;
trans-5-[4-[4-(4-Chlorphenyl)-5-(methoxymethyl)-1,2,4-triazol-3-yl]cyclohexyl]oxy-2-methylpyridin;
trans-2-[4-[5-(Methoxymethyl)-4-(4-methylphenyl)-1,2,4-triazol-3-yl]cyclohexyl]oxypyrazin;
trans-2-[4-[4-(4-Chlorphenyl)-5-(methoxymethyl)-1,2,4-triazol-3-yl]cyclohexyl]oxypyrazin;
trans-2-[4-[4-(4-Chlorphenyl)-5-methyl-1,2,4-triazol-3-yl]cyclohexyl]oxy-5-methylpyrazin;
trans-2-Methyl-5-[4-[5-methyl-4-(4-methylphenyl)-1,2,4-triazol-3-yl]cyclohexyl]oxypyrazin;
trans-2-[4-[4-(4-Chlorphenyl)-5-(methoxymethyl)-1,2,4-triazol-3-yl]cyclohexyl]oxy-5-methylpyrazin;
oder ein pharmazeutisch unbedenkliches Salz davon.

8. Verbindung nach einem der Ansprüche 1 bis 7, ausgewählt aus:
trans-2-(4-(4-(4-Chlorphenyl)-5-methyl-4H-1,2,4-triazol-3-yl)cyclohexyloxy)pyridin;
trans-2-(4-(4-(4-Chlorphenyl)-5-(methoxymethyl)-4H-1,2,4-triazol-3-yl)cyclohexyloxy)pyridin;
trans-2-((4-(4-(4-Chlorphenyl)-5-methyl-4H-1,2,4-triazol-3-yl)cyclohexyl)oxy)-5-methylpyridin;
trans-5-Methyl-2-[4-[5-methyl-4-(4-methylphenyl)-1,2,4-triazol-3-yl]cyclohexyl]oxypyridin;
trans-2-((4-(4-(4-Chlorphenyl)-5-(methoxymethyl)-4H-1,2,4-triazol-3-yl)cyclohexyl)oxy)-5-methylpyridin;
trans-2-[4-[5-(Methoxymethyl)-4-(4-methylphenyl)-1,2,4-triazol-3-yl]cyclohexyl]oxy-5-methylpyridin;
trans-5-[4-[4-(4-Chlorphenyl)-5-methyl-1,2,4-triazol-3-yl]cyclohexyl]oxy-2-methylpyridin;
trans-2-Methyl-5-[4-[5-methyl-4-(4-methylphenyl)-1,2,4-triazol-3-yl]cyclohexyl]oxypyridin;
trans-5-[4-[5-(Methoxymethyl)-4-(4-methylphenyl)-1,2,4-triazol-3-yl]cyclohexyl]oxy-2-methylpyridin;
trans-5-[4-[4-(4-Chlorphenyl)-5-(methoxymethyl)-1,2,4-triazol-3-yl]cyclohexyl]oxy-2-methylpyridin;
trans-2-[4-[4-(4-Chlorphenyl)-5-(methoxymethyl)-1,2,4-triazol-3-yl]cyclohexyl]oxy-5-methylpyrazin;
oder ein pharmazeutisch unbedenkliches Salz davon.

9. Verfahren zur Herstellung einer Verbindung nach einem der Ansprüche 1 bis 8 oder eines pharmazeutisch unbedenklichen Salzes davon, umfassend das Reagieren einer Verbindung der Formel VII zu einer Verbindung der Formel I: 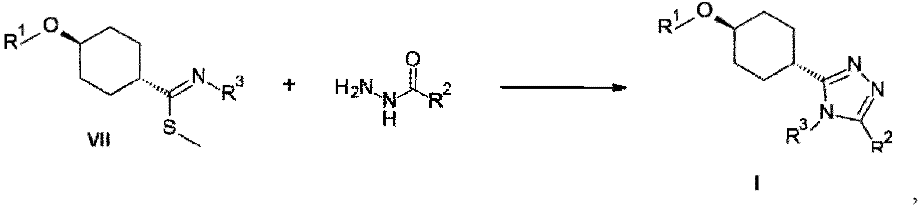 wobei R¹, R² und R³ wie oben für Formel I definiert sind.

10. Verfahren zur Herstellung einer Verbindung nach einem der Ansprüche 1 bis 8 oder eines pharmazeutisch unbedenklichen Salzes davon, umfassend das Reagieren einer Verbindung der Formel V zu einer Verbindung der Formel I: 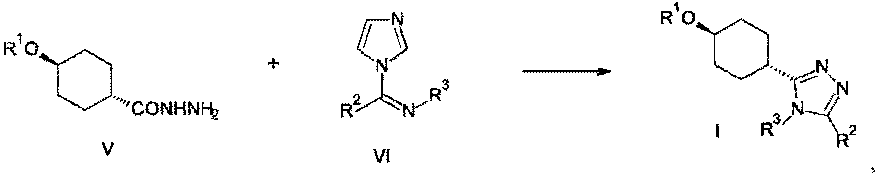 wobei R¹, R² und R³ wie oben für Formel I definiert sind.

11. Verbindung nach einem der Ansprüche 1 bis 8 oder ein pharmazeutisch unbedenkliches Salz davon zur Verwendung als eine therapeutisch wirksame Substanz.

12. Pharmazeutische Zusammensetzung, umfassend eine Verbindung nach einem der Ansprüche 1 bis 8 oder ein pharmazeutisch unbedenkliches Salz davon und einen pharmazeutisch unbedenklichen Hilfsstoff.

13. Verbindung nach einem der Ansprüche 1 bis 8 oder ein pharmazeutisch unbedenkliches Salz zur Verwendung bei der therapeutischen und/oder prophylaktischen Behandlung von Leiden im Zusammenhang mit unangemessener Sekretion von Vasopressin, Angst, depressiven Störungen, Zwangsstörung, Autismus-Spektrum-Störungen, Schizophrenie, aggressivem Verhalten, sozialer Angststörung, posttraumatischer Belastungsstörung (PTSD), Hirnödem bei Schlaganfall oder traumatischer Hirnverletzung und Schlafstörungen aufgrund von Phasenverschiebung, insbesondere Jetlag.

## Revendications

1. Composé de formule I 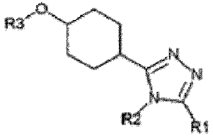 dans lequel
R¹ représente H, un alkyle, un cycloalkyle, un halogénoalkyle, un hydroxyalkyle, un alcoxyalkyle, un sulfanylalkyle, un sulfinylalkyle, un sulfonylalkyle, un [tert-butoxycarbonyl-(méthyl)amino]alkyle, un aminoalkyle, un acétyl(méthyl)aminoalkyle, un benzoxyalkyle, un sulfonyle, un alcoxy, un aminoalcoxy ou un amino ;
R² représente un cycle aryle non substitué, un cycle aryle substitué ou un cycle hétéroaryle substitué dans lequel le cycle aryle substitué ou le cycle hétéroaryle substitué est substitué par un ou plusieurs substituants indépendamment choisis parmi un halogène, un alkyle, un alcoxy, un sulfanyle, un aminoalkyle, un halogénoalkyle ou un cyano ;
R³ représente un cycle aryle non substitué, un cycle hétéroaryle non substitué ou un cycle hétéroaryle substitué, dans lequel le cycle hétéroaryle substitué est substitué par un ou plusieurs substituants indépendamment choisis parmi un alkyle, un halogène, un halogénoalkyle, un alcoxy ou un hydroxy ;
ou un sel pharmaceutiquement acceptable de celui-ci.

2. Composé selon la revendication 1, ou un sel pharmaceutiquement acceptable de celui-ci, dans lequel R¹ représente un alkyle ou un alcoxyalkyle.

3. Composé selon l'une quelconque des revendications 1 à 2, ou un sel pharmaceutique acceptable de celui-ci, dans lequel R² représente un phényle non substitué, un phényle substitué, un cycle hétéroaryle contenant un azote à 6 chaînons substitué ou un cycle hétéroaryle contenant un soufre à 5 chaînons substitué, dans lequel le phényle substitué, le cycle hétéroaryle contenant un azote à 6 chaînons substitué ou le cycle hétéroaryle contenant un soufre à 5 chaînons substitué est substitué par un ou plusieurs substituants indépendamment choisis parmi un halogène, un alkyle, un alcoxy, un sulfanyle, un aminoalkyle, un halogénoalkyle ou un cyano.

4. Composé selon l'une quelconque des revendications 1 à 3, ou un sel pharmaceutiquement acceptable de celui-ci, dans lequel R³ représente un phényle non substitué, un cycle hétéroaryle contenant un azote non substitué ou un cycle hétéroaryle contenant un azote substitué, dans lequel le cycle hétéroaryle contenant un azote substitué est substitué par un alkyle, un halogène, un halogénoalkyle, un alcoxy ou un hydroxy.

5. Composé selon la revendication 1, dans lequel :
R¹ représente un alkyle ou un alcoxyalkyle ;
R² représente un phényle substitué par un à trois substituants indépendamment choisis parmi un halogène ou un alkyle ;
R³ représente un cycle hétéroaryle à 6 chaînons, non substitué ou substitué par un alkyle ;
ou un sel pharmaceutiquement acceptable de celui-ci.

6. Composé selon la revendication 5, dans lequel
R¹ représente un méthyle ou un méthoxyméthyle ;
R² représente un cycle phényle substitué substitué par un méthyle ou un chlore ;
R³ représente un cycle pyridyle non substitué, un cycle pyrazinyle non substitué, un cycle pyridyle substitué ou un cycle pyrazinyle substitué, dans lequel le cycle pyridyle substitué ou le cycle pyrazinyle substitué est substitué par un méthyle ;
ou un sel pharmaceutiquement acceptable de celui-ci.

7. Composé selon l'une quelconque des revendications 1 à 6, choisi parmi :
le trans-4-(4-chloro-phényl)-3-méthyl-5-(4-phénoxy-cyclohexyl)-4H-[1,2,4]triazole ;
le cis-4-(4-chloro-2-méthoxy-phényl)-3-méthyl-5-(4-phénoxy-cyclohexyl)-4H-[1,2,4]triazole ;
la trans-2-(4-(4-(4-chlorophényl)-5-méthyl-4H-1,2,4-triazol-3-yl)cyclohexyloxy)pyridine ;
la trans-2-((4-(4-(4-fluorophényl)-5-méthyl-4H-1,2,4-triazol-3-yl)cyclohexyl)oxy)pyridine ;
la trans-2-((4-(5-méthyl-4-phényl-4H-1,2,4-triazol-3-yl)cyclohexyl)oxy)pyridine ;
la trans-2-((4-(5-méthyl-4-(p-tolyl)-4H-1,2,4-triazol-3-yl)cyclohexyl)oxy)pyridine ;
la trans-2-((4-(4-(4-(difluorométhyl)phényl)-5-méthyl-4H-1,2,4-triazol-3-yl)cyclohexyl)oxy)pyridine ;
le trans-4-(3-méthyl-5-(4-(pyridin-2-yloxy)cyclohexyl)-4H-1,2,4-triazol-4-yl)benzonitrile ;
la trans-2-((4-(4-(4-bromophényl)-5-méthyl-4H-1,2,4-triazol-3-yl)cyclohexyl)oxy)pyridine ;
la trans-2-((4-(4-(4-méthoxyphényl)-5-méthyl-4H-1,2,4-triazol-3-yl)cyclohexyl)oxy)pyridine ;
la trans-2-((4-(4-(3-chlorophényl)-5-méthyl-4H-1,2,4-triazol-3-yl)cyclohexyl)oxy)pyridine ;
la trans-2-((4-(4-(4-chloro-2-fluorophényl)-5-méthyl-4H-1,2,4-triazol-3-yl)cyclohexyl)oxy)pyridine ;
la trans-2-((4-(4-(4-chloro-2-méthylphényl)-5-méthyl-4H-1,2,4-triazol-3-yl)cyclohexyl)oxy)pyridine ;
la trans-2-[4-[4-(4-chloro-2-méthylsulfanylphényl)-5-méthyl-1,2,4-triazol-3-yl]cyclohexyl]oxypyridine ;
la trans-1-[5-chloro-2-[3-méthyl-5-(4-pyridin-2-yloxycyclohexyl)-1,2,4-triazol-4-yl]phényl]-N,N-diméthylméthanamine ;
la trans-2-((4-(4-(2,4-diméthylphényl)-5-méthyl-4H-1,2,4-triazol-3-yl)cyclohexyl)oxy)pyridine ;
la trans-2-((4-(4-(2-fluoro-4-méthylphényl)-5-méthyl-4H-1,2,4-triazol-3-yl)cyclohexyl)oxy)pyridine ;
la trans-2-((4-(4-(2-chloro-4-méthylphényl)-5-méthyl-4H-1,2,4-triazol-3-yl)cyclohexyl)oxy)pyridine ;
la trans-2-((4-(4-(4-chloro-3-fluorophényl)-5-méthyl-4H-1,2,4-triazol-3-yl)cyclohexyl)oxy)pyridine ;
la trans-2-((4-(4-(3-fluoro-4-méthylphényl)-5-méthyl-4H-1,2,4-triazol-3-yl)cyclohexyl)oxy)pyridine ;
la trans-2-((4-(4-(3,4-difluorophényl)-5-méthyl-4H-1,2,4-triazol-3-yl)cyclohexyl)oxy)pyridine ;
la trans-2-[4-[5-méthyl-4-(2,4,6-triméthylphényl)-1,2,4-triazol-3-yl]cyclohexyl]oxypyridine ;
la trans-5-chloro-2-(3-méthyl-5-(4-(pyridin-2-yloxy)cyclohexyl)-4H-1,2,4-triazol-4-yl)pyridine ;
la trans-5-chloro-3-fluoro-2-(3-méthyl-5-(4-(pyridin-2-yloxy)cyclohexyl)-4H-1,2,4-triazol-4-yl)pyridine ;
la trans-2-((4-(4-(5-chlorothiophén-2-yl)-5-méthyl-4H-1,2,4-triazol-3-yl)cyclohexyl)oxy)pyridine ;
la trans-2-((4-(4-(4-chlorophényl)-5-éthyl-4H-1,2,4-triazol-3-yl)cyclohexyl)oxy)pyridine ;
la trans-2-((4-(5-éthyl-4-(p-tolyl)-4H-1,2,4-triazol-3-yl)cyclohexyl)oxy)pyridine ;
la trans-2-((4-(5-éthyl-4-(4-fluorophényl)-4H-1,2,4-triazol-3-yl)cyclohexyl)oxy)pyridine ;
la trans-2-((4-(4-(4-chlorophényl)-5-isopropyl-4H-1,2,4-triazol-3-yl)cyclohexyl)oxy)pyridine ;
la trans-2-[4-[4-(4-chlorophényl)-5-cyclopropyl-1,2,4-triazol-3-yl]cyclohexyl]oxypyridine ;
la trans-2-((4-(5-(difluorométhyl)-4-(p-tolyl)-4H-1,2,4-triazol-3-yl)cyclohexyl)oxy)pyridine ;
la trans-2-((4-(4-(4-chlorophényl)-5-(difluorométhyl)-4H-1,2,4-triazol-3-yl)cyclohexyl)oxy)pyridine ;
la trans-2-((4-(4-(4-chlorophényl)-5-(trifluorométhyl)-4H-1,2,4-triazol-3-yl)cyclohexyl)oxy)pyridine ;
le trans-(4-(4-chlorophényl)-5-(4-(pyridin-2-yloxy)cyclohexyl)-4H-1,2,4-triazol-3-yl)méthanol ;
la trans-2-(4-(4-(4-chlorophényl)-5-(méthoxyméthyl)-4H-1,2,4-triazol-3-yl)cyclohexyloxy)pyridine ;
la trans-2-[4-[4-(4-chlorophényl)-5-(méthylsulfanylméthyl)-1,2,4-triazol-3-yl]cyclohexyl]oxypyridine ;
la trans-(rac)-2-((4-(4-(4-chlorophényl)-5-((méthylsulfinyl)méthyl)-4H-1,2,4-triazol-3-yl)cyclohexyl)oxy)pyridine ;
la trans-(-)-2-((4-(4-(4-chlorophényl)-5-((méthylsulfinyl)méthyl)-4H-1,2,4-triazol-3-yl)cyclohexyl)oxy)pyridine ;
la trans-(+)-2-((4-(4-(4-chlorophényl)-5-((méthylsulfinyl)méthyl)-4H-1,2,4-triazol-3-yl)cyclohexyl)oxy)pyridine ;
la trans-2-((4-(4-(4-chlorophényl)-5-((méthylsulfonyl)méthyl)-4H-1,2,4-triazol-3-yl)cyclohexyl)oxy)pyridine ;
le trans-((4-(4-chlorophényl)-5-(4-(pyridin-2-yloxy)cyclohexyl)-4H-1,2,4-triazol-3-yl)méthyl)(méthyl)carbamate de tert-butyle ;
la trans-1-(4-(4-chlorophényl)-5-(4-(pyridin-2-yloxy)cyclohexyl)-4H-1,2,4-triazol-3-yl)-N-méthylméthanamine ;
la trans-1-(4-(4-chlorophényl)-5-(4-(pyridin-2-yloxy)cyclohexyl)-4H-1,2,4-triazol-3-yl)-N,N-diméthylméthanamine ;
le trans-N-((4-(4-chlorophényl)-5-(4-(pyridin-2-yloxy)cyclohexyl)-4H-1,2,4-triazol-3-yl)méthyl)-N-méthylacétamide ;
la trans-2-[4-[4-(4-chlorophényl)-5-(2-phénylméthoxyéthyl)-1,2,4-triazol-3-yl]cyclohexyl]oxypyridine ;
la trans-2-((4-(4-(4-chlorophényl)-5-(2-(méthylthio)éthyl)-4H-1,2,4-triazol-3-yl)cyclohexyl)oxy)pyridine ;
la trans-2-[4-(4-chlorophényl)-5-(4-pyridin-2-yloxycyclohexyl)-1,2,4-triazol-3-yl]-N,N-diméthyléthanamine ;
la trans-2-((4-(4-(4-chlorophényl)-5-(méthylsulfonyl)-4H-1,2,4-triazol-3-yl)cyclohexyl)oxy)pyridine ;
la trans-2-((4-(4-(4-chlorophényl)-5-méthoxy-4H-1,2,4-triazol-3-yl)cyclohexyl)oxy)pyridine ;
la trans-2-((4-(4-(4-chlorophényl)-5-éthoxy-4H-1,2,4-triazol-3-yl)cyclohexyl)oxy)pyridine ;
la trans-2-[[4-(4-chlorophényl)-5-(4-pyridin-2-yloxycyclohexyl)-1,2,4-triazol-3-yl]oxy]-N,N-diméthyléthanamine ;
la trans-2-((4-(4-(4-chlorophényl)-4H-1,2,4-triazol-3-yl)cyclohexyl)oxy)pyridine ;
la trans-2-((4-(4-(4-chloro-2-méthylphényl)-4H-1,2,4-triazol-3-yl)cyclohexyl)oxy)pyridine ;
la trans-2-((4-(4-(2,4-diméthylphényl)-4H-1,2,4-triazol-3-yl)cyclohexyl)oxy)pyridine ;
la trans-4-(4-chlorophényl)-N,N-diméthyl-5-(4-(pyridin-2-yloxy)cyclohexyl)-4H-1,2,4-triazol-3-amine ;
la trans-2-((4-(4-(4-chlorophényl)-5-méthyl-4H-1,2,4-triazol-3-yl)cyclohexyl)oxy)-5-méthylpyridine ;
la trans-5-méthyl-2-[4-[5-méthyl-4-(4-méthylphényl)-1,2,4-triazol-3-yl]cyclohexyl]oxypyridine ;
la trans-2-((4-(4-(4-chlorophényl)-5-(méthoxyméthyl)-4H-1,2,4-triazol-3-yl)cyclohexyl)oxy)-5-méthylpyridine ;
la trans-2-[4-[5-(méthoxyméthyl)-4-(4-méthylphényl)-1,2,4-triazol-3-yl]cyclohexyl]oxy-5-méthylpyridine ;
la trans-2-((4-(4-(4-chlorophényl)-5-méthyl-4H-1,2,4-triazol-3-yl)cyclohexyl)oxy)-5-(difluorométhyl)pyridine ;
la trans-5-bromo-2-(4-(4-(4-chlorophényl)-5-méthyl-4H-1,2,4-triazol-3-yl)cyclohexyloxy)pyridine ;
la trans-2-(4-(4-(4-chlorophényl)-5-méthyl-4H-1,2,4-triazol-3-yl)cyclohexyloxy)-5-méthoxypyridine ;
le trans-6-(4-(4-(4-chlorophényl)-5-méthyl-4H-1,2,4-triazol-3-yl)cyclohexyloxy)pyridin-3-ol ;
la trans-5-[4-[4-(4-chlorophényl)-5-méthyl-1,2,4-triazol-3-yl]cyclohexyl]oxy-2-méthylpyridine ;
la trans-2-méthyl-5-[4-[5-méthyl-4-(4-méthylphényl)-1,2,4-triazol-3-yl]cyclohexyl]oxypyridine ;
la trans-5-[4-[5-(méthoxyméthyl)-4-(4-méthylphényl)-1,2,4-triazol-3-yl]cyclohexyl]oxy-2-méthylpyridine ;
la trans-5-[4-[4-(4-chlorophényl)-5-(méthoxyméthyl)-1,2,4-triazol-3-yl]cyclohexyl]oxy-2-méthylpyridine ;
la trans-2-[4-[5-(méthoxyméthyl)-4-(4-méthylphényl)-1,2,4-triazol-3-yl]cyclohexyl]oxypyrazine ;
la trans-2-[4-[4-(4-chlorophényl)-5-(méthoxyméthyl)-1,2,4-triazol-3-yl]cyclohexyl]oxypyrazine ;
la trans-2-[4-[4-(4-chlorophényl)-5-méthyl-1,2,4-triazol-3-yl]cyclohexyl]oxy-5-méthylpyrazine ;
la trans-2-méthyl-5-[4-[5-méthyl-4-(4-méthylphényl)-1,2,4-triazol-3-yl]cyclohexyl]oxypyrazine ;
la trans-2-[4-[4-(4-chlorophényl)-5-(méthoxyméthyl)-1,2,4-triazol-3-yl]cyclohexyl]oxy-5-méthylpyrazine ;
ou un sel pharmaceutiquement acceptable de ceux-ci.

8. Composé selon l'une quelconque des revendications 1 à 7 choisi parmi :
la trans-2-(4-(4-(4-chlorophényl)-5-méthyl-4H-1,2,4-triazol-3-yl)cyclohexyloxy)pyridine ;
la trans-2-(4-(4-(4-chlorophényl)-5-(méthoxyméthyl)-4H-1,2,4-triazol-3-yl)cyclohexyloxy)pyridine ;
la trans-2-((4-(4-(4-chlorophényl)-5-méthyl-4H-1,2,4-triazol-3-yl)cyclohexyl)oxy)-5-méthylpyridine ;
la trans-5-méthyl-2-[4-[5-méthyl-4-(4-méthylphényl)-1,2,4-triazol-3-yl]cyclohexyl]oxypyridine ;
la trans-2-((4-(4-(4-chlorophényl)-5-(méthoxyméthyl)-4H-1,2,4-triazol-3-yl)cyclohexyl)oxy)-5-méthylpyridine ;
la trans-2-[4-[5-(méthoxyméthyl)-4-(4-méthylphényl)-1,2,4-triazol-3-yl]cyclohexyl]oxy-5-méthylpyridine ;
la trans-5-[4-[4-(4-chlorophényl)-5-méthyl-1,2,4-triazol-3-yl]cyclohexyl]oxy-2-méthylpyridine ;
la trans-2-méthyl-5-[4-[5-méthyl-4-(4-méthylphényl)-1,2,4-triazol-3-yl]cyclohexyl]oxypyridine ;
la trans-5-[4-[5-(méthoxyméthyl)-4-(4-méthylphényl)-1,2,4-triazol-3-yl]cyclohexyl]oxy-2-méthylpyridine ;
la trans-5-[4-[4-(4-chlorophényl)-5-(méthoxyméthyl)-1,2,4-triazol-3-yl]cyclohexyl]oxy-2-méthylpyridine ;
la trans-2-[4-[4-(4-chlorophényl)-5-(méthoxyméthyl)-1,2,4-triazol-3-yl]cyclohexyl]oxy-5-méthylpyrazine ;
ou un sel pharmaceutiquement acceptable de ceux-ci.

9. Procédé de préparation d'un composé selon l'une quelconque des revendications 1 à 8, ou d'un sel pharmaceutiquement acceptable de celui-ci, comprenant la réaction d'un composé de formule **VII** en un composé de formule **I** : 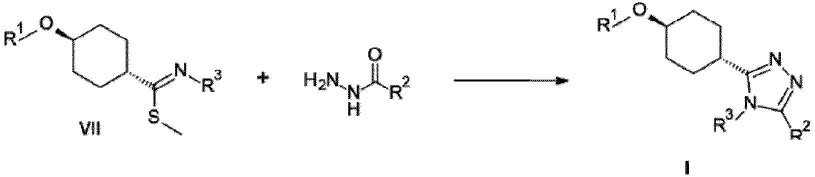 dans lequel R¹, R² et R³ sont tels que définis ci-dessus pour la formule **I**.

10. Procédé de préparation d'un composé selon l'une quelconque des revendications 1 à 8, ou d'un sel pharmaceutiquement acceptable de celui-ci, comprenant la réaction d'un composé de formule **V** en un composé de formule **I** : 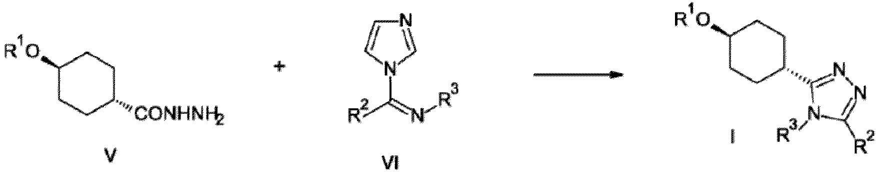 dans lequel R¹, R² et R³ sont tels que définis ci-dessus pour la formule **I**.

11. Composé selon l'une quelconque des revendications 1 à 8, ou un sel pharmaceutiquement acceptable de celui-ci, pour une utilisation comme substance thérapeutiquement active.

12. Composition pharmaceutique comprenant un composé selon l'une quelconque des revendications 1 à 8, ou un sel pharmaceutiquement acceptable de celui-ci, et un excipient pharmaceutiquement acceptable.

13. Composé selon l'une quelconque des revendications 1 à 8, ou un sel pharmaceutiquement acceptable de celui-ci, pour une utilisation dans le traitement thérapeutique et/ou prophylactique d'affections de la sécrétion inappropriée de la vasopressine, d'une anxiété, de troubles dépressifs, d'un trouble obsessionnel compulsif, de troubles du spectre autistique, d'une schizophrénie, d'un comportement agressif, d'un trouble d'anxiété sociale, d'un trouble du stress post-traumatique (TSPT), d'un oedème cérébral dans un accident vasculaire cérébral ou une lésion cérébrale traumatique et de troubles du sommeil associés à un déphasage, en particulier un décalage horaire.
